# EUROPEAN PATENT APPLICATION

(11) **EP 4 813 977 A1**
(43) Date of publication of application: **30.09.2026**
(21) Application number: 24894591.7
(22) Date of filing: 20.11.2024
(51) Int. Cl.: C07D 401/04, C07D 401/14, C07D 471/04, C07D 405/14

(54) **NOVEL COMPOUND AND USE THEREOF**

(30) Priority: 21.11.2023 KR 20230162212; 19.11.2024 KR 20240165547
(71) Applicant: Protier Biotech Inc., Seoul 05855 (KR)
(72) Inventor: KIM, Hyun Tae, Seoul 05855 (KR); SEO, Jeong Jea, Seoul 05855 (KR); LEE, Ji Hyun, Seoul 05855 (KR); NA, Jeong Eun, Yongin-si, Gyeonggi-do 16953 (KR)
(74) Representative: EIP
(86) International application number: PCT/KR2024/018363
(87) International publication number: WO 2025/110699

(57) **Abstract**

The present disclosure relates to a novel compound and a use thereof. The novel compound of the present disclosure, isomer thereof, pharmaceutically acceptable salt thereof, or solvate thereof has an excellent binding affinity for Cereblon, and thus can effectively degrade GSPT1 through the E3 ubiquitin ligase activity of a Cereblon complex, thereby effectively treating a disease such as a proliferative disease.

## Description

### Technical Field

The present disclosure relates to a novel compound and a use thereof.

### Background Art

Generally, a drug exerts a therapeutic effect by binding to a specific site of a specific protein, thereby inhibiting the function of the protein. In other words, the targetability of a specific active site or a binding pocket of a disease-related protein determines whether the disease-related protein is a druggable target for discovery of novel therapeutics.

According to a 2016 report, the proteins utilized as targets for drugs approved by the US Food and Drug Administration (FDA) number approximately 400 types, and these target proteins predominantly consist of enzymes, receptors, transporters, various channels, and membrane proteins. However, current knowledge indicates that approximately 3,000 proteins cause human diseases, and only 13 % of these disease-causing proteins are being developed as target proteins. The reason for this limitation is that current drug development methods fail to target the majority of disease-causing proteins and inhibit the functions thereof. For example, c-Myc, a well-known target for anticancer drugs, functions as a transcription factor and lacks a hydrophobic pocket structure to which a drug can bind, and as for protein aggregates causing neurodegenerative diseases, such as Tau tangles, current technologies cannot remove the protein entanglements. Therefore, novel attempts to target undruggable target proteins are continuously being conducted.

For example, a method for selectively removing a target protein utilizing the ubiquitin-proteasome pathway has recently been proposed. Recently, ubiquitin-mediated protein degradation has been reported to be associated with various diseases, particularly neurodegenerative diseases such as Alzheimer's disease and Parkinson's disease, and genetic diseases, and abnormalities in the protein degradation pathway are considered to be the cause of various forms of diseases including cancer. Accordingly, therapeutics based on a proteolysis-targeting chimaera (PROTAC) are being researched and developed as a treatment modality that removes a disease-causing protein by utilizing the activation of the ubiquitin-proteasome pathway. A PROTAC is a bifunctional small organic molecule consisting of a ligand that binds to a target protein, a ligand that binds to an E3 ubiquitin ligase, and a linker, and the PROTAC possesses a structure by which the target protein can be readily degraded by tethering the targeted protein in close proximity to the E3 ubiquitin ligase capable of initiating protein degradation machinery. This is expected to achieve a desired therapeutic efficacy by positioning a desired disease-related protein near the E3 ubiquitin ligase, thereby degrading the target protein. However, PROTACs have a disadvantage of low cell permeability efficiency due to a relatively large molecular size. In contrast, a molecular glue degrades a target protein by inducing the formation of a complex between an E3 ubiquitin ligase and the target protein without a linker, and thus the molecular glue has a lower molecular weight than the PROTAC, thereby facilitating the development of a therapeutic agent having excellent pharmacokinetic properties.

GSPT1 (G1 to S phase transition 1), which is a translation termination factor regulating a termination stage of protein translation, is involved in cell cycle regulation, apoptosis, and the like. Therefore, GSPT1 is primarily studied in proliferative diseases in which protein translation actively occurs, and the translation termination factor is known to be overexpressed in various cancers, including acute myeloid leukemia (AML), gastric cancer, colon cancer, liver cancer, and breast cancer. Furthermore, since MYC-induced protein translation in cancer cells is known to be dependent on GSPT1, it is expected to exhibit anti-tumor effects in MYC-driven cancers.

Accordingly, the present inventors have developed novel compounds and uses thereof as molecular glues, thereby completing the present disclosure.

### Disclosure of Invention

### Technical Problem

One aspect provides a compound represented by Formula 78, an isomer thereof, a pharmaceutically acceptable salt thereof, or a solvate thereof.

Another aspect provides a pharmaceutical composition for preventing or treating a proliferative disease, including the compound, isomer thereof, pharmaceutically acceptable salt thereof, or solvate thereof.

Another aspect provides a pharmaceutical composition for preventing or treating a GSPT1-related disease, including the compound, isomer thereof, pharmaceutically acceptable salt thereof, or solvate thereof.

Another aspect provides a method for preventing or treating a proliferative disease or a GSPT1-related disease, the method including administering the compound, the isomer thereof, the pharmaceutically acceptable salt thereof, the solvate thereof, or a composition including the same to a subject.

Another aspect provides a use of the compound, isomer thereof, pharmaceutically acceptable salt thereof, or solvate thereof for preventing or treating a proliferative disease or a GSPT1-related disease.

Another aspect provides a use of the compound, isomer thereof, pharmaceutically acceptable salt thereof, or solvate thereof in the manufacture of a medicament for preventing or treating a proliferative disease or a GSPT1-related disease.

Another aspect provides a pharmaceutical composition including the compound, isomer thereof, pharmaceutically acceptable salt thereof, or solvate thereof.

Another aspect provides a GSPT1 degrader including the compound, isomer thereof, pharmaceutically acceptable salt thereof, or solvate thereof.

### Solution to Problem

One aspect provides a compound represented by Formula 78 below, isomer thereof, pharmaceutically acceptable salt thereof, or solvate thereof:

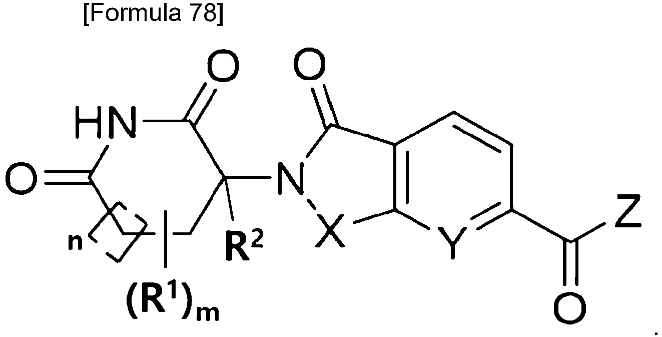

In Formula 78,
one or more R¹ are each independently selected from the group consisting of hydrogen, halogen, a substituted or unsubstituted C₁-C₆ alkyl group, a substituted or unsubstituted C₁-C₆ haloalkyl group, a substituted or unsubstituted C₁-C₆ alkoxy group, a substituted or unsubstituted C₁-C₆ alkylamino group, a substituted or unsubstituted C₁-C₆ alkenyl group, a substituted or unsubstituted C₁-C₆ alkynyl group, -CH₂R³, -OR³, -OCH₂R³, -NR³R⁴, -NH(C=O)R³, -(C=O)R³, -(C=O)OR³, -(C=O)NR³R⁴, -SO₂NHR³, -CN, -NO₂, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group,
R² is selected from the group consisting of hydrogen, deuterium, a substituted or unsubstituted C₁-C₆ alkyl group, and a substituted or unsubstituted C₁-C₆ haloalkyl group,
each R³ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
each R⁴ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
m is an integer in a range of 0 to 2,
n is an integer in a range of 0 to 2,
X is C=O or CH₂,
Y is CH or N,
Z is selected from the group consisting of a substituted or unsubstituted C₁-C₁₀ alkyl group, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group.

Definitions of the substituted substituents used in the formulas herein are as follows.

The term "alkyl" used in the formulas refers to a fully saturated branched or unbranched (or straight-chain or linear) hydrocarbon group. Non-limiting examples of the "alkyl" include methyl, ethyl, n-propyl, isopropyl, n-butyl, isobutyl, sec-butyl, n-pentyl, isopentyl, neopentyl, isoamyl, n-hexyl, 3-methylhexyl, 2,2-dimethylpentyl, 2,3-dimethylpentyl, n-heptyl, and the like.

The term "halogen" used in the formulas includes fluorine, bromine, chlorine, iodine, and the like.

The term "halogen-substituted alkyl group (haloalkyl)" used in the formulas refers to an alkyl group substituted with one or more halo groups, and non-limiting examples thereof include a monohaloalkyl, a dihaloalkyl, or a polyhaloalkyl including a perhaloalkyl. The monohaloalkyl refers to an alkyl group having one iodine, bromine, chlorine, or fluorine therein, and the dihaloalkyl and the polyhaloalkyl refer to an alkyl group having two or more identical or different halo atoms.

The term "alkoxy" used in the formulas represents alkyl-O-, wherein the alkyl is as described above. Non-limiting examples of the alkoxy include methoxy, ethoxy, propoxy, 2-propoxy, butoxy, tert-butoxy, pentyloxy, hexyloxy, cyclopropoxy, cyclohexyloxy, and the like.

The term "alkenyl" used in the formulas refers to a branched or unbranched hydrocarbon having at least one carbon-carbon double bond. Non-limiting examples of the alkenyl include vinyl, allyl, butenyl, isopropenyl, isobutenyl, and the like.

The term "alkynyl" used in the formulas refers to a branched or unbranched hydrocarbon having at least one carbon-carbon triple bond. Non-limiting examples of the alkynyl include ethynyl, butynyl, isobutynyl, isopropynyl, and the like.

The term "cycloalkyl" used in the formulas refers to an alkyl forming a ring. The alkyl is as described above. Non-limiting examples of the cycloalkyl include cyclopropyl, cyclobutyl, cyclopentyl, cyclohexyl, and the like. In addition, "cycloalkenyl" refers to an alkenyl forming a ring, wherein the alkenyl is as described above.

The term "heterocycloalkyl" used in the formulas refers to a fully saturated cyclic hydrocarbon containing one or more heteroatoms selected from N, O, P, or S as ring atoms. In addition, "heterocycloalkenyl" refers to a cyclic hydrocarbon containing one or more heteroatoms selected from N, O, P, or S as ring atoms and having at least one double bond.

The term "aryl" used in the formulas, used alone or in combination, refers to an aromatic hydrocarbon including one or more rings. The term "aryl" also includes a group in which an aromatic ring is fused to one or more cycloalkyl rings. Non-limiting examples of the "aryl" include phenyl, naphthyl, tetrahydronaphthyl, and the like.

The term "arylalkyl" used in the formulas refers to an alkyl substituted with an aryl. Examples of the arylalkyl include benzyl, phenyl-CH₂CH₂-, and the like. In addition, "aryloxy" refers to -O-aryl, and examples of the aryloxy include phenoxy and the like.

The term "heteroaryl" used in the formulas refers to a monocyclic or bicyclic organic compound containing one or more heteroatoms selected from N, O, P, or S, with the remaining ring atoms being carbon. The heteroaryl group may contain, for example, 1 to 5 heteroatoms, and may include 3 to 12 ring members. The S or N atom may be oxidized to have various oxidation states.

The term "heteroarylalkyl" used in the formulas refers to an alkyl substituted with a heteroaryl. In addition, the term "heteroaryloxy" refers to an -O-heteroaryl moiety.

The term "amino group" used in the formulas refers to a group in which a nitrogen atom is covalently bonded to at least one carbon or heteroatom. The amino group includes, for example, -NH₂ and substituted moieties. The term "amino group" may include an alkylamino group in which a nitrogen atom is bonded to at least one additional alkyl group, and an arylamino group and a diarylamino group in which a nitrogen atom is bonded to one or two or more independently selected aryl groups.

The '*' used in the formulas represents a point of attachment to an adjacent atom.

As used herein, the term "substituted" or a "substituent" group means that one or more hydrogen atoms are replaced with one or more non-hydrogen atom groups, provided that valence requirements are satisfied and a chemically stable compound results from the substitution. Within the present specification, unless explicitly stated as "unsubstituted", all substituents should be construed as being substituted or unsubstituted.

For example, the "substitution" in the term "substituted" used above in the alkyl group, haloalkyl group, alkoxy group, alkylamino group, alkenyl group, alkynyl group, cycloalkyl group, cycloalkenyl group, heterocycloalkyl group, heterocycloalkenyl group, aryl group, heteroaryl group, and the like, may mean that one or more hydrogen atoms are substituted with halogen, a halogen-substituted C₁-C₁₀ alkyl group (e.g., CCF₃, CHCF₂, CH₂F, CCl₃, etc.), an alkoxy group, a hydroxy group, a nitro group, a cyano group, an amino group, an amide group, a sulfonamide group, a ketone group, an ester group, an alkyl group, an alkenyl group, an alkynyl group, a cycloalkyl group, a cycloalkenyl group, a heterocycloalkyl group, a heterocycloalkenyl group, an aryl group, an arylalkyl group, an aryloxy group, a heteroaryl group, a heteroarylalkyl group, a heteroaryloxy group, or the like.

In an embodiment, the substitution may be substitution with one or more R^{a}, and the one or more R^{a} may each independently be selected from the group consisting of hydrogen, halogen, a substituted or unsubstituted C₁-C₆ alkyl group, a substituted or unsubstituted C₁-C₆ haloalkyl group, a substituted or unsubstituted C₁-C₆ alkoxy group, a substituted or unsubstituted C₁-C₆ alkenyl group, a substituted or unsubstituted C₁-C₆ alkynyl group, -CH₂R³, -OR³, -OCH₂R³, -NR³R⁴, -NH(C=O)R³, -NHSO₂R³, -(C=O)R³, - (C=O)OR³, -(C=O)NR³R⁴, -SO₂NHR³, -CN, -NO₂, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group.

As used herein, the term "pharmaceutically acceptable salt" refers to a salt in a form suitable for pharmaceutical use among salts, which are substances wherein a cation and an anion are bound by electrostatic attraction, and typically may include a metal salt, a salt with an organic base, a salt with an inorganic acid, a salt with an organic acid, a salt with a basic or acidic amino acid, or the like. For example, the metal salt may be an alkali metal salt (sodium salt, potassium salt, etc.), an alkaline earth metal salt (calcium salt, magnesium salt, barium salt, etc.), an aluminum salt, or the like; the salt with an organic base may be a salt with triethylamine, pyridine, picoline, 2,6-lutidine, ethanolamine, diethanolamine, triethanolamine, cyclohexylamine, dicyclohexylamine, N,N-dibenzylethylenediamine, or the like; the salt with an inorganic acid may be a salt with hydrochloric acid, hydrobromic acid, nitric acid, sulfuric acid, phosphoric acid, or the like; the salt with an organic acid may be a salt with formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid, or the like; the salt with a basic amino acid may be a salt with arginine, lysine, ornithine, or the like; and the salt with an acidic amino acid may be a salt with aspartic acid, glutamic acid, or the like. Particularly preferred salts include, when the compound has an acidic functional group therein, an inorganic salt such as an alkali metal salt (e.g., sodium salt, potassium salt, etc.), an alkaline earth metal salt (e.g., calcium salt, magnesium salt, barium salt, etc.), or the like, and an organic salt such as an ammonium salt; and when the compound has a basic functional group therein, a salt with an inorganic acid such as hydrochloric acid, hydrobromic acid, nitric acid, sulfuric acid, phosphoric acid, or the like, and a salt with an organic acid such as acetic acid, phthalic acid, fumaric acid, oxalic acid, tartaric acid, maleic acid, citric acid, succinic acid, methanesulfonic acid, p-toluenesulfonic acid, or the like.

As used herein, the term "isomer" refers to a compound or a salt thereof having the same chemical or molecular formula but differing optically or stereochemically. Specifically, the isomer may include one or more selected from the group consisting of an enantiomer, a stereoisomer, a mixture of enantiomers, a diastereomer, a mixture of diastereomers, a racemate, and a pharmaceutically acceptable salt thereof.

As used herein, the term "solvate" refers to a compound solvated in an organic or inorganic solvent. The solvate may be, for example, a hydrate.

The compound may be one wherein, in Formula 78, X is CH₂ and Y is CH. Specifically, the compound may be represented by Formula 79 below.

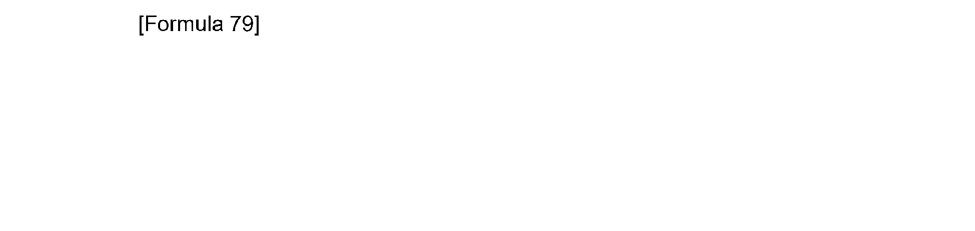

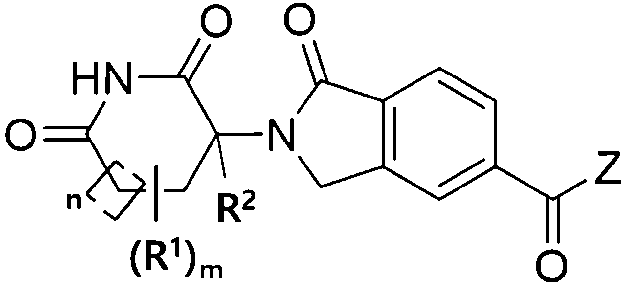

The compound may be one wherein, in Formula 78, m is 0, n is 1, and R² is hydrogen. Specifically, the compound may be represented by Formula 80 below.

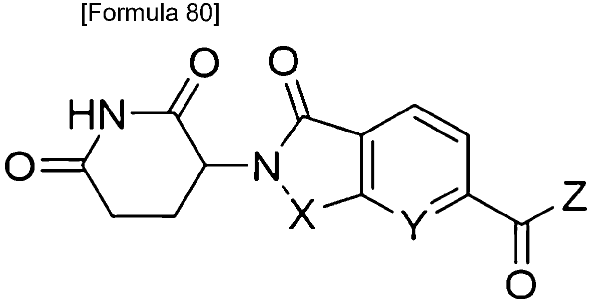

The compound may be one wherein, in Formula 78, X is CH₂, Y is CH, m is 0, n is 1, and R² is hydrogen. Specifically, the compound may be represented by Formula 81 below.

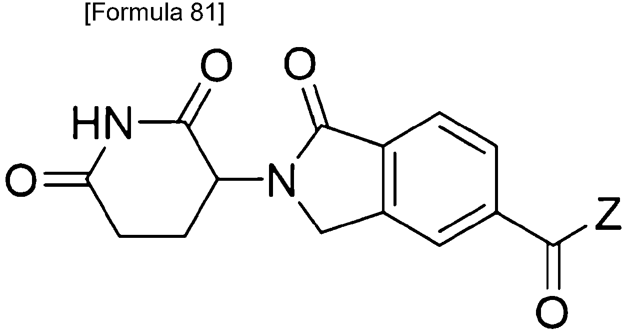

In an embodiment, the compound, isomer thereof, pharmaceutically acceptable salt thereof, or solvate thereof may be a compound according to Formula 81, isomer thereof, pharmaceutically acceptable salt thereof, or solvate thereof.

The compound may be one wherein, in any one or more of Formulas 78 to 81 (specifically, Formula 81),
Z is selected from the group consisting of a substituted or unsubstituted C₁-C₁₀ alkyl group, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group, wherein
when Z is a substituted functional group, Z is substituted with one or more R^{a},
the one or more R^{a} are each independently selected from the group consisting of hydrogen, halogen, a substituted or unsubstituted C₁-C₆ alkyl group, a substituted or unsubstituted C₁-C₆ haloalkyl group, a substituted or unsubstituted C₁-C₆ alkoxy group, a substituted or unsubstituted C₁-C₆ alkenyl group, a substituted or unsubstituted C₁-C₆ alkynyl group, -CH₂R³, -OR³, -OCH₂R³, -NR³R⁴, -NH(C=O)R³, -NHSO₂R³, -(C=O)R³, - (C=O)OR³, -(C=O)NR³R⁴, -SO₂NHR³, -CN, -NO₂, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group,
each R³ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group, and
each R⁴ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group.

In an embodiment, Z may be bonded to a carbon (C) atom of a ketone group of any one or more of Formulas 78 to 81, and specifically, an arbitrary carbon (C) atom in Z may be bonded to the carbon (C) atom of the ketone group of said Formulas.

The compound may be one wherein, in any one or more of Formulas 78 to 81 (specifically, Formula 81), Z includes one or more selected from the group consisting of a substituted or unsubstituted C₁-C₁₀ alkyl group, a substituted or unsubstituted C₃-C₁₂ cycloalkyl group, a substituted or unsubstituted 3- to 12-membered heteroaryl group, and a group represented by Formulas 82-1 to 82-52 below:

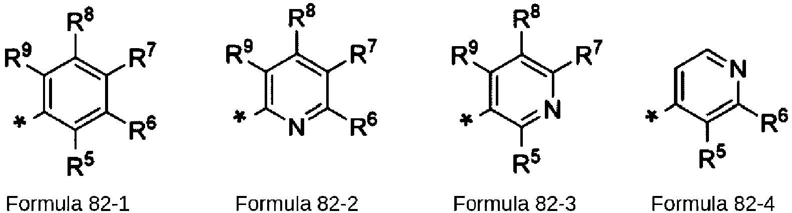

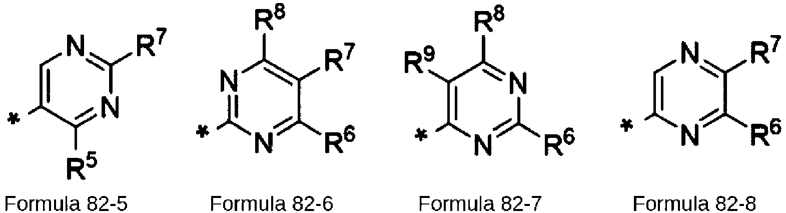

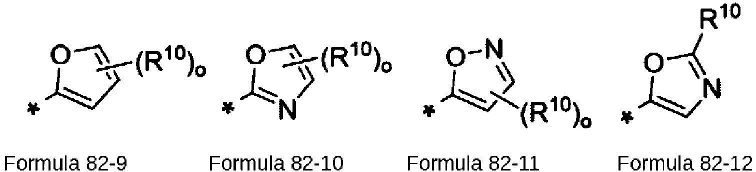

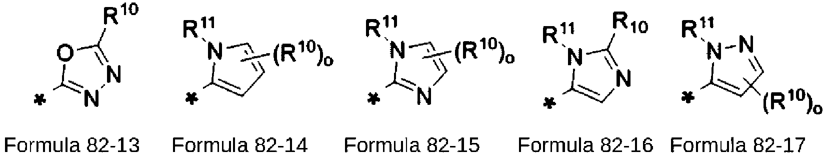

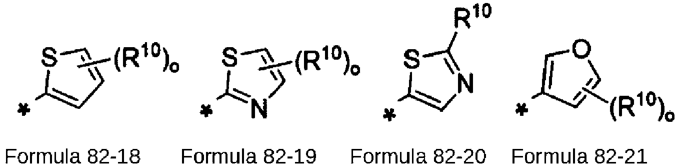

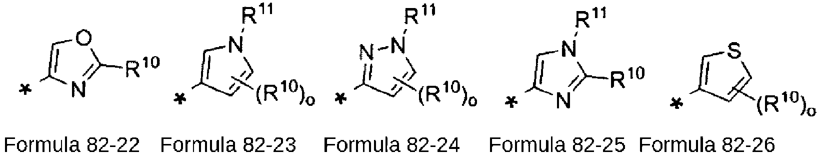

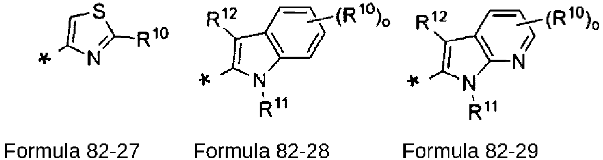

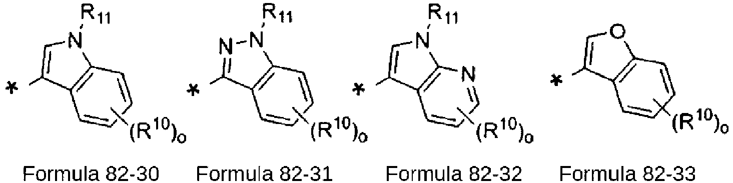

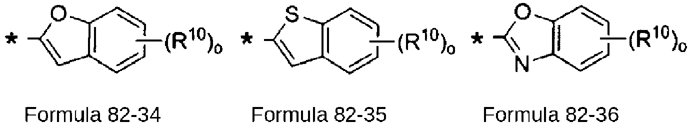

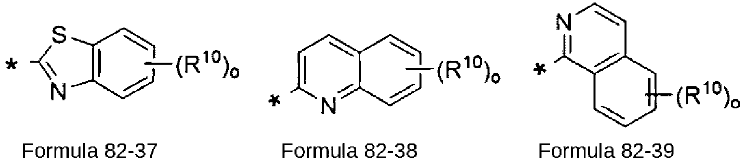

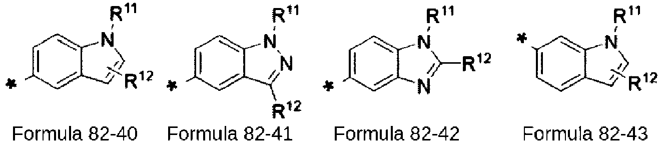

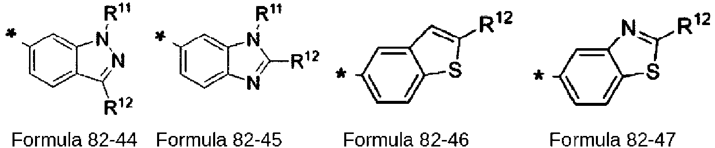

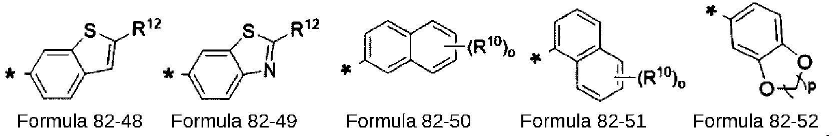

In any one or more of Formulas 82-1 to 82-52,
R⁵, R⁶, R⁷, R⁸, and R⁹ are each independently selected from the group consisting of hydrogen, halogen, a substituted or unsubstituted C₁-C₆ alkyl group, a substituted or unsubstituted C₁-C₆ haloalkyl group, a substituted or unsubstituted C₁-C₆ alkoxy group, a substituted or unsubstituted C₁-C₆ alkylamino group, a substituted or unsubstituted C₁-C₆ alkenyl group, a substituted or unsubstituted C₁-C₆ alkynyl group, -CH₂R³, -OR³, -OCH₂R³, -NR³R⁴, -NH(C=O)R³, -NHSO₂R³, -(C=O)R³, -(C=O)OR³, -(C=O)NR³R⁴, -SO₂NHR³, -CN, -NO₂, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group,
R¹⁰, R¹¹, and R¹² are each independently selected from the group consisting of hydrogen, halogen, -OR³, -NR³R⁴, a substituted or unsubstituted C₁-C₆ alkyl group, a substituted or unsubstituted C₁-C₆ haloalkyl group, a substituted or unsubstituted phenyl group, and a substituted or unsubstituted benzyl group,
each R³ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
each R⁴ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
o is an integer in a range of 0 to 2,
p is an integer in a range of 1 to 3, and
* represents a point of attachment to an adjacent atom.

In an embodiment, the compound may be one wherein, in any one or more of Formulas 78 to 81 (specifically, Formula 81), Z includes one or more selected from the group consisting of groups represented by Formulas 82-1 to 82-5, 82-8 to 82-10, 82-14, 82-15, 82-18, 82-19, 82-28, 82-30, 82-31, 82-33, 82-34, 82-37 to 82-39, 82-44, and 82-49 to 82-52 below:

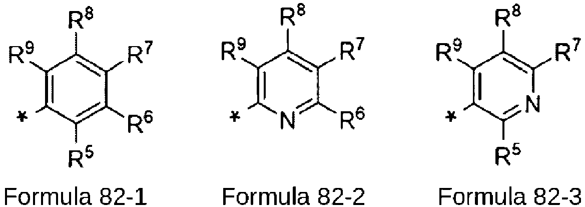

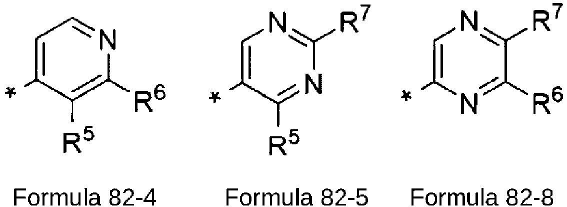

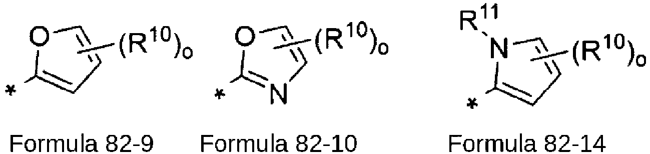

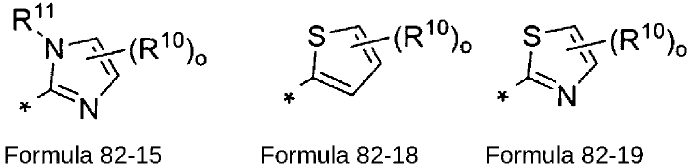

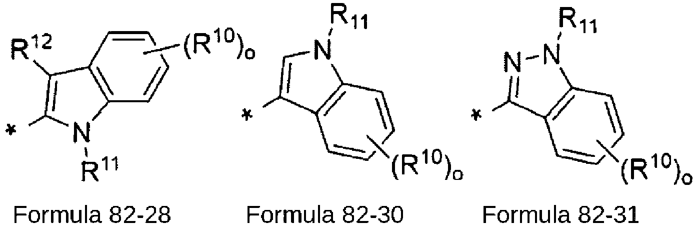

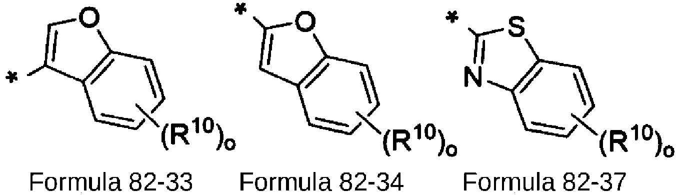

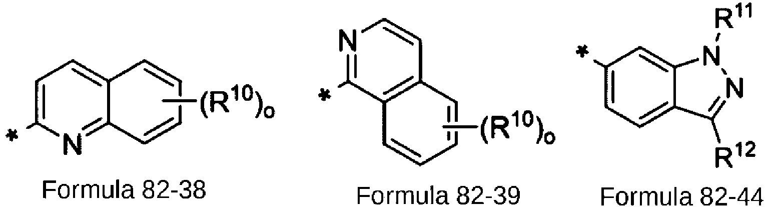

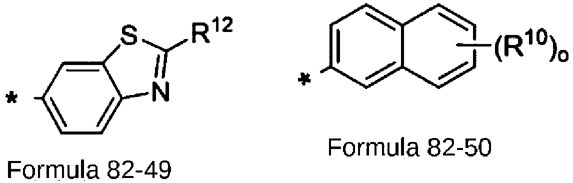

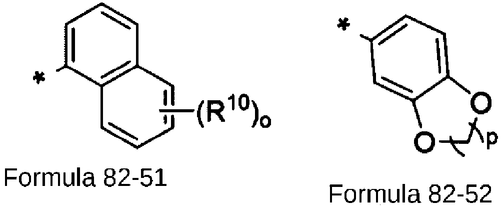

In an embodiment, in any one or more of Formulas 78 to 81 (specifically, Formula 81), Z includes one or more selected from the group consisting of groups represented by Formulas 82-1, 82-14, 82-28, 82-30, 82-31, 82-33, 82-34, 82-49, and 82-52 below:

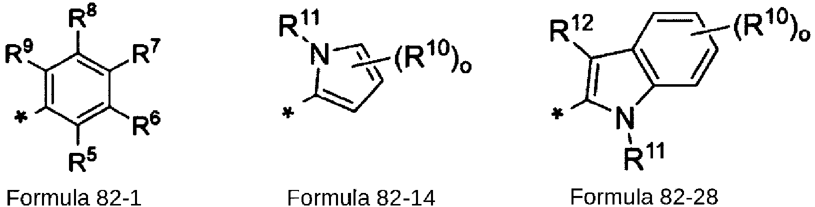

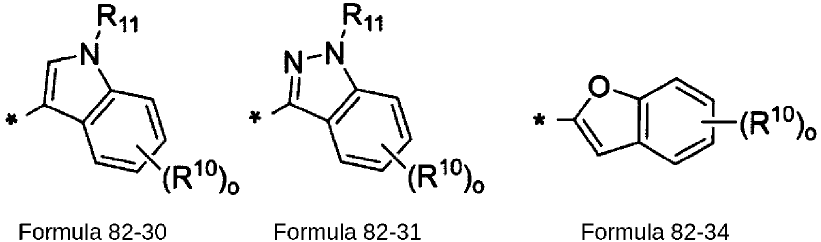

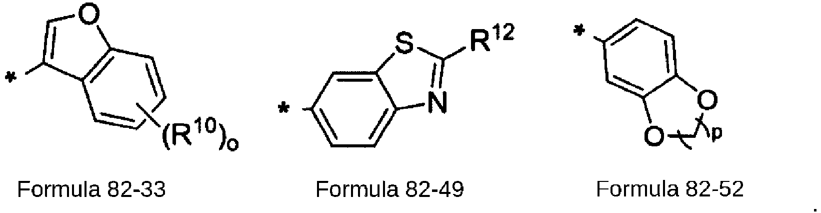

In Formula 82-1, R⁵, R⁸, and R⁹ are hydrogen.

In an embodiment, the compound may be one wherein, in any one or more of Formulas 78 to 81 (specifically, Formula 81), Z includes one or more selected from the group consisting of a substituted or unsubstituted C₁-C₁₀ alkyl group, a substituted or unsubstituted C₃-C₁₂ cycloalkyl group, a substituted or unsubstituted 3- to 12-membered heteroaryl group, and groups represented by Formulas 82-1 to 82-52 below; R⁶ and R⁷ in Formulas 82-1 to 82-52 are each independently selected from the group consisting of hydrogen, halogen, a hydroxy group, a C₁-C₆ alkyl group, a C₁-C₃ haloalkyl group, -CH₂R³, -OR³, -NR³R⁴, a C₃-C₈ cycloalkyl group, a 3- to 8-membered heterocycloalkyl group, a C₆-C₁₀ aryl group, and a 5- to 10-membered heteroaryl group; and o is 0 or 1.

In an embodiment, the compound may be one wherein, in any one or more of Formulas 78 to 81 (specifically, Formula 81), Z includes one or more selected from the group consisting of groups represented by Formulas 82-1, 82-14, 82-28, 82-30, 82-31, 82-33, 82-34, 82-49, and 82-52, and in Formulas 82-1, 82-14, 82-28, 82-30, 82-31, 82-33, 82-34, 82-49, and 82-52, R⁵, R⁶, R⁷, R⁸, R⁹, R¹⁰, R¹¹, and R¹² may each be as follows:
1) R⁵, R⁸, and R⁹ are hydrogen;
2) R⁶ and R⁷ may each independently be selected from the group consisting of hydrogen, halogen, a hydroxy group, a C₁-C₆ alkyl group, a C₁-C₃ haloalkyl group, -CH₂R³, -OR³, -NR³R⁴, a C₃-C₈ cycloalkyl group, a 3- to 8-membered heterocycloalkyl group, a C₆-C₁₀ aryl group, and a 5- to 10-membered heteroaryl group, and specifically, may be selected from the group consisting of hydrogen, halogen, a hydroxy group, methyl, tert-butyl, cyclohexyl, -NH(C₆H₅), and -N(CH₃)₂;
3) R¹⁰ may be selected from the group consisting of hydrogen, halogen, -OR³, - NR³R⁴, a substituted or unsubstituted C₁-C₆ alkyl group, a substituted or unsubstituted C₁-C₆ haloalkyl group, a substituted or unsubstituted phenyl group, and a substituted or unsubstituted benzyl group, and specifically, may be selected from the group consisting of hydrogen, halogen, a phenyl group, and -NHCH₃;
4) R¹¹ may be selected from the group consisting of hydrogen, halogen, -OR³, - NR³R⁴, a substituted or unsubstituted C₁-C₆ alkyl group, a substituted or unsubstituted C₁-C₆ haloalkyl group, a substituted or unsubstituted phenyl group, and a substituted or unsubstituted benzyl group, and specifically, may be selected from the group consisting of hydrogen, a methyl group, and a phenyl group;
5) R¹² may be selected from the group consisting of hydrogen, a halogen, -OR³, - NR³R⁴, a substituted or unsubstituted C₁-C₆ alkyl group, a substituted or unsubstituted C₁-C₆ haloalkyl group, a substituted or unsubstituted phenyl group, and a substituted or unsubstituted benzyl group, and specifically, may be hydrogen;
6) o may be 0, 1, or 2, and specifically, may be 0 or 1; and
7) p may be 1 or 2, and specifically, may be 1.

In an embodiment, the compound may be one wherein, in any one or more of Formulas 78 to 81 (specifically, Formula 81), Z includes a substituted or unsubstituted 6-membered heteroaryl, and the 6-membered heteroaryl may include 1 or 2 nitrogen (N) atoms as ring atoms.

In an embodiment, the compound may be one wherein, in any one or more of Formulas 78 to 81 (specifically, Formula 81), Z includes a substituted or unsubstituted 5-membered heteroaryl, and the 5-membered heteroaryl may include one or more, specifically 1 or 2, selected from the group consisting of nitrogen (N), oxygen (O), and sulfur (S) as ring atoms.

In an embodiment, the compound may be one wherein, in any one or more of Formulas 78 to 81 (specifically, Formula 81), Z includes a substituted or unsubstituted 9-membered heteroaryl, and the 9-membered heteroaryl may include one or more, specifically 1 or 2, selected from the group consisting of nitrogen (N), oxygen (O), and sulfur (S) as ring atoms.

In an embodiment, the compound may be one wherein, in any one or more of Formulas 78 to 81 (specifically, Formula 81), Z includes a substituted or unsubstituted 10-membered heteroaryl, and the 10-membered heteroaryl may include 1 or 2 nitrogen (N) atoms as ring atoms.

In an embodiment, the compound may be one wherein, in any one or more of Formulas 78 to 81 (specifically, Formula 81), Z includes a substituted or unsubstituted C₆-C₁₀ aryl.

In an embodiment, Z may be bonded to a carbon (C) atom of a ketone group of any one or more of Formulas 78 to 81, and specifically, an arbitrary nitrogen (N) atom in Z may be bonded to the carbon (C) atom of the ketone group of said Formulas.

The compound may be one wherein, in any one or more of Formulas 78 to 81 (specifically, Formula 81), Z includes one or more selected from the group consisting of groups represented by Formulas 83-1 to 83-11 below:

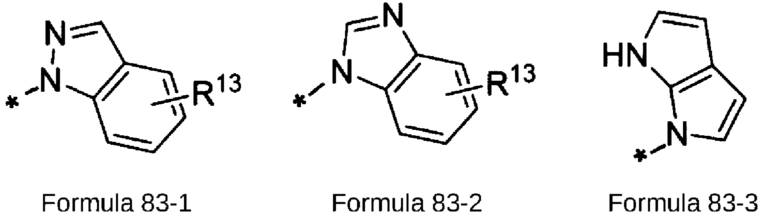

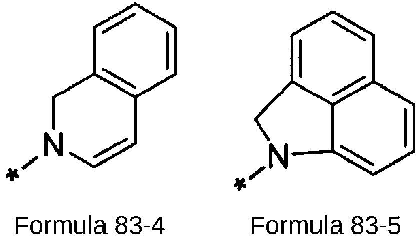

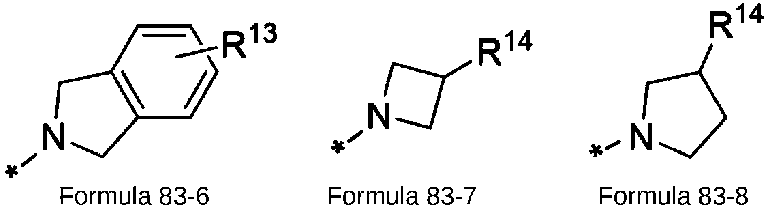

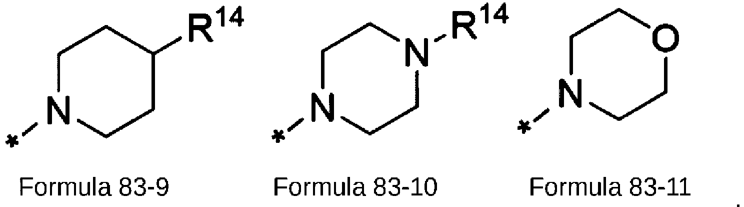

In the formulas,
R¹³ and R¹⁴ are each independently selected from the group consisting of hydrogen, halogen, a substituted or unsubstituted C₁-C₄ alkyl group, -CF₃, -NHR¹⁵, -OR¹⁵, and a substituted or unsubstituted C₃-C₁₂ aryl group,
each R¹⁵ is independently selected from the group consisting of hydrogen, a substituted or unsubstituted C₁-C₄ alkyl group, a substituted or unsubstituted C₁-C₂ haloalkyl group, and a substituted or unsubstituted C₃-C₁₂ aryl group, and
* represents a point of attachment to an adjacent atom.

In an embodiment, in any one or more of Formulas 78 to 81 (specifically, Formula 81), Z includes one or more selected from the group consisting of groups represented by Formulas 83-1, 83-2, 83-5, 83-6, and 83-8 below:

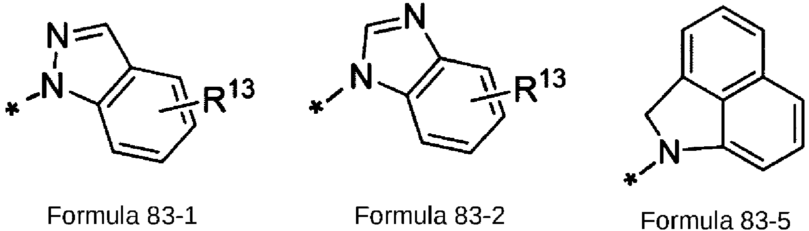

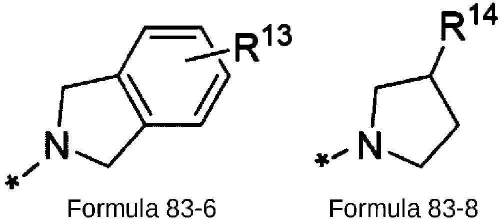

In an embodiment, the compound may include one or more compounds selected from the group consisting of Compounds 1 to 77 listed in Tables 1 to 8. Specifically, Compounds 1 to 77 may be represented by Formulas 1 to 77, respectively, and the formulas of Compounds 1 to 77 are described below.

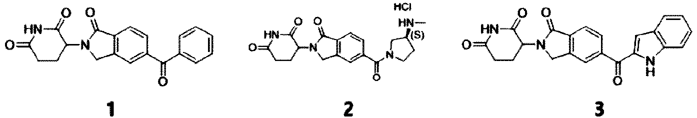

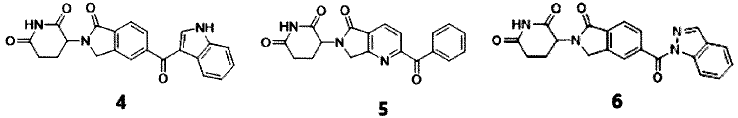

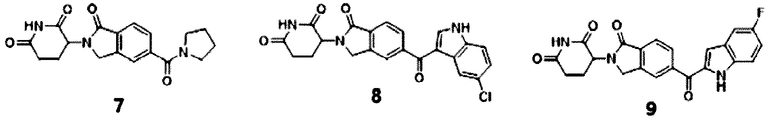

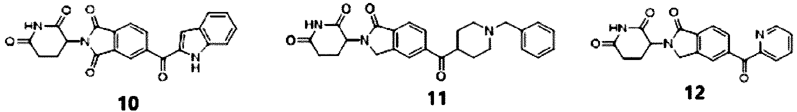

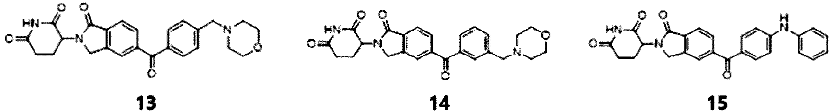

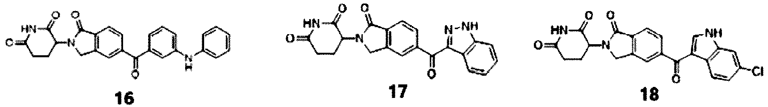

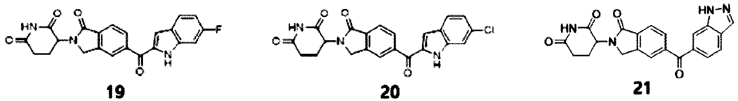

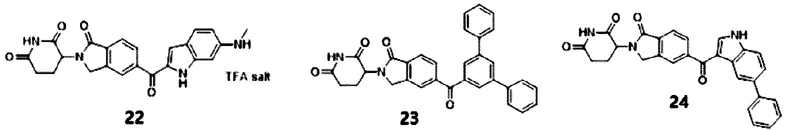

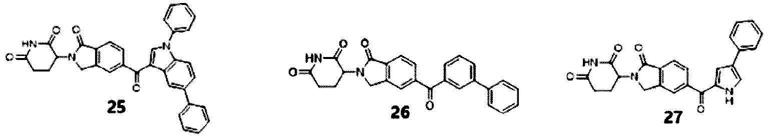

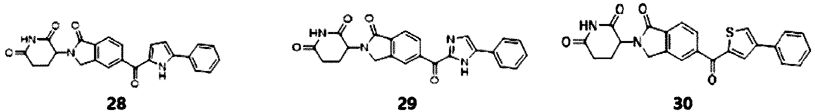

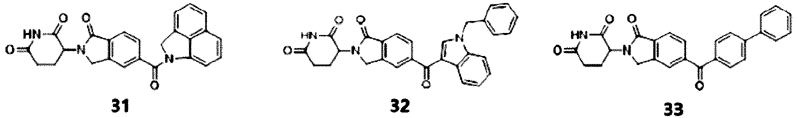

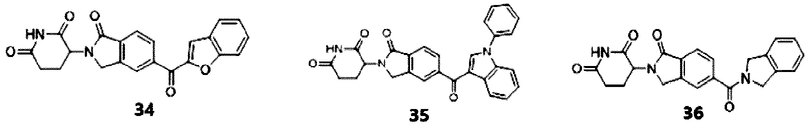

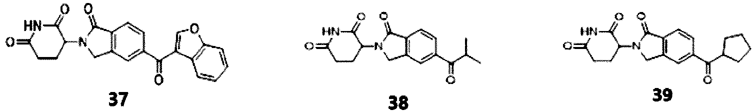

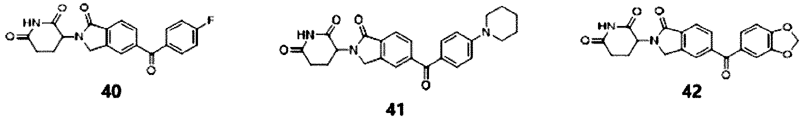

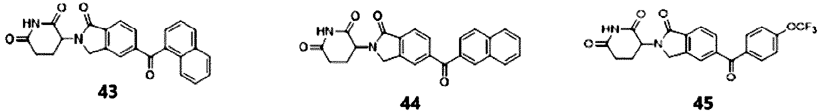

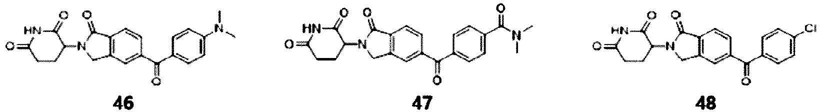

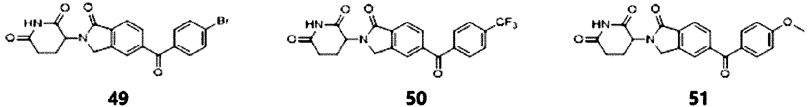

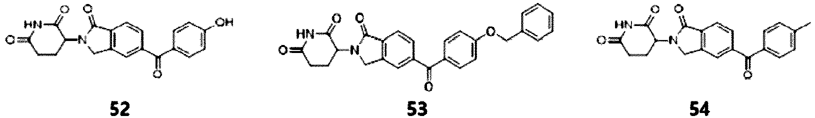

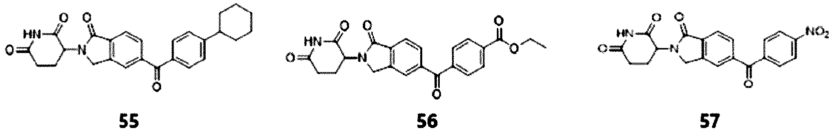

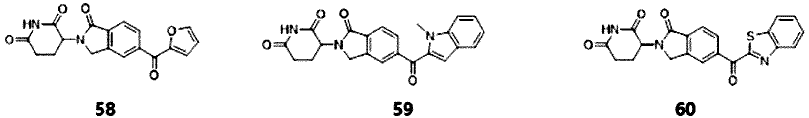

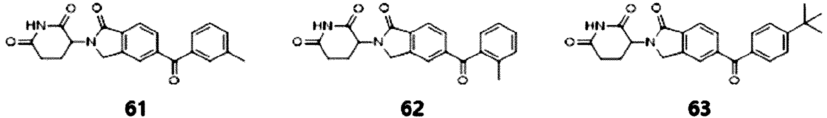

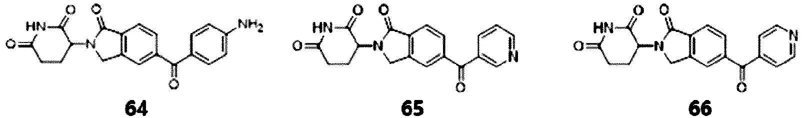

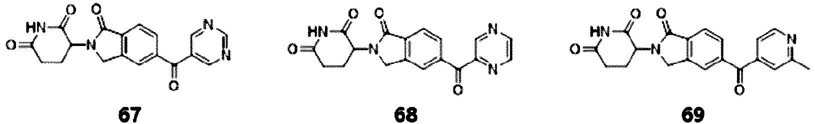

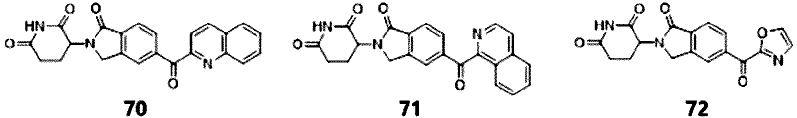

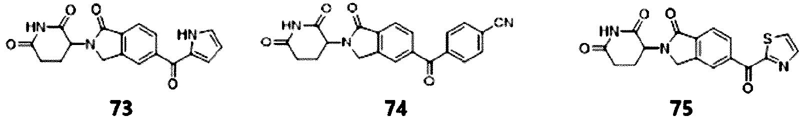

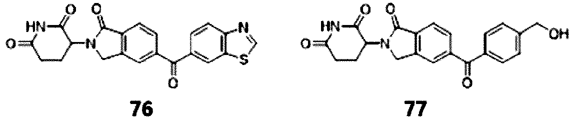

Therefore, the compound, isomer thereof, pharmaceutically acceptable salt thereof, or solvate thereof may include one or more selected from the group consisting of any one or more compounds selected from Formulas 1 to 77, isomer thereof, pharmaceutically acceptable salt thereof, and solvate thereof.

The compound, isomer thereof, pharmaceutically acceptable salt thereof, or solvate thereof may be capable of binding to a Cereblon (CRBN) protein, specifically, may have binding affinity for the Cereblon protein, and more specifically, may have excellent binding affinity for the Cereblon protein.

As used herein, the term "Cereblon (CRBN)" refers to a protein encoded by a human CRBN gene located at a cytogenetic location of 3p26.2 and a molecular location between base pairs 3,148,489 and 3,179,716 on chromosome 3 (UCSC Genome Browser on Human, December 2013 (GRCh38/hg38) assembly). CRBN is a substrate recognition component of a DCX (DDB1-CUL4-X-box) E3 protein ligase complex mediating ubiquitination and subsequent proteasomal degradation of a target protein. The DCX (DDB1-CUL4-X-box) E3 protein ligase complex may consist of at least CRBN, CUL4A, DDB1, and RBX1. The CRBN protein has two isoforms generated by alternative splicing: isoform 1 has 442 amino acids and a molecular weight of 50,546 Da; and isoform 2 has 441 amino acids and a molecular weight of 50,475 Da.

The compound, isomer thereof, pharmaceutically acceptable salt thereof, or solvate thereof may be for modulating an activity of the Cereblon protein or a complex including the Cereblon protein, and specifically, may be for enhancing the activity.

The complex including the Cereblon protein is an E3 ubiquitin ligase complex, and may include a complex including the Cereblon protein and DDB1 (DNA damage-binding protein 1) (CRBN-DDB1 complex); specifically, may include a complex including the Cereblon protein, DBB1, and Cullin-4 (CUL4) (CRBN-DDB1-CUL4 complex); and more specifically, may include a complex including the Cereblon protein, DBB1, CUL4, and Rbx1 (RING-box protein 1) (CRBN-DDB1-CUL4-Rbx1 complex).

The activity of the Cereblon protein or the complex including the Cereblon protein may include an E3 ubiquitin ligase activity, and specifically, may include a degradation activity of the Cereblon protein or the complex including the same toward a target protein.

In an embodiment, the compound, isomer thereof, pharmaceutically acceptable salt thereof, or solvate thereof may bind to a Cereblon protein to enhance degradation activity toward a target protein.

The compound, isomer thereof, pharmaceutically acceptable salt thereof, or solvate thereof may modulate an activity or a degradation level of a GSPT1 (eukaryotic peptide chain release factor GTP-binding subunit ERF3A) protein, and specifically, may induce degradation of the GSPT1 protein to decrease the activity and/or decrease an expression level thereof.

As used herein, the term "GSPT1 (eukaryotic peptide chain release factor GTP-binding subunit ERF3A)" refers to a translation termination factor that binds to eRF1 to mediate termination codon recognition and release of a nascent protein from a ribosome. Ubiquitination and degradation of GSPT1 are known to induce suppression of expression levels of translationally addicted oncoproteins (e.g., c-MYC, N-MYC, L-MYC, BCL-2, MCL-1, etc.) whose expression levels are maintained at high levels in cancer cells a manner dependent on protein translation. In addition, degradation of GSPT1 is known to activate an apoptotic mechanism of cells called an "integrated stress response".

In an embodiment, the compound, isomer thereof, pharmaceutically acceptable salt thereof, or solvate thereof may have a use as a molecular glue, and specifically, may bind to the Cereblon protein to induce degradation of the GSPT1 protein.

In an embodiment, the compound, isomer thereof, pharmaceutically acceptable salt thereof, or solvate thereof may have a use as a GSPT1 degrader, and specifically, may bind to the Cereblon protein to induce degradation of the GSPT1 protein.

In an embodiment, the compound, isomer thereof, pharmaceutically acceptable salt thereof, or solvate thereof may have an efficacy for preventing or treating a proliferative disease.

In an embodiment, the compound, isomer thereof, pharmaceutically acceptable salt thereof, or solvate thereof may have an efficacy for preventing or treating a GSPT1-related disease.

Another aspect provides a pharmaceutical composition for preventing or treating a proliferative disease, including the compound, isomer thereof, pharmaceutically acceptable salt thereof, or solvate thereof. The same details as described above apply *mutatis mutandis* to the composition.

The proliferative disease may include cancer.

In an embodiment, the proliferative disease may include adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen-dependent and independent breast cancer, Burkitt's lymphoma, corpus cancer, carcinoma of unknown primary tumor (CUP-syndrome), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose, and throat tumors, hematologic tumor, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose, and throat regions), colon carcinoma, craniopharyngiomas, oral cancer, cancer of the central nervous system, liver cancer, liver metastases, leukemia, eyelid tumor, lung cancer, lymphomas, stomach cancer, malignant melanoma, malignant neoplasia, malignant tumors of the gastrointestinal tract, breast carcinoma, rectal cancer, medulloblastomas, melanoma, meningiomas, Hodgkin's/non-Hodgkin's lymphomas, mycosis fungoides, nasal cancer, neurinoma, neuroblastoma, kidney cancer, renal cell carcinomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, esophageal cancer, T-cell lymphoma, thymoma, tube carcinoma, eye tumors, urethral cancer, urologic tumors, urothelial carcinoma, vulva cancer, wart appearance, soft tissue tumors, soft tissue sarcoma, nephroblastoma, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypothalamic glioma, cerebellar astrocytoma, cerebral astrocytoma, neuroectodermal tumor, pineal tumors, sarcoma of the uterus, salivary gland cancers, anal gland adenocarcinomas, mast cell tumors, pelvis tumor, ureter tumor, hereditary papillary renal cancers, sporadic papillary renal cancers, intraocular melanoma, hepatocellular carcinoma, cholangiocarcinoma, mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymph sarcoma, rhabdomyosarcoma, malignant histiocytosis, fibroblastic sarcoma, hemangiosarcoma, hemangiopericytoma, leiomyosarcoma (LMS), canine mammary carcinoma, feline mammary carcinoma, and combinations thereof.

The proliferative disease may include a GSPT1-related disease.

The GSPT1-related disease may include a disease caused by or capable of being caused by expression and/or an activity of a GSPT1 protein, and specifically, may include a disease caused by or capable of being caused by overexpression and/or overactivity of the GSPT1 protein.

In an embodiment, the GSPT1-related disease may include acute myeloid leukemia, gastric cancer, colon cancer, liver cancer, breast cancer, and MYC-driven cancers.

The pharmaceutical composition may include a pharmaceutically acceptable carrier. The term "pharmaceutically acceptable carrier" may refer to a carrier or diluent that does not irritate an organism and does not inhibit the biological activity and properties of an administered compound. Here, the term "pharmaceutically acceptable" means that it does not inhibit an activity of an active ingredient and does not possess toxicity beyond what is tolerable to a subject to whom the composition is to be applied (prescribed).

Any carrier conventionally used in the art and pharmaceutically acceptable may be used as the carrier usable in the pharmaceutical composition. Non-limiting examples of the carrier include lactose, dextrose, maltodextrin, sucrose, sorbitol, mannitol, xylitol, erythritol, maltitol, glycerol, ethanol, starch, acacia gum, alginate, gelatin, calcium phosphate, calcium silicate, cellulose, methylcellulose, microcrystalline cellulose, polyvinylpyrrolidone, water, saline, sterile water, Ringer's solution, buffered saline, an albumin injection solution, methyl hydroxybenzoate, propyl hydroxybenzoate, talc, magnesium stearate, mineral oil, and the like. These may be used alone or as a mixture of two or more thereof. The pharmaceutical composition may be formulated into an oral dosage form or a parenteral dosage form depending on a route of administration by a conventional method known in the art, by including a pharmaceutically acceptable carrier in addition to the active ingredient. The pharmaceutical composition may be formulated and used in the form of an oral dosage form such as a powder, a granule, a tablet, a capsule, a suspension, an emulsion, a syrup, an aerosol, or the like, a preparation for external use, a suppository, or a sterile injectable solution according to a conventional method, respectively.

The pharmaceutical composition may be formulated and used in the form of an oral dosage form such as a powder, a granule, a tablet, a capsule, a suspension, an emulsion, a syrup, an aerosol, or the like, a preparation for external use, a suppository, or a sterile injectable solution according to a conventional method, respectively. When the pharmaceutical composition is formulated, the composition may be prepared by adding a diluent or an excipient such as a commonly used filler, extender, binder, wetting agent, disintegrant, or surfactant.

When the pharmaceutical composition is prepared as an oral dosage form, the formulation may be prepared into a dosage form such as a powder, a granule, a tablet, a pill, a dragee, a capsule, a solution, a gel, a syrup, a suspension, a wafer, or the like along with a suitable carrier according to a method known in the art. Here, examples of the suitable pharmaceutically acceptable carrier include saccharides such as lactose, glucose, sucrose, dextrose, sorbitol, mannitol, and xylitol; starches such as corn starch, potato starch, and wheat starch; celluloses such as cellulose, methylcellulose, ethylcellulose, sodium carboxymethylcellulose, and hydroxypropyl methylcellulose; polyvinylpyrrolidone; water; methyl hydroxybenzoate; propyl hydroxybenzoate; magnesium stearate; mineral oil; malt; gelatin; talc; polyols; vegetable oils; and the like. When formulated, the formulation may be prepared to include a diluent and/or an excipient such as a filler, an extender, a binder, a wetting agent, a disintegrant, a surfactant, or the like as needed.

When the pharmaceutical composition is prepared as a parenteral dosage form, the formulation may be prepared in the form of an injection, a transdermal preparation, a nasal inhalant, or a suppository along with a suitable carrier according to a method known in the art. When formulated as an injection, examples of the suitable carrier include sterile water, ethanol, polyols such as glycerol or propylene glycol, or mixtures thereof, and preferably, Ringer's solution, PBS (phosphate-buffered saline) containing triethanolamine, sterile water for injection, an isotonic solution such as 5 % dextrose, or the like may be used. When formulated as a transdermal preparation, the formulation may be prepared in the form of an ointment, a cream, a lotion, a gel, a solution for external use, a paste, a liniment, an aerosol, or the like. In the case of a nasal inhalant, the formulation may be prepared in the form of an aerosol spray using a suitable propellant such as dichlorofluoromethane, trichlorofluoromethane, dichlorotetrafluoroethane, carbon dioxide, or the like, and when formulated as a suppository, Witepsol, Tween 61, polyethylene glycols, cacao butter, laurin butter, polyoxyethylene sorbitan fatty acid esters, polyoxyethylene stearates, sorbitan fatty acid esters, or the like may be used as a base thereof.

The pharmaceutical composition may be administered in a pharmaceutically effective amount, wherein the term "pharmaceutically effective amount" refers to an amount sufficient to treat or prevent a disease at a reasonable benefit/risk ratio applicable to medical treatment or prevention, and an effective dose level may be determined according to factors including the severity of the disease, the activity of the drug, the age, body weight, health, and sex of the patient, the sensitivity of the patient to the drug, the time of administration, the route of administration, and the excretion rate of the composition of the present disclosure used, the duration of treatment, drugs combined or used concurrently with the composition of the present disclosure used, and other factors well known in the medical field. The pharmaceutical composition may be administered alone or in combination with an ingredient known to exhibit a therapeutic effect on a known proliferative disease or GSPT1-related disease. Taking all the above factors into consideration, it is important to administer an amount capable of obtaining a maximum effect with a minimum amount without side effects.

The dosage of the pharmaceutical composition may be determined by a person skilled in the art in consideration of the purpose of use, the severity of the disease, the age, body weight, sex, and medical history of the patient, the type of the substance used as an active ingredient, and the like. For example, the pharmaceutical composition of the present disclosure may be administered at a dose of about 0.1 ng to about 1,000 mg/kg, preferably 1 ng to about 100 mg/kg, per adult, and the administration frequency of the composition of the present disclosure is not particularly limited, but the composition may be administered once a day, or may be administered in divided doses several times a day. The dosage or the administration frequency does not limit the scope of the present application in any way.

Another aspect provides a pharmaceutical composition for preventing or treating a GSPT1-related disease, including the compound, isomer thereof, pharmaceutically acceptable salt thereof, or solvate thereof. The same details as described above apply mutatis mutandis to the composition.

The GSPT1-related disease may include a disease caused by or capable of being caused by expression and/or an activity of a GSPT1 protein, and specifically, may include a disease caused by or capable of being caused by overexpression and/or overactivity of the GSPT1 protein.

In an embodiment, the GSPT1-related disease may include acute myeloid leukemia, gastric cancer, colon cancer, liver cancer, breast cancer, and MYC-driven cancers.

Another aspect provides a method of preventing or treating a proliferative disease or a GSPT1-related disease, the method including administering to a subject the compound, isomer thereof, pharmaceutically acceptable salt thereof, solvate thereof, or a composition including the same. The same details as described above apply *mutatis mutandis* to the method.

As used herein, the term "subject" may include, without limitation, a mammal including a dog, a cat, a mouse, livestock, a human, and the like, a bird, a reptile, a cultured fish, and the like, having or at risk of developing a proliferative disease or a GSPT1-related disease. The subject may exclude a human.

The pharmaceutical composition may be administered in a single dose or multiple doses in a pharmaceutically effective amount. Here, the composition may be formulated and administered in the form of a solution, a powder, an aerosol, an injection, an infusion solution (e.g., Ringer's solution), a capsule, a pill, a tablet, a suppository, or a patch. A route of administration of the pharmaceutical composition may be any general route as long as the composition can reach a target tissue.

The pharmaceutical composition may be administered via a route such as intraperitoneal administration, intravenous administration, intramuscular administration, subcutaneous administration, intradermal administration, transdermal patch administration, oral administration, intranasal administration, intrapulmonary administration, intrarectal administration, or the like, depending on a desired purpose, although not particularly limited thereto. However, for oral administration, the composition may be administered even in an unformulated form; and since an active ingredient of the pharmaceutical composition may be denatured or degraded by gastric acid, an oral composition may be administered into an oral cavity in a form formulated to coat an active drug or to be protected from degradation in the stomach, or in the form of an oral patch. In addition, the composition may be administered by any device capable of delivering an active substance to a target cell.

Another aspect provides a use of the compound, isomer thereof, pharmaceutically acceptable salt thereof, or solvate thereof for preventing or treating a proliferative disease or a GSPT1-related disease. The same details as described above apply mutatis mutandis to the use.

Another aspect provides a use of the compound, isomer thereof, pharmaceutically acceptable salt thereof, or solvate thereof in the manufacture of a medicament for preventing or treating a proliferative disease or a GSPT1-related disease. The same details as described above apply mutatis mutandis to the use.

Another aspect provides a pharmaceutical composition including the compound, isomer thereof, pharmaceutically acceptable salt thereof, or solvate thereof. The same details as described above apply mutatis mutandis to the composition.

Another aspect provides a GSPT1 degrader including the compound, isomer thereof, pharmaceutically acceptable salt thereof, or solvate thereof. The same details as described above apply mutatis mutandis to the degrader.

In an embodiment, the compound, isomer thereof, pharmaceutically acceptable salt thereof, or solvate thereof may modulate an activity of a Cereblon protein or a complex including the same to degrade a GSPT1 protein or decrease an expression level thereof.

### Advantageous Effects of Invention

The novel compound, isomer thereof, pharmaceutically acceptable salt thereof, or solvate thereof of the present disclosure has excellent binding affinity for Cereblon, and thus can effectively degrade GSPT1 through the E3 ubiquitin ligase activity of the Cereblon complex, thereby effectively treating diseases such as proliferative diseases.

### Brief Description of Drawings

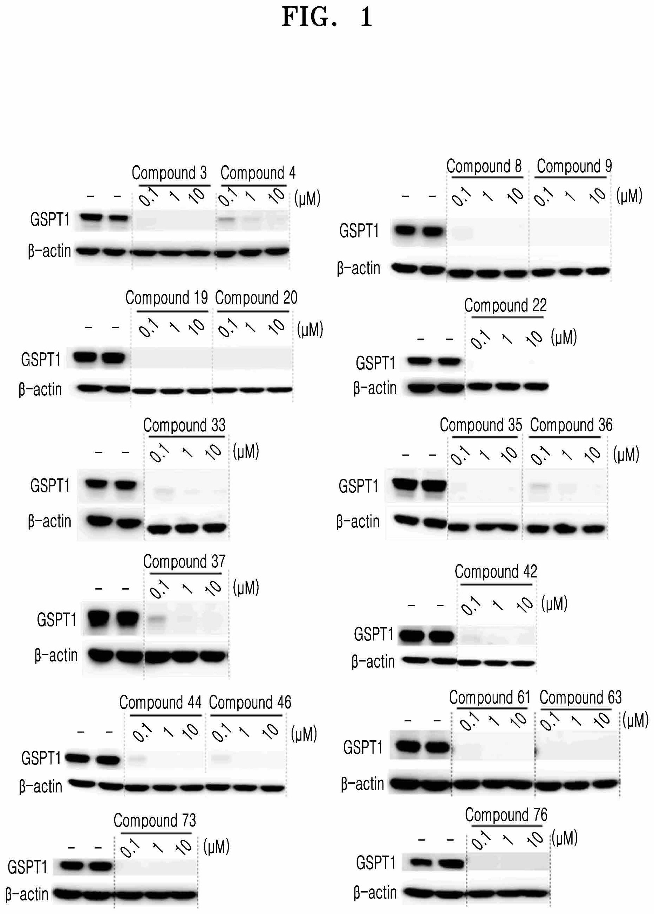

FIG. 1 shows results of Western blotting analysis confirming GSPT1 degradation activity of novel compounds of the present disclosure.

### Best Mode for Carrying out the Invention

### Mode for the Invention

Hereinafter, the present disclosure will be described in more detail through examples. However, these examples are provided for illustrative purposes, and the scope of the present disclosure is not limited to these examples.

### Preparation Example 1: Synthesis of Compound 1

### 1.1: Synthesis of Compound A2

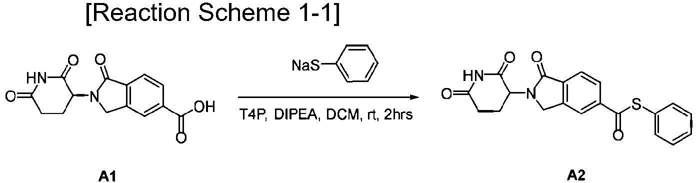

To a solution of 2-(2,6-dioxopiperidin-3-yl)-1-oxoisoindoline-5-carboxylic acid (A1, 4 g, 0.014 mol) in DCM (100 mL) were added sodium benzenethiolate (1.84 g, 0.014 mol), DIPEA (3.59 g, 0.028 mol), and 2,4,6-tributyl-1,3,5,2,4,6-trioxatriphosphorinane 2,4,6-trioxide (T4P, 50 wt% in EtOAc, 30.05 g, 0.042 mol) at room temperature. The reaction mixture was stirred at room temperature for 2 hours. The mixture was concentrated under reduced pressure. The residue was purified by flash chromatography (DCM/MeOH = 100:0 to 90:10) to give A2 (3.8 g, yield 64 %) as a white solid.

### 1.2: Synthesis of Compound 1

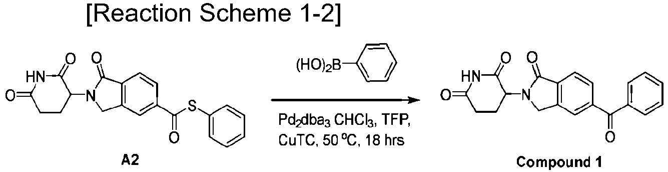

A mixture of Compound A2 (50 mg, 0.1314 mmol), phenylboronic acid (19 mg, 0.16 mmol), a Pd₂(dba)₃-chloroform adduct (13 mg, 0.013 mmol), tri(2-furyl)phosphine (TFP, 6.1 mg, 0.026 mmol), and copper(I) thiophene-2-carboxylate (CuTC, 38 mg, 0.20 mmol) in THF (3 mL) was stirred at 50 °C for 18 hours under an N₂ atmosphere. The mixture was diluted with water (5 mL) and extracted with EtOAc (5 mL × 3). The combined organic layers were dried over Na₂SO₄, filtered, and concentrated. The residue was purified by silica gel chromatography (DCM:MeOH = 97 %:3 %) and washed with MeOH to give Compound 1 (18 mg, yield 39 %) as a white solid.

### Preparation Example 2: Synthesis of Compound 2

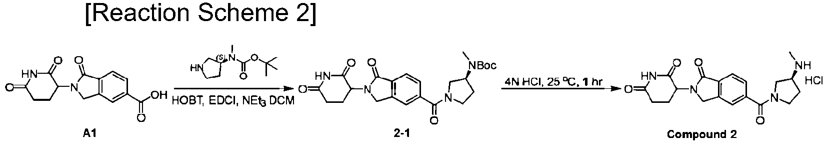

### 2-1: Synthesis of Compound 2-1

To a suspension of 2-(2,6-dioxopiperidin-3-yl)-1-oxoisoindoline-5-carboxylic acid (A1, 100 mg, 0.35 mmol), *tert-*butyl (*S*)-methyl(pyrrolidin-3-yl)carbamate (104 mg, 0.52 mmol), 1-hydroxybenzotriazole (56 mg, 0.42 mmol), and NEt₃ (88 mg, 0.42 mmol) in DCM (5 mL) was slowly added a suspension of EDCI (80 mg, 0.42 mmol) in DCM (3 mL) at 0 °C. The reaction mixture was stirred at 25 °C for 18 hours. The mixture was diluted with water (5 mL) and extracted with EtOAc (5 mL × 3). The combined organic layers were dried over Na₂SO₄, filtered, and concentrated. The residue was purified by silica gel chromatography (DCM:MeOH = 97 %:3 %) and washed with MeOH to give Compound 2-1 (100 mg, yield 55 %) as a white solid.

### 2-2: Synthesis of Compound 2

To a solution of Compound 2-1 (100 mg, 0.2125 mmol) in MeOH (2 mL) was added HCl (2 mL, 4 N in dioxane), and the mixture was stirred at 25 °C for 1 hour. The mixture was dried under a nitrogen atmosphere. The resulting solid was directly purified by prep-HPLC (column: Phenomenex Luna C18 250 mm × 100 mm × 10 µm; mobile phase: [H₂O (0.1 % HCl)/MeCN = 0 % - 50 %]) to give Compound 2 (12 mg, yield 14 %) as a white solid.

### Preparation Example 3: Synthesis of Compound 3

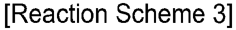

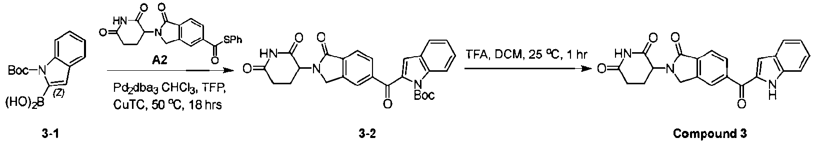

### 3-1: Synthesis of Compound 3-2

(1-(*tert*-Butoxycarbonyl)-1*H*-indol-2-yl)boronic acid (3-1, 38 mg, 0.14 mmol) was subjected to the same synthetic procedure as for Compound 1 to give Compound 3-2 (20 mg, yield 28 %) as a brown solid.

### 3-2: Synthesis of Compound 3

To a solution of Compound 3-2 (20 mg, 0.04 mmol) in DCM (1 mL) was added TFA (1 mL), and the mixture was stirred at 25 °C for 1 hour. The solvent in this solution was removed under a nitrogen atmosphere, and the pH of the mixture was adjusted to 7 with a saturated aqueous NaHCO₃ solution. The aqueous solution was extracted with DCM (5 mL × 3), and the organic layer was concentrated to give a crude product. The crude product was then washed with MeOH, and the solid was collected by centrifugation to give Compound 3 (12 mg, yield 71 %) as a pale white solid.

### Preparation Example 4: Synthesis of Compound 4

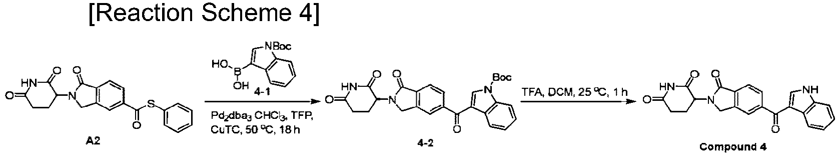

### 4-1: Synthesis of Compound 4-2

(1-(*tert*-Butoxycarbonyl)-1*H*-indol-3-yl)boronic acid (4-1, 76 mg, 0.29 mmol) was subjected to the same synthetic procedure as for Compound 1 to give Compound 4-2 (60 mg, yield 42 %) as a white solid.

### 4-2: Synthesis of Compound 4

Compound 4-2 (49 mg, 0.10 mmol) was subjected to the same synthetic procedure as for Compound 3 to give Compound 4 (20 mg, yield 49 %) as a white solid.

### Preparation Example 5: Synthesis of Compound 5

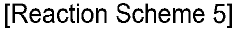

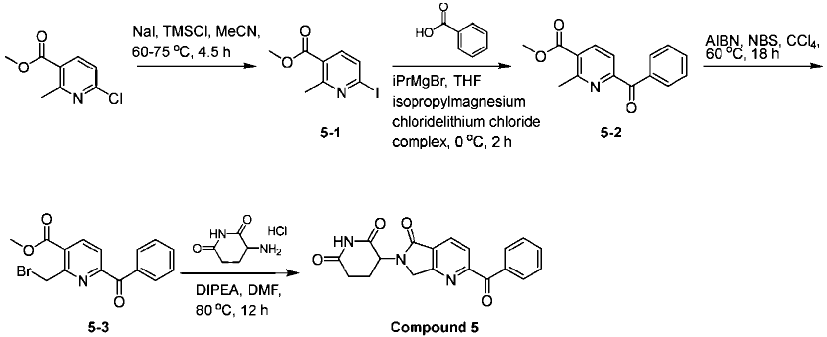

### 5-1: Synthesis of Compound 5-1

To a solution of methyl 6-chloro-2-methylnicotinate (1 g, 0.005 mol) in MeCN (15 mL) were added NaI (2.43 g, 0.016 mol) and TMSCI (0.59 g, 0.005 mol) at 25 °C under an N₂ atmosphere. The mixture was stirred at 75 °C for 4.5 hours. After the reaction was completed, the solution was cooled to room temperature, diluted with water (20 mL), and extracted with EtOAc (10 mL × 3). The combined organic layers were washed with brine, dried over anhydrous sodium sulfate, and concentrated. The residue was purified by silica gel column chromatography (EtOAc/PE, 5 % to 10 %) to give methyl 6-iodo-2-methylnicotinate (Compound 5-1, 1 g, yield 59 %) as a yellow solid.

### 5-2: Synthesis of Compound 5-2

To a solution of benzoic acid (340 mg, 2.78 mmol) in THF (10 mL) were added *i*PrMgBr (431 mg, 2.92 mmol), Compound 5-1 (849 mg, 3.06 mmol), and an isopropylmagnesium chloride-lithium chloride complex (485 mg, 3.34 mmol) at 0 °C. The reaction mixture was stirred at 0 °C for 2 hours. After the reaction was completed, the reaction was quenched with aq. NH₄Cl (20 mL), and the mixture was extracted with EtOAc (20 mL × 3). The combined organic layers were washed with brine, dried over anhydrous sodium sulfate, and concentrated. The residue was purified by silica gel column chromatography (EtOAc/PE, 0 % to 10 %) to give Compound 5-2 (160 mg, yield 20 %) as a yellow solid.

### 5-3: Synthesis of Compound 5-3

To a solution of Compound 5-2 (200 mg, 0.78 mmol) in CCl₄ (10 mL) were added NBS (167 mg, 0.94 mmol) and AIBN (64 mg, 0.39 mmol) at room temperature under an N₂ atmosphere. The mixture was stirred at 60 °C for 18 hours. After the reaction was completed, the mixture was diluted with water (20 mL) and extracted with EtOAc (10 mL × 3). The combined organic layers were washed with brine, dried over anhydrous sodium sulfate, and concentrated. The residue was purified by silica gel column chromatography (EtOAc/PE, 5 % to 10 %) to give Compound 5-3 (110 mg, yield 43 %) as a yellow solid.

### 5-4: Synthesis of Compound 5

To a solution of 3-aminopiperidine-2,6-dione hydrochloride (108 mg, 0.65 mmol) in DMF (5 mL) was added DIPEA (213 mg, 1.64 mmol) at room temperature under an N₂ atmosphere. The mixture was stirred at room temperature for 30 minutes. Then, Compound 5-3 (110 mg, 0.33 mmol) was added to the solution, and the mixture was stirred at 80 °C for 12 hours. After the reaction was completed, the mixture was diluted with water (20 mL) and extracted with EtOAc (10 mL × 3). The combined organic layers were washed with brine, dried over anhydrous sodium sulfate, and concentrated. The residue was purified by silica gel column chromatography (MeOH/DCM, 5 % to 10 %) to give a crude product. The crude product was washed with MeOH to give Compound 5 (11.95 mg, yield 10 %) as a white solid.

### Preparation Example 6: Synthesis of Compound 6

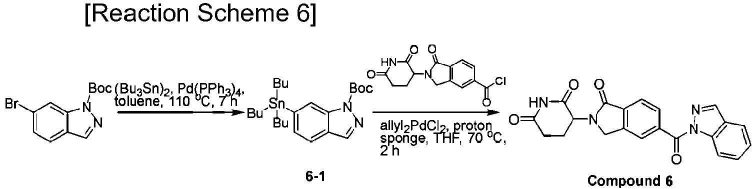

### 6-1: Synthesis of Compound 6-1

A solution of *tert-*butyl 6-bromo-1*H*-indazole-1-carboxylate (0.5 g, 1.69 mmol), Pd(PPh₃)₄ (0.14 g, 0.17 mmol), and (Bu₃Sn)₂ (1.18 g, 2.03 mmol) in toluene (5 mL) was stirred at 110 °C for 7 hours under an N₂ atmosphere. After the reaction was completed, the mixture was diluted with water (5 mL) and extracted with EtOAc (5 mL × 3). The combined organic layers were dried over Na₂SO₄, filtered, and concentrated. The residue was purified by silica gel chromatography (EtOAc/PE = 5 %) to give Compound 6-1 (0.4 g, yield 41 %) as a yellow liquid.

### 6-2: Synthesis of Compound 6

To a solution of Compound 6-1 (167 mg, 0.33 mmol) and 1,8-bis(dimethylamino)naphthalene (35 mg, 0.16 mmol) in THF (5 mL) was added 2-(2,6-dioxopiperidin-3-yl)-1-oxoisoindoline-5-carbonyl chloride (100 mg, 0.33 mmol). After 15 minutes, an allylpalladium chloride dimer (allyl₂PdCl₂, 10 mg, 0.03 mmol) was added. The reaction mixture was stirred at room temperature for 5 minutes and then at 70 °C for 2 hours. After confirming that the reaction proceeded by LC-MS, the mixture was diluted with water (5 mL) and extracted with EtOAc (5 mL × 3). The combined organic layers were dried over Na₂SO₄, filtered, and concentrated. The residue was purified by silica gel chromatography (MeOH/DCM = 5 %) to give a crude product. The crude product was purified by prep-HPLC (column: Phenomenex Luna C18 250 mm × 100 mm × 10 µm; mobile phase: [H₂O (0.05 % FA)-ACN]; B %: 20 % to 55 %, 35 minutes) to give Compound 6 (10.08 mg, yield 7.6 %) as a white solid.

### Preparation Example 7: Synthesis of Compound 7

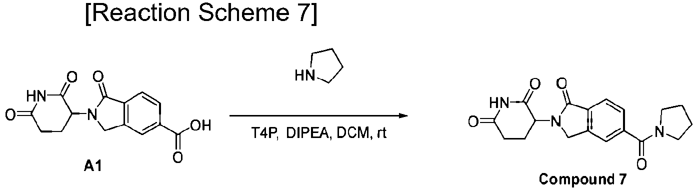

A solution of 2-(2,6-dioxopiperidin-3-yl)-1-oxoisoindoline-5-carboxylic acid (Compound A1, 50 mg, 0.17 mmol), pyrrolidine (20 mg, 0.28 mmol), DIPEA (112 mg, 0.87 mmol), and 2,4,6-tributyl-1,3,5,2,4,6-trioxatriphosphorinane 2,4,6-trioxide (T4P, 50 wt% in EtOAc, 250 mg, 0.35 mmol) in DCM (5 mL) was stirred at room temperature for 1 hour. The mixture was diluted with water (15 mL) and extracted with DCM (20 mL × 3). The combined organic layers were dried over Na₂SO₄, filtered, and concentrated. The residue was purified by silica gel chromatography (DCM:MeOH = 100:0 to 97:3) and triturated with methyl *tert-*butyl ether (MTBE) to give Compound 7 (22.47 mg, yield 37 %) as a white solid.

### Preparation Example 8: Synthesis of Compound 8

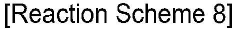

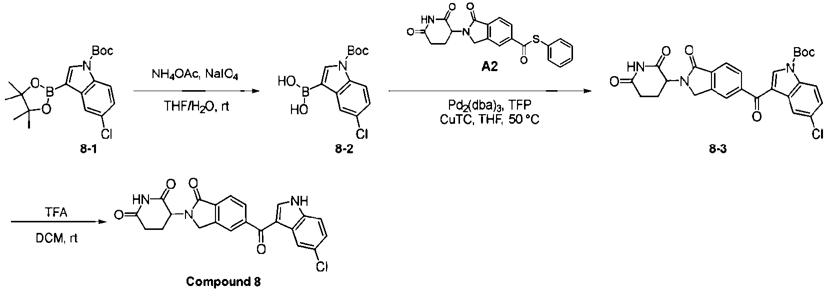

### 8-1: Synthesis of Compound 8-2

To a solution of tert-butyl 5-chloro-3-(4,4,5,5-tetramethyl-1,3,2-dioxaborolan-2-yl)-1H-indole-1-carboxylate (Compound 8-1, 280 mg, 0.74 mmol) in THF/H₂O (1:1, 30 mL) were added NaIO₄ (475 mg, 2.22 mmol) and NH₄OAc (171 mg, 2.22 mmol). The mixture was stirred at room temperature for 12 hours. The mixture was diluted with water (100 mL) and extracted with EtOAc (100 mL × 3). The combined organic layers were washed with brine (100 mL × 2), dried over Na₂SO₄, filtered, and concentrated under reduced pressure. The residue was purified by flash column chromatography (PE/EtOAc = 100:0 to 50:50) to give Compound 8-2 (180 mg, yield 73 %) as a white solid.

### 8-2: Synthesis of Compound 8-3

Compound 8-2 (180 mg, 0.61 mmol) was subjected to the same synthetic procedure as for Compound 1 to give Compound 8-3 (80 mg, yield 17 %) as a yellow liquid.

### 8-3: Synthesis of Compound 8

Compound 8-3 (80 mg, 0.15 mmol) was subjected to the same synthetic procedure as for Compound 3 to give Compound 8 (28.13 mg, yield 43 %) as a white solid.

### Preparation Example 9: Synthesis of Compound 9

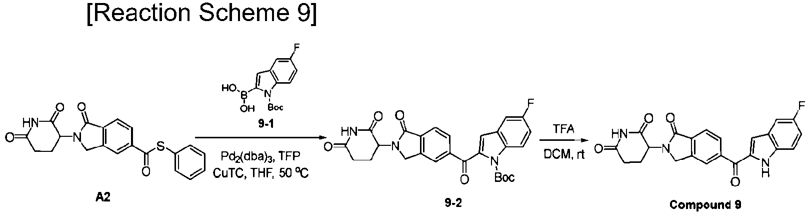

### 9-1: Synthesis of Compound 9-2

(1-(tert-Butoxycarbonyl)-5-fluoro-1H-indol-2-yl)boronic acid (Compound 9-1, 97 mg, 0.35 mmol) was subjected to the same synthetic procedure as for Compound 1 to give Compound 9-2 (25 mg, yield 16 %) as a white solid.

### 9-2: Synthesis of Compound 9

Compound 9-2 (25 mg, 0.050 mmol) was subjected to the same synthetic procedure as for Compound 3 to give Compound 9 (10.65 mg, yield 53 %) as a white solid.

### Preparation Example 10: Synthesis of Compound 10

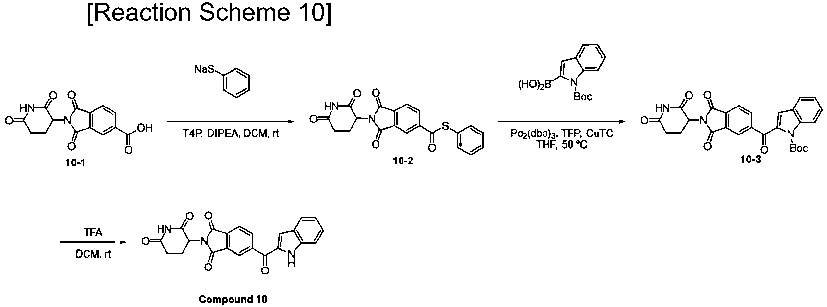

### 10-1: Synthesis of Compound 10-2

2-(2,6-Dioxopiperidin-3-yl)-1,3-dioxoisoindoline-5-carboxylicacid (Compound 10-1, 520 mg, 1.72 mmol) was subjected to the same synthetic procedure as for Compound A2 to give Compound 10-2 (360 mg, yield 74 %) as a yellow solid.

### 10-2: Synthesis of Compound 10-3

Compound 10-2 (220 mg, 0.56 mmol) and (1-(tert-butoxycarbonyl)-1H-indol-2-yl)boronic acid (219 mg, 0.84 mmol) were subjected to the same synthetic procedure as for Compound 1 to give Compound 10-3 (180 mg, yield 44 %) as a brown solid.

### 10-3: Synthesis of Compound 10

Compound 10-3 (180 mg, 0.36 mmol) was subjected to the same synthetic procedure as for Compound 3 to give Compound 10 (15.24 mg, yield 10 %) as a yellow solid.

### Preparation Example 11: Synthesis of Compound 11

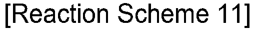

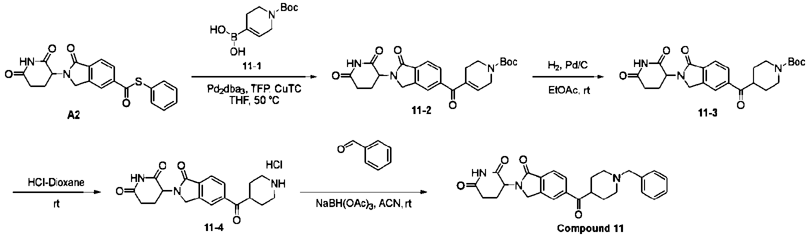

### 11-1: Synthesis of Compound 11-2

(1-(tert-Butoxycarbonyl)-1,2,3,6-tetrahydropyridin-4-yl)boronic acid (Compound 11-1, 252 mg, 1.10 mmol) was subjected to the same synthetic procedure as for Compound 1 to give Compound 11-2 (180 mg, yield 64 %) as a yellow solid.

### 11-2: Synthesis of Compound 11-3

To a solution of Compound 11-2 (180 mg, 0.40 mmol) in EtOAc (10 mL) was added PtO₂ (18 mg). The mixture was evacuated and backfilled with hydrogen three times, then backfilled with hydrogen again. The mixture was stirred at room temperature for 3 hours. The mixture was then filtered through Celite and concentrated, and the residue was purified by flash chromatography (DCM/MeOH = 100:0 to 97:3) to give Compound 11-3 (120 mg, yield 59 %) as a yellow solid.

### 11-3: Synthesis of Compound 11-4

A solution of Compound 11-3 (120 mg, 0.26 mmol) in HCl in dioxane (4 N, 5 mL) was stirred at room temperature for 10 minutes. The mixture was concentrated under reduced pressure to give Compound 11-4 (100 mg, yield 77 %) as a yellow solid.

### 11-4: Synthesis of Compound 11

To a solution of Compound 11-4 (100 mg, 0.26 mmol) in ACN (5 mL) were added benzaldehyde (81 mg, 0.77 mmol) and NaBH(OAc)₃ (108 mg, 0.51 mmol) at room temperature. The reaction mixture was stirred at room temperature for 10 minutes. The mixture was quenched with water and concentrated under reduced pressure. The residue was purified by flash chromatography (DCM/MeOH = 100:0 to 97:3) and prep-HPLC (Gemini 5 µm C18 150 × 21.2 mm; mobile phase: ACN-H₂O (0.1 % FA), 40-60 %), and triturated with MeOH to give Compound 11 (10.54 mg, yield 8 %) as a white solid.

### Preparation Example 12: Synthesis of Compound 12

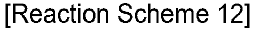

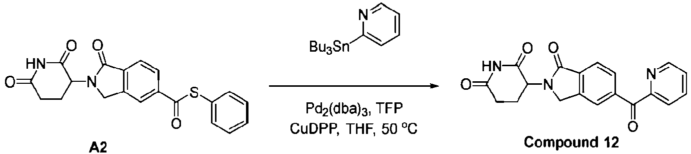

To a solution of 2-(tributylstannyl)pyridine (145 mg, 0.39 mmol) and Compound A2 (100 mg, 0.26 mmol) in THF (10 mL) were added Pd₂(dba)₃ (24 mg, 0.026 mmol), tri(2-furyl)phosphine (TFP, 12 mg, 0.052 mmol), and copper(I) diphenylphosphinate (CuDPP, 163 mg, 0.58 mmol). The reaction mixture was stirred at 50 °C for 1 hour. The mixture was cooled to room temperature and concentrated under reduced pressure. The residue was purified by flash chromatography (DCM/MeOH = 100:0 to 97:3) and prep-HPLC (Gemini 5 µm C18 150 × 21.2 mm; mobile phase: MeCN-H₂O (0.1 % FA), 30-50 %) to give Compound 12 (14.17 mg, yield 15 %) as a white solid.

### Preparation Example 13: Synthesis of Compound 13

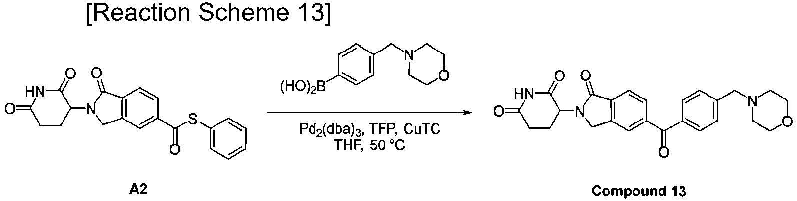

(4-(Morpholinomethyl)phenyl)boronic acid (105 mg, 0.47 mmol) was subjected to the same synthetic procedure as for Compound 1 to give Compound 13 (10.45 mg, yield 9 %) as a pale yellow solid.

### Preparation Example 14: Synthesis of Compound 14

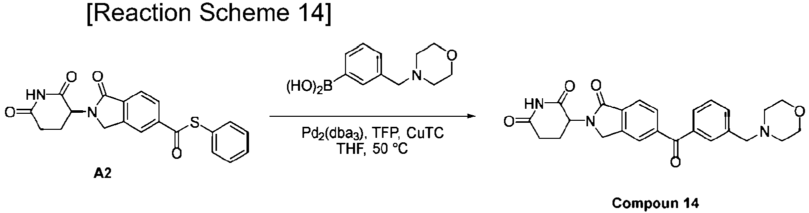

(3-(Morpholinomethyl)phenyl)boronic acid (203 mg, 0.92 mmol) was subjected to the same synthetic procedure as for Compound 1 to give Compound 14 (10.97 mg, yield 6 %) as a white solid.

### Preparation Example 15: Synthesis of Compound 15

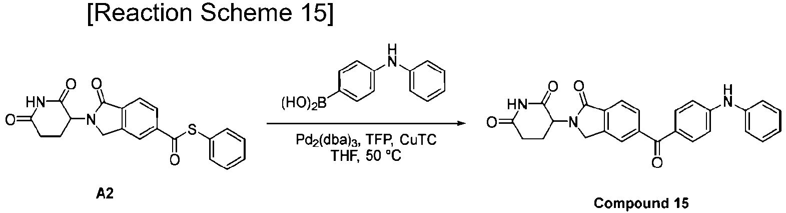

(4-(Phenylamino)phenyl)boronic acid (180 mg, 0.84 mmol) was subjected to the same synthetic procedure as for Compound 1 to give Compound 15 (13.07 mg, yield 7 %) as a yellow solid.

### Preparation Example 16: Synthesis of Compound 16

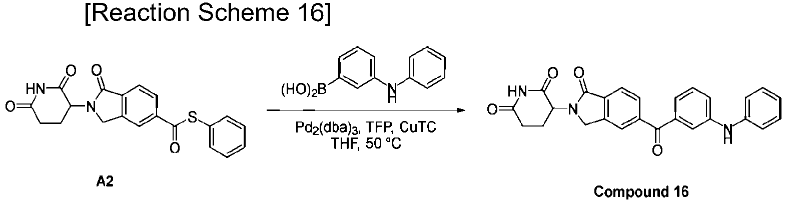

(3-(Phenylamino)phenyl)boronic acid (92 mg, 0.43 mmol) was subjected to the same synthetic procedure as for Compound 1 to give Compound 16 (10.9 mg, yield 8 %) as a yellow solid.

### Preparation Example 17: Synthesis of Compound 17

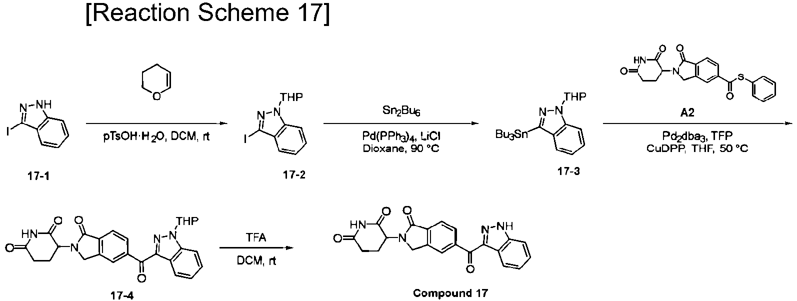

### 17-1: Synthesis of Compound 17-2

To a solution of 3-iodo-1H-indazole (17-1, 500 mg, 2.05 mmol) and 3,4-dihydro-2H-pyran (207 mg, 2.46 mmol) in DCM (20 mL) was added p-TsOH·H₂O (39 mg, 0.20 mmol) at room temperature. The reaction mixture was stirred at room temperature for 2 hours. The mixture was concentrated under reduced pressure, and the residue was purified by flash column chromatography (PE/EtOAc = 100:0 to 60:40) to give Compound 17-2 (600 mg, yield 80 %) as a white solid.

### 17-2: Synthesis of Compound 17-3

To a solution of Compound 17-2 (600 mg, 1.83 mmol) and 1,1,1,2,2,2-hexabutyldistannane (1.6 g, 2.74 mmol) in dioxane (15 mL) were added Pd(PPh₃)₄ (211 mg, 0.18 mmol) and LiCl (155 mg, 3.66 mmol) at room temperature. The reaction mixture was stirred at 90 °C for 3 hours. The mixture was cooled to room temperature and concentrated under reduced pressure. The residue was purified by flash chromatography (PE/EtOAc = 100:0 to 90:10) to give Compound 17-3 (380 mg, yield 38 %) as a yellow solid.

### 17-3: Synthesis of Compound 17-4

Compound 17-3 (336 mg, 0.68 mmol) was subjected to the same synthetic procedure as for Compound 13 to give Compound 17-4 (150 mg, yield 51 %) as a brown solid.

### 17-4: Synthesis of Compound 17

Compound 17-4 (150 mg, 0.32 mmol) was subjected to the same synthetic procedure as for Compound 3 to give Compound 17 (19.93 mg, yield 15 %) as a white solid.

### Preparation Example 18: Synthesis of Compound 18

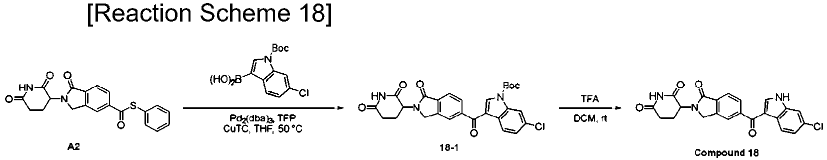

### 18-1: Synthesis of Compound 18-1

(1-(tert-Butoxycarbonyl)-6-chloro-1H-indol-3-yl)boronic acid (100 mg, 0.34 mmol) was subjected to the same synthetic procedure as for Compound 1 to give Compound 18-1 (100 mg, yield 51 %) as a white solid.

### 18-2: Synthesis of Compound 18

Compound 18-1 (100 mg, 0.19 mmol) was subjected to the same synthetic procedure as for Compound 3 to give Compound 18 (15.16 mg, yield 18 %) as a white solid.

### Preparation Example 19: Synthesis of Compound 19

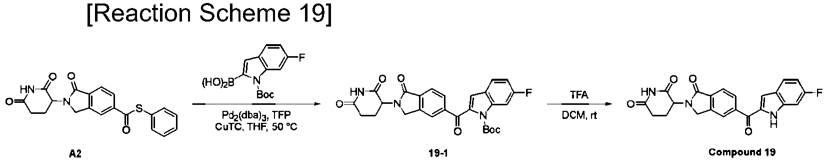

### 19-1: Synthesis of Compound 19-1

(1-(tert-Butoxycarbonyl)-6-fluoro-1H-indol-2-yl)boronic acid (110 mg, 0.39 mmol) was subjected to the same synthetic procedure as for Compound 1 to give Compound 19-1 (40 mg, yield 27 %) as a white solid.

### 19-2: Synthesis of Compound 19

Compound 19-1 (40 mg, 0.079 mmol) was subjected to the same synthetic procedure as for Compound 3 to give Compound 19 (9.49 mg, yield 29 %) as a yellow solid.

### Preparation Example 20: Synthesis of Compound 20

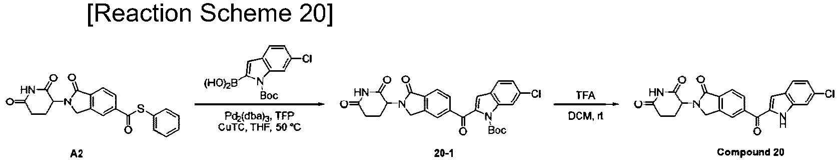

### 20-1: Synthesis of Compound 20-1

(1-(tert-Butoxycarbonyl)-6-chloro-1H-indol-2-yl)boronic acid (468 mg, 1.58 mmol) was subjected to the same synthetic procedure as for Compound 1 to give Compound 20-1 (310 mg, yield 52 %) as a pale brown solid.

### 20-2: Synthesis of Compound 20

Compound 20-1 (310 mg, 0.41 mmol) was subjected to the same synthetic procedure as for Compound 3 to give Compound 20 (81.17 mg, yield 45 %) as a white solid.

### Preparation Example 21: Synthesis of Compound 21

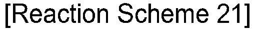

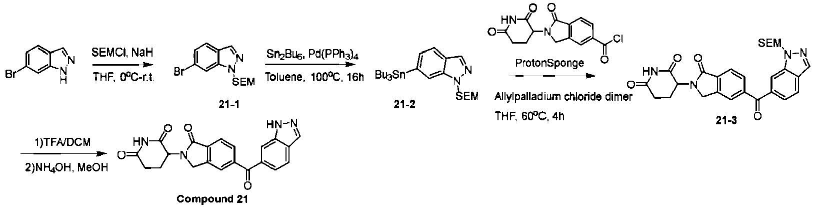

### 21-1: Synthesis of Compound 21-1

To a solution of 6-bromo-1H-indazole (3 g, 15 mmol) in THF (40 mL) was added NaH (60 %, 0.91 g, 22.8 mmol) at 0 °C under an N₂ atmosphere. The solution was stirred at 0 °C for 30 minutes, and then SEMCI (3.04 g, 18.2 mmol) was slowly added to the solution. The reaction mixture was slowly warmed to room temperature and stirred for an additional 2 hours. After the reaction was completed, the mixture was quenched with water (20 mL) and extracted with EtOAc (30 mL × 3). The combined organic layers were dried over Na₂SO₄ and concentrated. The residue was purified by silica gel chromatography (PE/EtOAc = 0-10 %) to give Compound 21-1 (3 g, yield 54 %) as a colorless liquid.

### 21-2: Synthesis of Compound 21-2

To a solution of Compound 21-1 (1 g, 3 mmol) in toluene (20 mL) were added hexabutylditin (Sn₂Bu₆, 2.09 g, 3.6 mmol) and tetrakis(triphenylphosphine)palladium (Pd(PPh₃)₄, 350 mg, 0.3 mmol). The reaction mixture was stirred at 100 °C for 16 hours under an N₂ atmosphere. After the reaction was completed, the mixture was concentrated. The residue was purified by silica gel chromatography (PE/EtOAc = 0-10 %) to give Compound 21-2 (950 mg, yield 57 %) as a colorless liquid.

### 21-3: Synthesis of Compound 21-3

To a solution of 2-(2,6-dioxopiperidin-3-yl)-1-oxoisoindoline-5-carbonyl chloride (210 mg, 0.69 mmol) in THF (10 mL) were added proton sponge (298 mg, 1.39 mmol), Compound 23-2 (748 mg, 1.39 mmol), and an allylpalladium chloride dimer (76 mg, 0.21 mmol). The reaction mixture was stirred at 60 °C for 4 hours under an N₂ atmosphere. After the reaction was completed, the mixture was concentrated. The residue was purified by silica gel chromatography (PE/EtOAc = 0-10 %) to give Compound 21-3 (170 mg, yield 33 %) as a yellow solid.

### 21-4: Synthesis of Compound 21

To a solution of Compound 21-3 (170 mg, 0.33 mmol) in DCM (10 mL) was added TFA (1 mL). The reaction mixture was stirred at room temperature for 2 hours. After the reaction was completed, the reaction was quenched with water (10 mL), and the pH of the mixture was adjusted to 8 with a saturated aqueous NaHCO₃ solution. The aqueous layer was extracted with DCM (30 mL × 2), and the organic layer was concentrated to give a crude product. The crude product was purified by prep-HPLC to give Compound 21 (11.02 mg, yield 8.6 %) as a white solid.

### Preparation Example 22: Synthesis of Compound 22

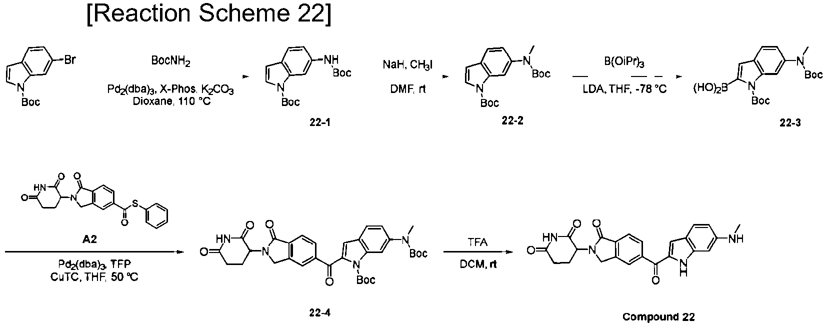

### 22-1: Synthesis of Compound 22-1

To a solution of tert-butyl 6-bromo-1H-indole-1-carboxylate (3.00 g, 0.010 mol) and tert-butyl carbamate (2.39 g, 0.020 mol) in dioxane (100 mL) were added K₂CO₃ (4.18 g, 0.030 mol), X-Phos (0.96 g, 0.0020 mol), and Pd₂(dba)₃ (1.85 g, 0.0020 mol) at room temperature. The mixture was stirred at 110 °C for 2 hours. The reaction mixture was filtered, and the filtrate was concentrated under reduced pressure. The residue was purified by flash column chromatography (PE/EtOAc = 100:0 to 0:100) to give Compound 22-1 (2.10 g, yield 56 %) as a yellow liquid.

### 22-2: Synthesis of Compound 22-2

To a solution of Compound 22-1 (2.10 g, 0.006 mol) in DMF (35 mL) was added NaH (60 wt% in mineral oil, 0.38 g, 0.009 mol) at room temperature. The mixture was stirred at room temperature for 30 minutes. Then, CH₃I (1.34 g, 0.009 mol) was added, and the mixture was stirred at room temperature for 1 hour. The reaction mixture was quenched with cold water (20 mL) and extracted with EtOAc (50 mL × 3). The combined organic layers were washed with water and brine, dried over Na₂SO₄, and concentrated under reduced pressure. The residue was purified by flash column chromatography (PE/EtOAc = 100:0 to 20:80) to give Compound 22-2 (1.6 g, yield 65 %) as a yellow liquid.

### 22-3: Synthesis of Compound 22-3

To a solution of Compound 22-2 (1.6 g, 0.005 mol) in THF (40 mL) was added LDA (1 M in THF, 6.9 mL, 0.007 mol) at -78 °C. The mixture was stirred at -78 °C for 30 minutes. Then, triisopropyl borate (1.3 g, 0.007 mol) was added at the same temperature, and the mixture was stirred at -78 °C for 30 minutes. The reaction mixture was quenched with water (15 mL) and extracted with EtOAc (50 mL × 3). The combined organic layers were washed with water and brine, dried over Na₂SO₄, filtered, and concentrated under reduced pressure. The residue was purified by flash column chromatography (PE/EtOAc = 100:0 to 10:90) to give Compound 22-3 (1.0 g, yield 50 %) as a yellow liquid.

### 22-4: Synthesis of Compound 22-4

Compound 22-3 (200 mg, 0.526 mmol) was subjected to the same synthetic procedure as for Compound 1 to give Compound 22-4 (40 mg, yield 11 %) as a white solid.

### 22-5: Synthesis of Compound 22

Compound 22-4 (40 mg, 0.065 mmol) was subjected to the same synthetic procedure as for Compound 3 to give Compound 22 (13.38 mg, yield 48 %) as an orange solid.

### Preparation Example 23: Synthesis of Compound 23

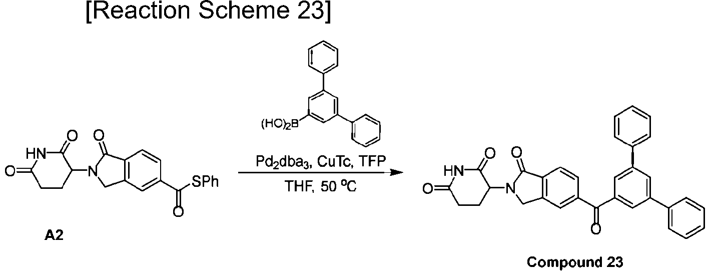

(3,5-Diphenylphenyl)boronic acid (48 mg, 0.17 mmol) was subjected to the same synthetic procedure as for Compound 1 to give Compound 23 (21.0 mg, yield 25 %) as a white solid.

### Preparation Example 24: Synthesis of Compound 24

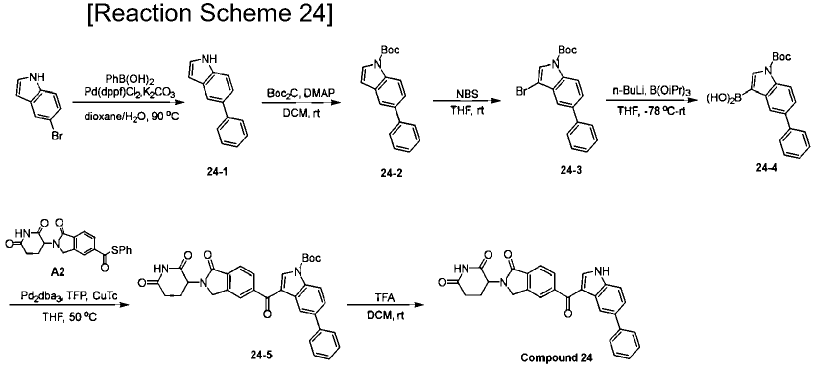

### 24-1: Synthesis of Compound 24-1

A solution of 5-bromo-1H-indole (5.0 g, 25.5 mmol), phenylboronic acid (4.66 g, 38.2 mmol), Pd(dppf)Cl₂ (1.04 g, 1.2 mmol), and K₂CO₃ (7.08 g, 51 mmol) in a mixture of dioxane (40 mL) and water (10 mL) was stirred at 90 °C for 4 hours under an N₂ atmosphere. The mixture was cooled to room temperature, filtered through Celite, and the filtrate was concentrated under reduced pressure. The residue was diluted with water and extracted with EtOAc. The combined organic layers were washed with water and brine, dried over anhydrous sodium sulfate, and concentrated under reduced pressure. The residue was purified by silica gel chromatography (EtOAc/PE, 0-10 %) to give Compound 24-1 (2.8 g, yield 61 %) as a white solid.

### 24-2: Synthesis of Compound 24-2

To a solution of Compound 24-1 (1.1 g, 5.7 mmol) in THF (20 mL) were slowly added (Boc)₂O (2.5 g, 11.4 mmol) and 4-dimethylaminopyridine (70 mg, 0.57 mmol) at 0 °C. The mixture was stirred at 20 °C for 1 hour. The resulting mixture was then diluted with water (15 mL) and extracted with EtOAc (30 mL × 3). The combined organic layers were washed with brine, dried over anhydrous sodium sulfate, and concentrated under reduced pressure. The residue was purified by silica gel column chromatography (EtOAc/PE, 0-3 %) to give Compound 24-2 (1.5 g, yield 90 %) as a white solid.

### 24-3: Synthesis of Compound 24-3

To a solution of Compound 24-2 (1.3 g, 4.4 mmol) in THF (5 mL) was added NBS (0.8 g, 4.6 mmol) at 20 °C. The mixture was stirred at 20 °C for 2 hours. The resulting mixture was then diluted with water (20 mL) and extracted with EtOAc (50 mL × 3). The combined organic layers were washed with brine, dried over anhydrous sodium sulfate, and concentrated under reduced pressure. The residue was purified by silica gel column chromatography (EtOAc/PE, 0-5 %) to give Compound 24-3 (1.3 g, yield 79 %) as a white solid.

### 24-4: Synthesis of Compound 24-4

Compound 24-3 (500 mg, 1.34 mmol) was subjected to the same synthetic procedure as for Compound 22-3 to give Compound 24-4 (200 mg, yield 44 %) as a white solid.

### 24-5: Synthesis of Compound 24-5

Compound 24-4 (185 mg, 0.55 mmol) was subjected to the same synthetic procedure as for Compound 1 to give Compound 24-5 (230 mg, yield 97 %) as a white solid.

### 24-6: Synthesis of Compound 24

Compound 24-5 (230 mg, 0.40 mmol) was subjected to the same synthetic procedure as for Compound 3 to give Compound 24 (23 mg, yield 12 %) as a white solid.

### Preparation Example 25: Synthesis of Compound 25

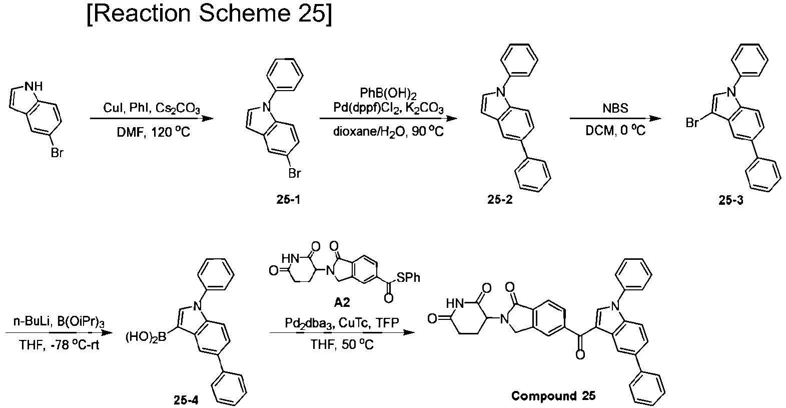

### 25-1: Synthesis of Compound 25-1

A solution of 5-bromo-1H-indole (5.0 g, 25.7 mmol), Cul (0.7 g, 3.6 mmol), iodobenzene (3.7 g, 18.4 mmol), and Cs₂CO₃ (11.9 g, 36.8 mmol) in DMF (30 mL) was stirred at 120 °C for 16 hours under an N₂ atmosphere. The mixture was then diluted with water (50 mL) and extracted with EtOAc (30 mL × 3). The combined organic layers were washed with water (100 mL), dried over Na₂SO₄, and concentrated. The residue was purified by silica gel chromatography (PE) to give Compound 25-1 (3.0 g, yield 53.8 %) as a colorless liquid.

### *406

### 25-2: Synthesis of Compound 25-2

Compound 25-1 (3.0 g, 11 mmol) was subjected to the same synthetic procedure as for Compound 24-1 to give Compound 25-2 (1.9 g, yield 57 %) as a white solid.

### 25-3: Synthesis of Compound 25-3

Compound 25-2 (1.9 g, 7.1 mmol) was subjected to the same synthetic procedure as for Compound 24-3 to give Compound 25-3 (2.5 g, yield 80 %) as a white solid.

### 25-4: Synthesis of Compound 25-4

Compound 25-3 (200 mg, 0.57 mmol) was subjected to the same synthetic procedure as for Compound 22-3 to give Compound 25-4 (200 mg, yield 78 %) as a white solid.

### 25-5: Synthesis of Compound 25

Compound 25-4 (86 mg, 0.27 mmol) was subjected to the same synthetic procedure as for Compound 1 to give Compound 25 (23.76 mg, yield 23 %) as a white solid.

### Preparation Example 26: Synthesis of Compound 26

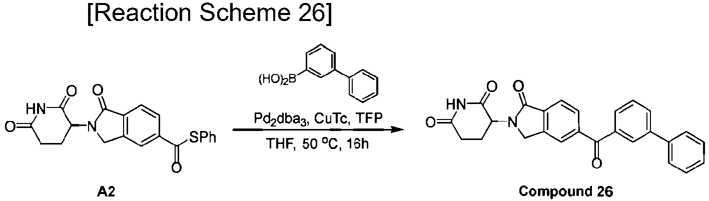

Biphenyl-3-boronic acid (50 mg, 0.25 mmol) was subjected to the same synthetic procedure as for Compound 1 to give Compound 26 (36.42 mg, yield 41 %) as a white solid.

### Preparation Example 27: Synthesis of Compound 27

### 27-1: Synthesis of Compound A4

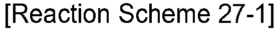

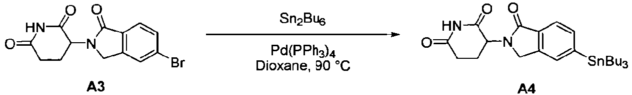

To a solution of 3-(5-bromo-1-oxoisoindolin-2-yl)piperidine-2,6-dione (A3, 1.0 g, 3.10 mmol) and hexabutyldistannane (Sn₂Bu₆, 2.7 g, 4.65 mmol) in dioxane (30 mL) was added Pd(PPh₃)₄ (360 mg, 0.31 mmol) at room temperature. The reaction mixture was stirred at 90 °C for 3 hours. The mixture was cooled to room temperature and concentrated under reduced pressure. The residue was purified by flash chromatography (PE/EtOAc = 100:0 to 65:35) to give Compound A4 (700 mg, yield 38 %) as a yellow solid.

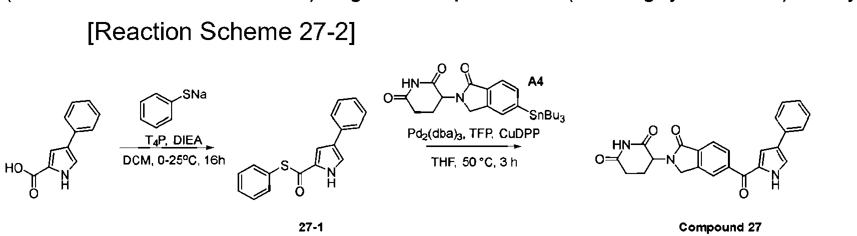

### 27-2: Synthesis of Compound 27-1

4-Phenyl-1H-pyrrole-2-carboxylic acid (2 g, 0.011 mol) was subjected to the same synthetic procedure as for Compound A2 to give Compound 27-1 (0.25 g, yield 8.1 %) as a yellow solid.

### 27-3: Synthesis of Compound 27

To a solution of Compound 27-1 (250 mg, 0.895 mmol) and Compound A4 (477 mg, 0.895 mmol) in THF (5 mL) were added Pd₂(dba)₃ (82 mg, 0.089 mmol), tri(2-furyl)phosphine (TFP, 104 mg, 0.447 mmol), and copper(I) diphenylphosphinate (CuDPP, 504 mg, 1.79 mmol) at room temperature. The reaction mixture was stirred at 50 °C for 3 hours under a nitrogen atmosphere. The mixture was cooled to room temperature and concentrated under reduced pressure. The residue was purified by pre-TLC (MeOH/DCM = 10/1) and prep-HPLC to give Compound 27 (10 mg, yield 2.7 %) as a yellow solid.

### Preparation Example 28: Synthesis of Compound 28

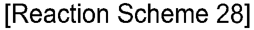

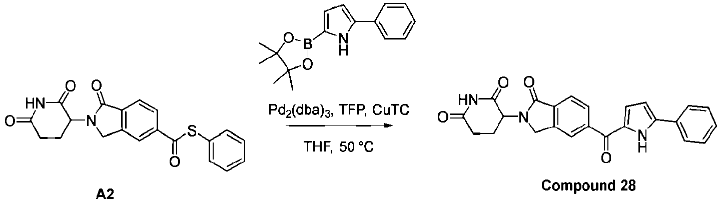

2-Phenyl-5-(4,4,5,5-tetramethyl-1,3,2-dioxaborolan-2-yl)-1H-pyrrole (50 mg, 0.186 mmol) was subjected to the same synthetic procedure as for Compound 1 to give Compound 28 (4 mg, yield 6.5 %) as a yellow solid.

### Preparation Example 29: Synthesis of Compound 29

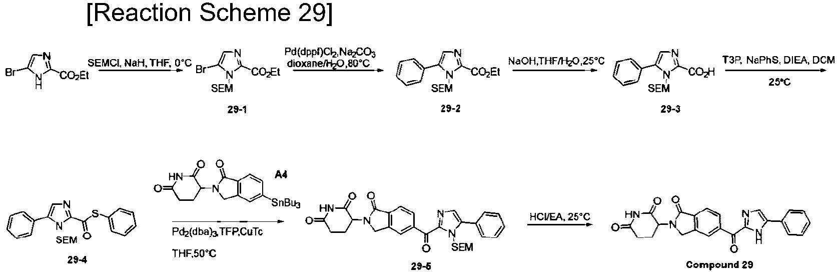

### 29-1: Synthesis of Compound 29-1

Ethyl 4-bromo-3H-imidazole-2-carboxylate (4.5 g, 0.021 mol) was subjected to the same synthetic procedure as for Compound 21-1 to give Compound 29-1 (4.0 g, yield 56 %) as a yellow liquid.

### 29-2: Synthesis of Compound 29-2

Compound 29-1 (4.5 g, 0.013 mol) was subjected to the same synthetic procedure as for Compound 24-1 to give Compound 29-2 (4 g, yield 90 %) as a yellow solid.

### 29-3: Synthesis of Compound 29-3

To a mixed solution of Compound 29-2 (4 g, 0.012 mol) in H₂O/THF (1/4, 40 mL) was added NaOH (1.38 g, 0.035 mmol) at 25 °C. The mixture was stirred at 25 °C for 4 hours. The mixture was poured into water (50 mL), the pH was adjusted to 8-9 using HCl (4 N), and the mixture was extracted with EtOAc (20 mL × 3). The combined organic extracts were washed with brine (30 mL), dried over Na₂SO₄, and concentrated to give Compound 29-3 (3.7 g, yield 86 %) as a yellow solid.

### 29-4: Synthesis of Compound 29-4

Compound 29-3 (1.4 g, 4.4 mmol) was subjected to the same synthetic procedure as for Compound A2 to give Compound 29-4 (500 mg, yield 28 %) as a yellow solid.

### 29-5: Synthesis of Compound 29-5

Compound 29-4 (500 mg, 0.94 mmol) was subjected to the same synthetic procedure as for Compound 27 to give Compound 29-5 (100 mg, yield 15 %) as a yellow solid.

### 29-6: Synthesis of Compound 29

Compound 29-5 (100 mg, 0.18 mmol) was dissolved in a solution of HCl/EA (4 mL, 2.5 N) and stirred at 25 °C for 4 hours. The reaction mixture was concentrated under reduced pressure. The residue was purified by prep-HPLC to give Compound 29 (15.85 mg, yield 21 %) as a yellow solid.

### Preparation Example 30: Synthesis of Compound 30

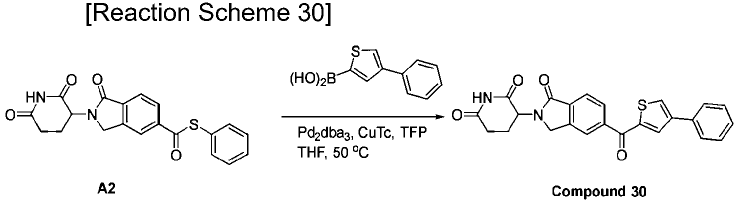

(4-Phenylthiophen-2-yl)boronic acid (200 mg, 0.98 mmol) was subjected to the same synthetic procedure as for Compound 1 to give Compound 30 (14.68 mg, yield 3.5 %) as an orange solid.

### Preparation Example 31: Synthesis of Compound 31

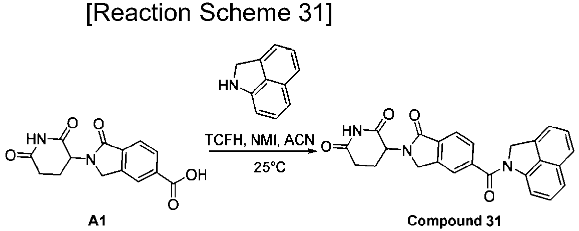

To a solution of 2-(2,6-dioxopiperidin-3-yl)-1-oxoisoindoline-5-carboxylic acid (A1, 200 mg, 0.70 mmol), 1,2-dihydrobenzo[cd]indole (108 mg, 0.70 mmol), and N,N,N',N'-tetramethylchloroformamidinium hexafluorophosphate (TCFH, 292 mg, 1.04 mmol) in ACN (6 mL) was added N-methylimidazole (NMI, 171 mg, 2.08 mmol) at 25 °C. The mixture was stirred at 25 °C for 2 hours. The mixture was concentrated, and the residue was purified by prep-HPLC to give Compound 31 (15.01 mg, yield 5.1 %) as a yellow solid.

### Preparation Example 32: Synthesis of Compound 32

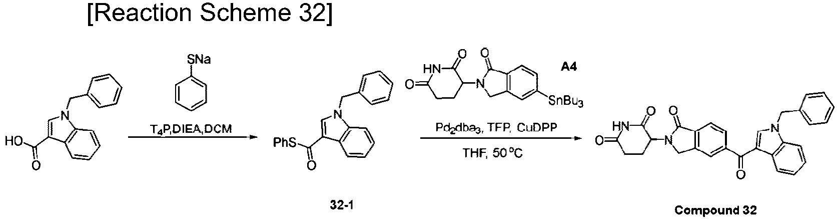

### 32-1: Synthesis of Compound 32-1

1-Benzyl-1H-indole-3-carboxylic acid (800 mg, 3.18 mmol) was subjected to the same synthetic procedure as for Compound A2 to give Compound 32-1 (150 mg, yield 14 %) as a white solid.

### 32-2: Synthesis of Compound 32

Compound 32-1 (150 mg, 0.44 mmol) was subjected to the same synthetic procedure as for Compound 27 to give Compound 32 (20.15 mg, yield 9.7 %) as a white solid.

### Preparation Example 33: Synthesis of Compound 33

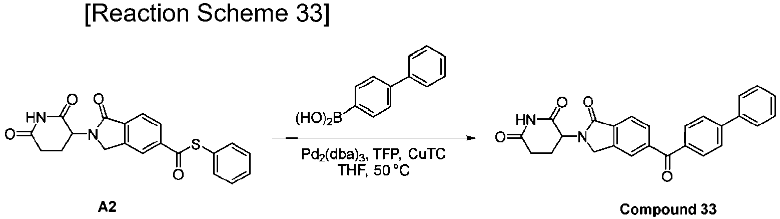

Biphenyl-4-boronic acid (100 mg, 0.51 mmol) was subjected to the same synthetic procedure as for Compound 1 to give Compound 33 (12.65 mg, yield 5.6 %) as a white solid.

### Preparation Example 34: Synthesis of Compound 34

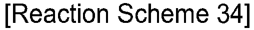

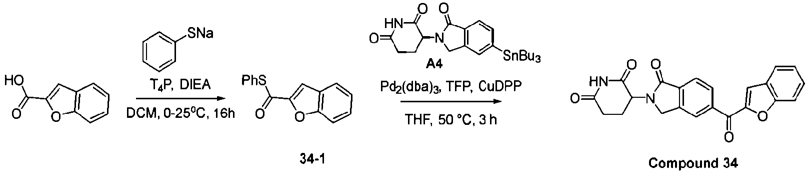

### 34-1: Synthesis of Compound 34-1

Benzofuran-2-carboxylic acid (600 mg, 3.70 mmol) was subjected to the same synthetic procedure as for Compound A2 to give Compound 34-1 (600 mg, yield 74 %) as a white solid.

### 34-2: Synthesis of Compound 34

Compound 34-1 (200 mg, 0.79 mmol) was subjected to the same synthetic procedure as for Compound 27 to give Compound 39 (13.51 mg, yield 4.3 %) as a white solid.

### Preparation Example 35: Synthesis of Compound 35

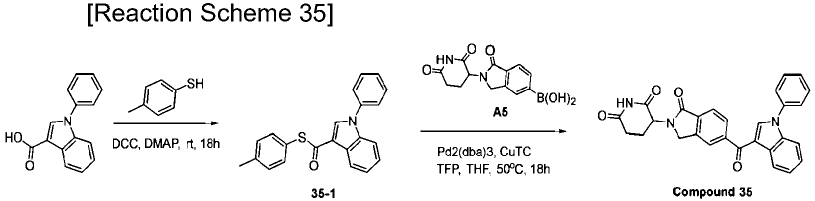

### 35-1: Synthesis of Compound 35-1

To a solution of 1-phenyl-1H-indole-3-carboxylic acid (1.3 g, 5.5 mmol) and 4-methylbenzenethiol (1.03 g, 8.25 mmol) in DCM (30 mL) were added DMAP (67 mg, 0.55 mmol) and DCC (1.25 g, 6.05 mmol). The mixture was stirred at room temperature for 16 hours under an N₂ atmosphere. The reaction was quenched with an aqueous ammonium chloride solution, and the mixture was extracted with DCM (50 mL × 3). The combined organic extracts were washed with brine and dried over Na₂SO₄. The residue was purified by silica gel (25 g) chromatography (PE/EtOAc = 1/3) to give Compound 35-1 (1.9 g, yield 100 %) as a colorless liquid.

### 35-2: Synthesis of Compound 35

To a solution of Compound 35-1 (300 mg, 0.875 mmol) and (2-(2,6-dioxopiperidin-3-yl)-1-oxoisoindolin-5-yl)boronic acid (A5, 200 mg, 0.7 mmol) in THF (10 mL) were added Pd₂(dba)₂ (160 mg, 0.175 mmol), CuTC (285 mg, 1.3 mmol), and TFP (47 mg, 0.2 mmol). The mixture was stirred at 50 °C for 16 hours under an N₂ atmosphere. The reaction was quenched with a saturated aqueous ammonium chloride solution, and the mixture was extracted with DCM (30 mL × 3). The combined organic extracts were washed with brine and dried over Na₂SO₄. The residue was purified by silica gel (25 g) chromatography (DCM/MeOH = 1/10) and concentrated. The concentrate was purified by prep-HPLC (FA) (column: Wepure Prep C18 10 µm 21.2 × 250 mm; mobile phase A: water (0.2 % FA), B: acetonitrile; B: 33-63 %) to give Compound 35 (20 mg, yield 4.9 %) as a white solid.

### Preparation Example 36: Synthesis of Compound 36

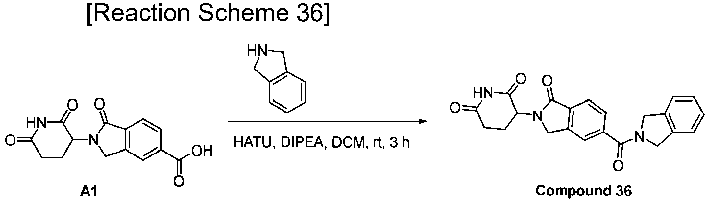

To a solution of 2-(2,6-dioxopiperidin-3-yl)-1-oxoisoindoline-5-carboxylic acid (A1, 95 mg, 0.33 mmol) in DCM (5 mL) were added isoindoline (47 mg, 0.395 mmol), HATU (187.8 mg, 0.494 mmol), and DIPEA (127.8 mg, 0.989 mmol) at 0 °C. The mixture was then stirred from 0 °C to room temperature for 3 hours, and H₂O (5 mL) was added. The mixture was extracted with DCM (3 × 5 mL). The combined organic extracts were dried, filtered, and concentrated. The residue was purified by prep-HPLC (FA) (column: Boston Prep C18 10 µm 21.2 × 250 mm; mobile phase A: water (0.2 % FA), B: acetonitrile; B: 13-43 %) to give Compound 36 (55 mg, yield 43 %) as a white solid.

### Preparation Example 37: Synthesis of Compound 37

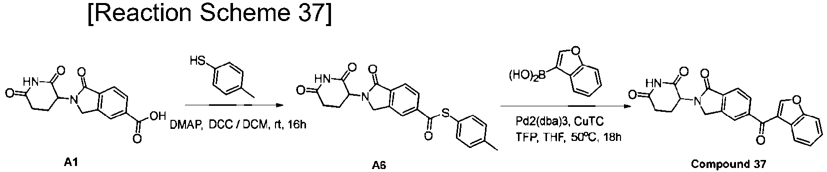

### 37-1: Synthesis of Compound A6

To a solution of 2-(2,6-dioxopiperidin-3-yl)-1-oxoisoindoline-5-carboxylic acid (A1, 500 mg, 1.74 mmol) and 4-methylbenzenethiol (260 mg, 2.1 mmol) in DCM (20 mL) were added DMAP (21 mg, 0.174 mol) and DCC (394 mg, 1.91 mmol). The reaction mixture was stirred at room temperature for 16 hours. The reaction was quenched with a saturated aqueous NH₄Cl solution and extracted with DCM (30 mL × 3). The combined organic layers were washed with brine and dried over Na₂SO₄. The residue was purified by silica gel chromatography (PE/EA = 1/3) to give Compound A6 (440 mg, yield 64 %) as a white solid.

### 37-2: Synthesis of Compound 37

Benzofuran-3-ylboronic acid (136 mg, 0.836 mmol) and Compound A6 (300 mg, 0.76 mmol) were subjected to the same synthetic procedure as for Compound 1 to give Compound 37 (160 mg, yield 54 %) as a white solid.

### Preparation Example 38: Synthesis of Compound 38

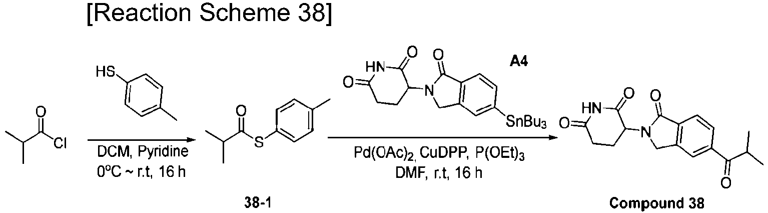

### 38-1: Synthesis of Compound 38-1

To a solution of 4-methylbenzenethiol (0.5 g, 4 mmol) in DCM (20 mL) were added isobutyryl chloride (0.5 g, 4.9 mmol) and pyridine (0.5 g, 6.3 mmol) at 0 °C under a nitrogen atmosphere. The reaction mixture was stirred at room temperature for 16 hours. The mixture was poured into water (50 mL) and extracted with EA (30 mL × 3). The combined organic layers were dried over Na₂SO₄ and concentrated. The residue was purified by silica gel chromatography (DCM/MeOH = 20/1) to give Compound 38-1 (0.8 g, yield 99 %) as a colorless liquid.

### 38-2: Synthesis of Compound 38

To a solution of Compound A4 (160 mg, 0.3 mmol), Compound 38-1 (60 mg, 0.3 mmol), CuDPP (105 mg, 0.37 mmol), and Pd(OAc)₂ (3 mg, 0.01 mmol) in DMF (5 mL) was added triethyl phosphite (10 mg, 0.06 mmol) under a nitrogen atmosphere. The mixture was stirred at room temperature for 16 hours. Potassium fluoride (60 mg, 1 mmol) was added to the mixture, and the mixture was stirred at room temperature for 1 hour. The mixture was poured into water (30 mL) and extracted with EA (20 mL × 3). The combined organic layers were dried over Na₂SO₄ and concentrated. The residue was purified by prep-HPLC (column: Xtimate Prep C18 10 µm 21.2 × 250 mm; mobile phase A: water (0.2 % FA), B: acetonitrile; B: 12-42 %, 8 min, held at 16 min) to give Compound 38 (17.2 mg, yield 18 %) as a white solid.

### Preparation Example 39: Synthesis of Compound 39

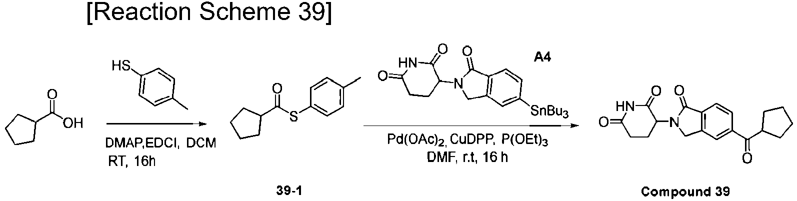

### 39-1: Synthesis of Compound 39-1

To a solution of cyclopentanecarboxylic acid (720 mg, 6.3 mmol) in DCM (20 mL) were added DMAP (77 mg, 0.63 mmol) and EDCI (1.81 g, 9.45 mmol). The mixture was stirred at room temperature for 5 minutes under a nitrogen atmosphere, and then 4-methylbenzenethiol (859 mg, 6.93 mmol) was added. The mixture was stirred at room temperature for 12 hours under a nitrogen atmosphere. The reaction mixture was poured into water (50 mL) and extracted with DCM (30 mL × 3). The combined organic layers were washed with brine and dried over anhydrous Na₂SO₄. The residue was purified by silica gel chromatography (PE/EA = 1/3) to give Compound 39-1 (400 mg, yield 29 %) as a colorless liquid.

### 39-2: Synthesis of Compound 39

Compound 39-1 (62 mg, 0.28 mmol) was subjected to the same synthetic procedure as for Compound 38 to give Compound 39 (22.9 mg, yield 24 %) as a white solid.

### Preparation Example 40: Synthesis of Compound 40

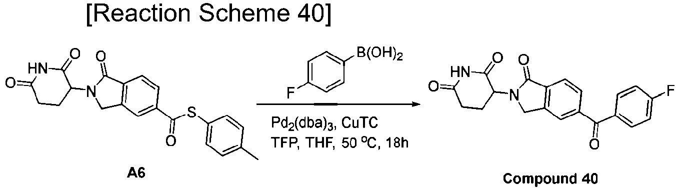

(4-Fluorophenyl)boronic acid (25.4 mg, 0.18 mmol) and Compound A6 (65 mg, 0.16 mmol) were subjected to the same synthetic procedure as for Compound 1 to give Compound 40 (20.1 mg, yield 33 %) as a white solid.

### Preparation Example 41: Synthesis of Compound 41

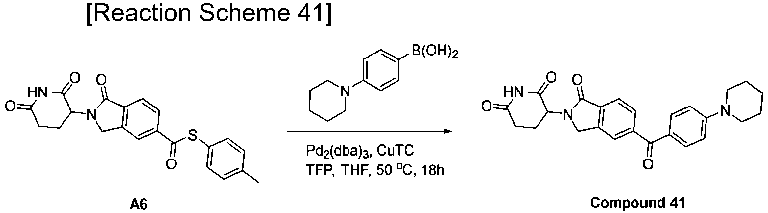

(4-(Piperidin-1-yl)phenyl)boronic acid (40 mg, 0.20 mmol) and Compound A6 (70 mg, 0.18 mmol) were subjected to the same synthetic procedure as for Compound 1 to give Compound 41 (15.0 mg, yield 20 %) as a yellow solid.

### Preparation Example 42: Synthesis of Compound 42

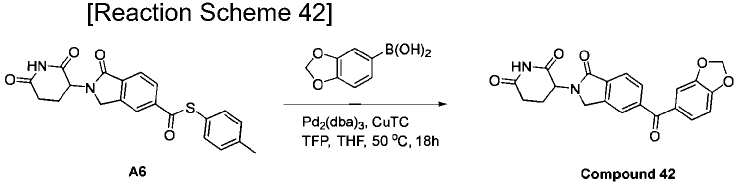

Benzo[d][1,3]dioxol-5-ylboronic acid (33 mg, 0.20 mmol) and Compound A6 (70 mg, 0.18 mmol) were subjected to the same synthetic procedure as for Compound 1 to give Compound 42 (19.1 mg, yield 27 %) as a white solid.

### Preparation Example 43: Synthesis of Compound 43

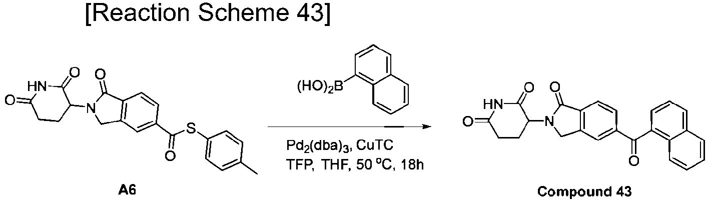

Naphthalen-1-ylboronic acid (34 mg, 0.20 mmol) and Compound A6 (70 mg, 0.18 mmol) were subjected to the same synthetic procedure as for Compound 1 to give Compound 43 (23.7 mg, yield 33 %) as a white solid.

### Preparation Example 44: Synthesis of Compound 44

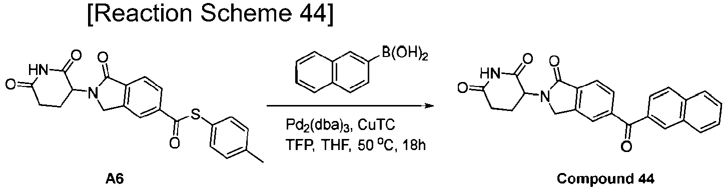

Naphthalen-2-ylboronic acid (34 mg, 0.20 mmol) and Compound A6 (70 mg, 0.18 mmol) were subjected to the same synthetic procedure as for Compound 1 to give Compound 44 (31.6 mg, yield 43 %) as a white solid.

### Preparation Example 45: Synthesis of Compound 45

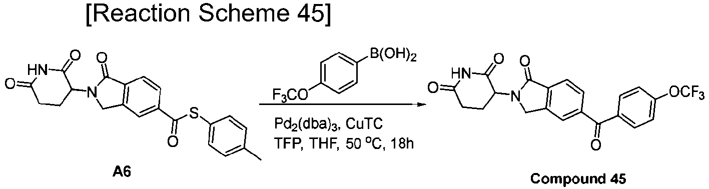

(4-(Trifluoromethoxy)phenyl)boronic acid (37.3 mg, 0.18 mmol) and Compound A6 (65 mg, 0.16 mmol) were subjected to the same synthetic procedure as for Compound 1 to give Compound 45 (21.5 mg, yield 30 %) as a white solid.

### Preparation Example 46: Synthesis of Compound 46

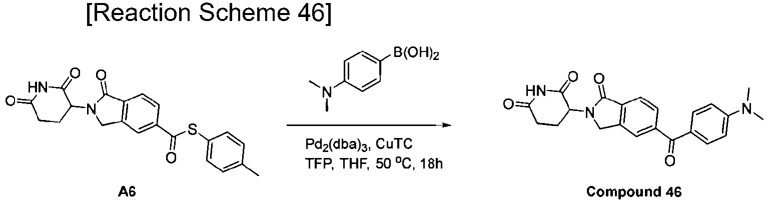

(4-(Dimethylamino)phenyl)boronic acid (34 mg, 0.20 mmol) and Compound A6 (70 mg, 0.18 mmol) were subjected to the same synthetic procedure as for Compound 1 to give Compound 46 (19.6 mg, yield 28 %) as a yellow solid.

### Preparation Example 47: Synthesis of Compound 47

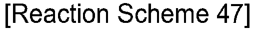

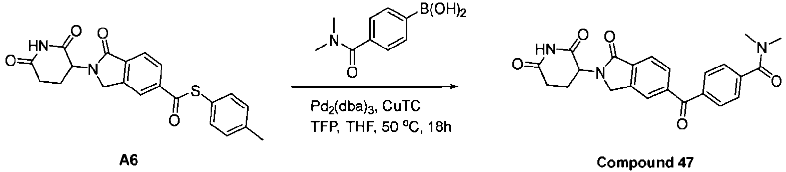

(4-(Dimethylcarbamoyl)phenyl)boronic acid (39 mg, 0.20 mmol) and Compound A6 (70 mg, 0.18 mmol) were subjected to the same synthetic procedure as for Compound 1 to give Compound 47 (21.8 mg, yield 29 %) as a white solid.

### Preparation Example 48: Synthesis of Compound 48

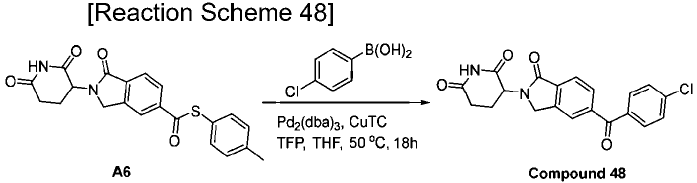

(4-Chlorophenyl)boronic acid (47.5 mg, 0.304 mmol) and Compound A6 (100 mg, 0.254 mmol) were subjected to the same synthetic procedure as for Compound 1 to give Compound 48 (52.1 mg, yield 54 %) as a white solid.

### Preparation Example 49: Synthesis of Compound 49

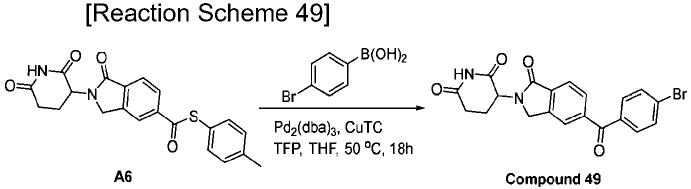

(4-Bromophenyl)boronic acid (39.4 mg, 0.223 mmol) and Compound A6 (80 mg, 0.203 mmol) were subjected to the same synthetic procedure as for Compound 1 to give Compound 49 (25.2 mg, yield 29 %) as a white solid.

### Preparation Example 50: Synthesis of Compound 50

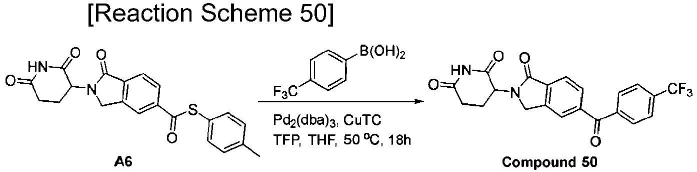

(4-(Trifluoromethyl)phenyl)boronic acid (42.4 mg, 0.223 mmol) and Compound A6 (80 mg, 0.203 mmol) were subjected to the same synthetic procedure as for Compound 1 to give Compound 50 (21.4 mg, yield 28 %) as a white solid.

### Preparation Example 51: Synthesis of Compound 51

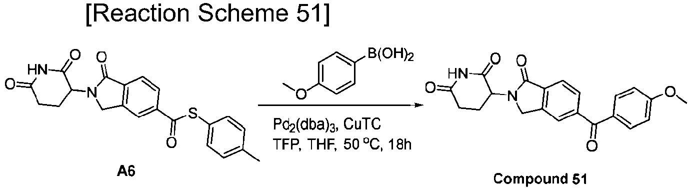

(4-Methoxyphenyl)boronic acid (36.9 mg, 0.223 mmol) and Compound A6 (80 mg, 0.203 mmol) were subjected to the same synthetic procedure as for Compound 1 to give Compound 51 (36.9 mg, yield 48 %) as a white solid.

### Preparation Example 52: Synthesis of Compound 52

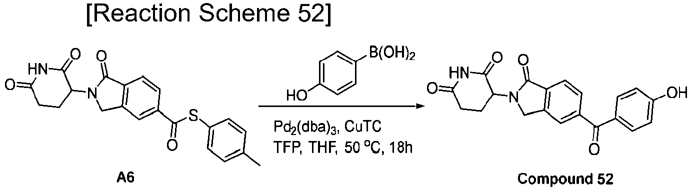

(4-Hydroxyphenyl)boronic acid (33.5 mg, 0.223 mmol) and Compound A6 (80 mg, 0.203 mmol) were subjected to the same synthetic procedure as for Compound 1 to give Compound 52 (19.6 mg, yield 27 %) as a white solid.

### Preparation Example 53: Synthesis of Compound 53

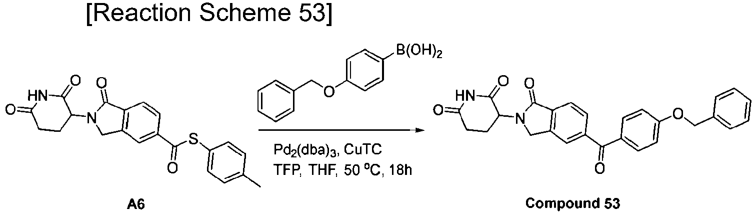

(4-(Benzyloxy)phenyl)boronic acid (55.4 mg, 0.223 mmol) and Compound A6 (80 mg, 0.203 mmol) were subjected to the same synthetic procedure as for Compound 1 to give Compound 53 (25.5 mg, yield 28 %) as a white solid.

### Preparation Example 54: Synthesis of Compound 54

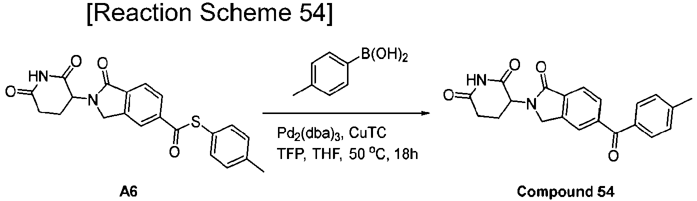

p-Tolylboronic acid (40 mg, 0.3 mmol) and Compound A6 (100 mg, 0.25 mmol) were subjected to the same synthetic procedure as for Compound 1 to give Compound 54 (13.5 mg, yield 15 %) as a white solid.

### Preparation Example 55: Synthesis of Compound 55

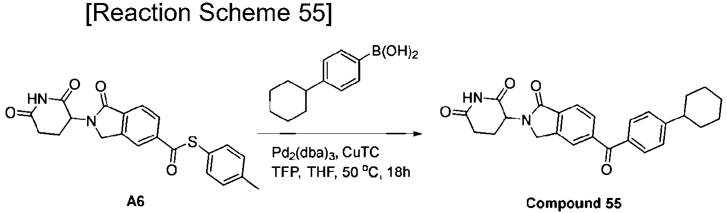

(4-Cyclohexylphenyl)boronic acid (43 mg, 0.21 mmol) and Compound A6 (70 mg, 0.18 mmol) were subjected to the same synthetic procedure as for Compound 1 to give Compound 55 (15 mg, yield 20 %) as a white solid.

### Preparation Example 56: Synthesis of Compound 56

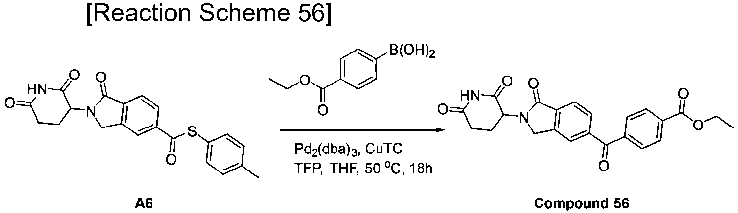

(4-(Ethoxycarbonyl)phenyl)boronic acid (42 mg, 0.21 mmol) and Compound A6 (70 mg, 0.18 mmol) were subjected to the same synthetic procedure as for Compound 1 to give Compound 56 (16 mg, yield 21 %) as a white solid.

### Preparation Example 57: Synthesis of Compound 57

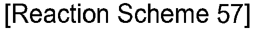

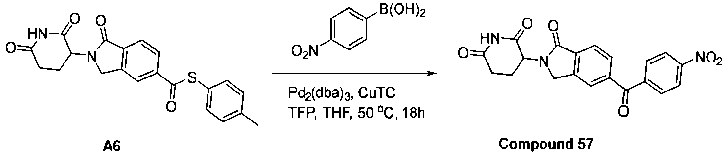

(4-Nitrophenyl)boronic acid (34 mg, 0.20 mmol) and Compound A6 (70 mg, 0.18 mmol) were subjected to the same synthetic procedure as for Compound 1 to give Compound 57 (13.5 mg, yield 19 %) as a white solid.

### Preparation Example 58: Synthesis of Compound 58

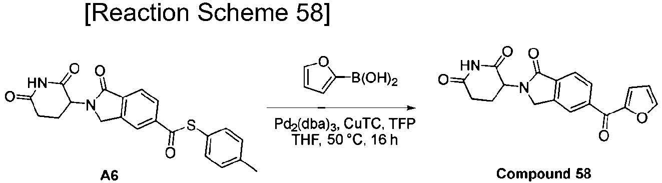

Furan-2-ylboronic acid (18 mg, 0.16 mmol) and Compound A6 (60 mg, 0.15 mmol) were subjected to the same synthetic procedure as for Compound 1 to give Compound 58 (17.6 mg, yield 35 %) as a white solid.

### Preparation Example 59: Synthesis of Compound 59

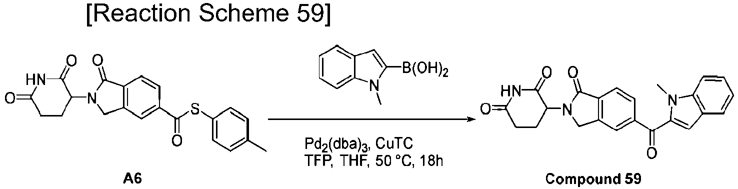

(1-Methyl-1*H*-indol-2-yl)boronic acid (48.8 mg, 0.28 mmol) and Compound A6 (100 mg, 0.25 mmol) were subjected to the same synthetic procedure as for Compound 1 to give Compound 59 (40 mg, yield 40 %) as a white solid.

### Preparation Example 60: Synthesis of Compound 60

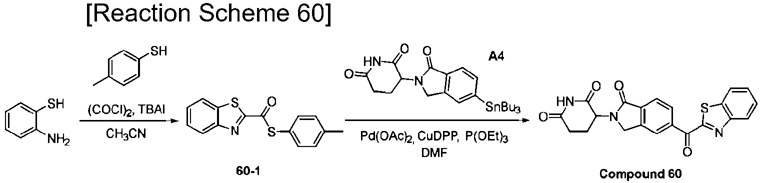

### 60-1: Synthesis of Compound 60-1

To a solution of 4-methylbenzenethiol (1 g, 8.05 mmol), oxalyl chloride (1.02 g, 8.05 mmol), and TBAI (0.3 g, 0.8 mmol) in acetonitrile (30 mL) was added dropwise a solution of 2-aminobenzenethiol (1 g, 8.05 mmol) in acetonitrile (5 mL) at room temperature, and the mixture was stirred at 60 °C for 4 hours. The reaction mixture was concentrated under reduced pressure, diluted with water (40 mL), and extracted with EA (40 mL × 3). The combined organic layers were dried over anhydrous sodium sulfate and concentrated under reduced pressure. The residue was purified by silica gel column chromatography (PE/EA = 1/10 to 1/5) to give Compound 60-1 (0.65 g, yield 28 %) as a yellow solid.

### 60-2: Synthesis of Compound 60

Compound 60-1 (120 mg, 0.42 mmol) was subjected to the same synthetic procedure as for Compound 38 to give Compound 60 (56 mg, yield 33 %) as a white solid.

### Preparation Example 61: Synthesis of Compound 61

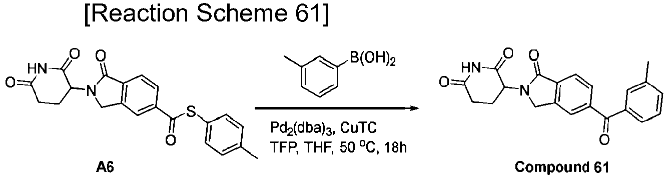

m-Tolylboronic acid (49 mg, 0.36 mmol) and Compound A6 (120 mg, 0.30 mmol) were subjected to the same synthetic procedure as for Compound 1 to give Compound 61 (40 mg, yield 36 %) as a white solid.

### Preparation Example 62: Synthesis of Compound 62

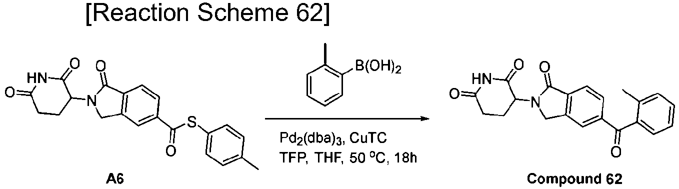

o-Tolylboronic acid (49 mg, 0.36 mmol) and Compound A6 (120 mg, 0.30 mmol) were subjected to the same synthetic procedure as for Compound 1 to give Compound 62 (6.5 mg, yield 5.9 %) as a white solid.

### Preparation Example 63: Synthesis of Compound 63

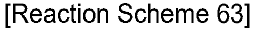

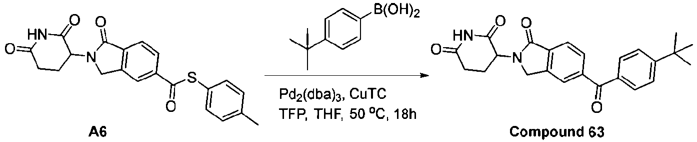

(4-(*tert*-Butyl)phenyl)boronic acid (64 mg, 0.36 mmol) and Compound A6 (120 mg, 0.30 mmol) were subjected to the same synthetic procedure as for Compound 1 to give Compound 63 (18 mg, yield 15 %) as a white solid.

### Preparation Example 64: Synthesis of Compound 64

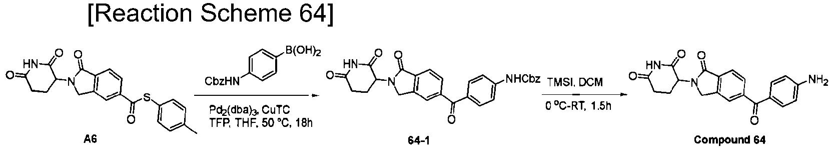

### 64-1: Synthesis of Compound 64-1

(4-(((Benzyloxy)carbonyl)amino)phenyl)boronic acid (78 mg, 0.40 mmol) and Compound A6 (140 mg, 0.36 mmol) were subjected to the same synthetic procedure as for Compound 1 to give Compound 64-1 (60 mg, yield 34 %) as a white solid.

### 64-2: Synthesis of Compound 64

To a solution of Compound 64-1 (60 mg, 0.12 mmol) in DCM (10 mL) was added TMSI (240 mg, 1.2 mmol) at 0 °C. The mixture was stirred at 0 °C for 30 minutes and then at room temperature for 1 hour. The reaction was quenched with ice water, and the mixture was extracted with DCM (30 mL × 3). The combined organic layers were dried over anhydrous Na₂SO₄ and concentrated. The residue was purified by prep-HPLC to give Compound 64 (14.2 mg, yield 33 %) as a white solid.
*671

### Preparation Example 65: Synthesis of Compound 65

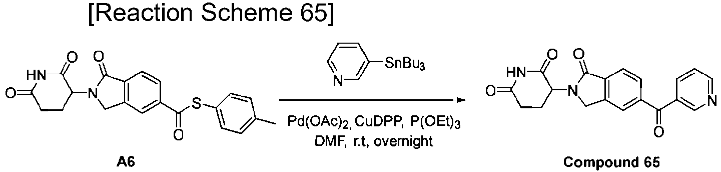

To a solution of Compound A6 (123 mg, 0.312 mmol) in DMF (8 mL) were added 3-(tributylstannyl)pyridine (115 mg, 0.312 mmol), Pd(OAc)₂ (1.4 mg, 0.00624 mmol), CuDPP (105 mg, 0.374 mmol), and P(OEt)₃ (10 mg, 0.0624 mmol). The mixture was stirred at room temperature overnight under a nitrogen atmosphere. To the mixture was added an aqueous solution of KF (60 mg), and the mixture was stirred at room temperature for 1 hour and filtered. The filtrate was extracted with EA (30 mL × 1). The organic layer was washed with brine (30 mL × 1), dried over Na₂SO₄, and concentrated. The residue was purified by prep-HPLC (A: water (0.2 % FA), B: acetonitrile, 10 % to 40 %) to give Compound 65 (15.7 mg, yield 14 %) as a white solid.

### Preparation Example 66: Synthesis of Compound 66

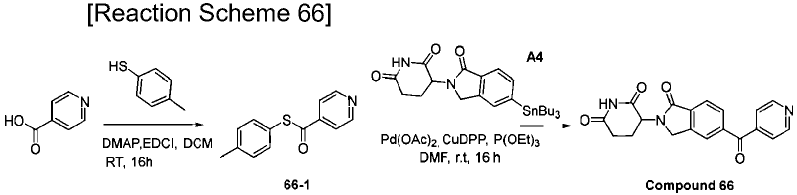

### 66-1: Synthesis of Compound 66-1

Isonicotinic acid (1.0 g, 8.1 mmol) was subjected to the same synthetic procedure as for Compound 39-1 to give Compound 66-1 (80 mg, yield 4.3 %) as a white solid.

### 66-2: Synthesis of Compound 66

Compound 66-1 (80 mg, 0.35 mmol) was subjected to the same synthetic procedure as for Compound 38 to give Compound 66 (9.82 mg, yield 8.1 %) as a white solid.

### Preparation Example 67: Synthesis of Compound 67

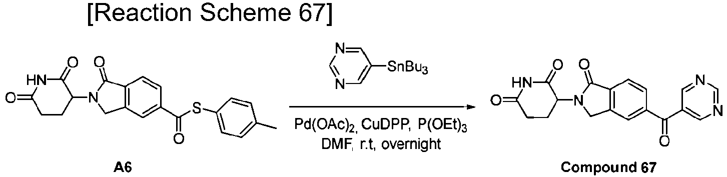

5-(Tributylstannyl)pyrimidine (140 mg, 0.38 mmol) was subjected to the same synthetic procedure as for Compound 65 to give Compound 67 (6.2 mg, yield 4.6 %) as a white solid.

### Preparation Example 68: Synthesis of Compound 68

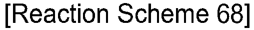

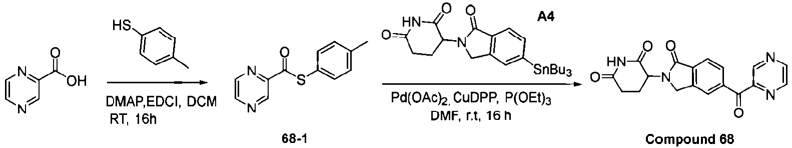

### 68-1: Synthesis of Compound 68-1

Pyrazine-2-carboxylic acid (1.0 g, 8.1 mmol) was subjected to the same synthetic procedure as for Compound 39-1 to give Compound 68-1 (70 mg, yield 3.8 %) as a white solid.

### 68-2: Synthesis of Compound 68

Compound 68-1 (62 mg, 0.28 mmol) was subjected to the same synthetic procedure as for Compound 38 to give Compound 68 (34 mg, yield 34 %) as a white solid.

### Preparation Example 69: Synthesis of Compound 69

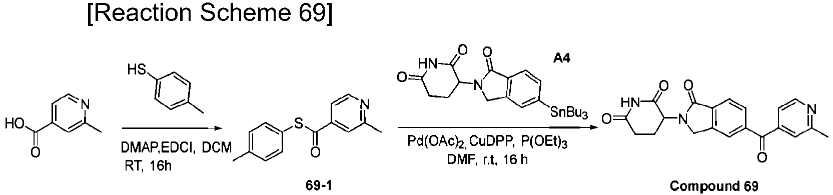

### 69-1: Synthesis of Compound 69-1

2-Methylisonicotinic acid (1.0 g, 7.3 mmol) was subjected to the same synthetic procedure as for Compound 39-1 to give Compound 69-1 (650 mg, yield 37 %) as a white solid.

### 69-2: Synthesis of Compound 69

Compound 69-1 (70 mg, 0.29 mmol) was subjected to the same synthetic procedure as for Compound 38 to give Compound 69 (19.7 mg, yield 19 %) as a white solid.

### Preparation Example 70: Synthesis of Compound 70

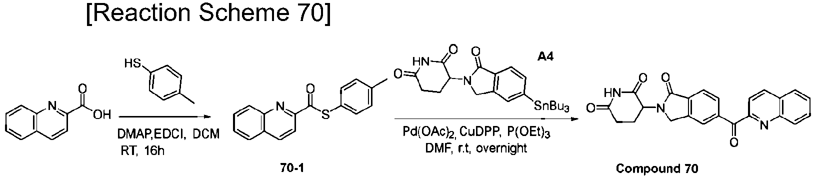

### 70-1: Synthesis of Compound 70-1

Quinoline-2-carboxylic acid (1.0 g, 5.8 mmol) was subjected to the same synthetic procedure as for Compound 39-1 to give Compound 70-1 (60 mg, yield 3.7 %) as a white solid.

### 70-2: Synthesis of Compound 70

Compound 70-1 (95 mg, 0.34 mmol) was subjected to the same synthetic procedure as for Compound 38 to give Compound 70 (14.7 mg, yield 11 %) as a white solid.

### Preparation Example 71: Synthesis of Compound 71

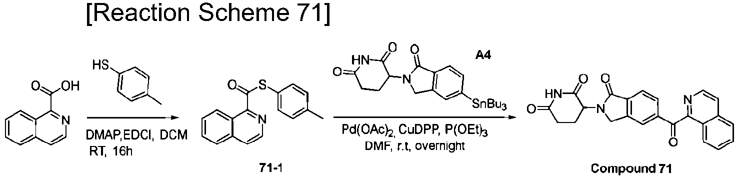

### 71-1: Synthesis of Compound 71-1

Isoquinoline-1-carboxylic acid (500 mg, 2.9 mmol) was subjected to the same synthetic procedure as for Compound 39-1 to give Compound 71-1 (130 mg, yield 16 %) as a white solid.

### 71-2: Synthesis of Compound 71

Compound 71-1 (90 mg, 0.32 mmol) was subjected to the same synthetic procedure as for Compound 38 to give Compound 71 (15 mg, yield 12 %) as a white solid.

### Preparation Example 72: Synthesis of Compound 72

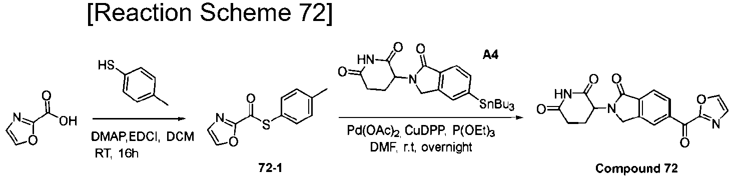

### 72-1: Synthesis of Compound 72-1

Oxazole-2-carboxylic acid (250 mg, 2.2 mmol) was subjected to the same synthetic procedure as for Compound 39-1 to give Compound 72-1 (45 mg, yield 9.3 %) as a white solid.

### 72-2: Synthesis of Compound 72

Compound 72-1 (45 mg, 0.21 mmol) was subjected to the same synthetic procedure as for Compound 38 to give Compound 72 (8.8 mg, yield 13 %) as a white solid.

### Preparation Example 73: Synthesis of Compound 73

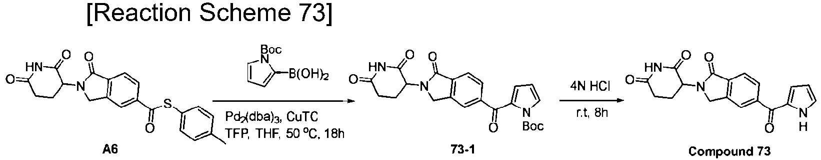

### 73-1: Synthesis of Compound 73-1

(1-(*tert*-Butoxycarbonyl)-1*H*-pyrrol-2-yl)boronic acid (76 mg, 0.36 mmol) and Compound A6 (120 mg, 0.30 mmol) were subjdddddddected to the same synthetic procedure as for Compound 1 to give Compound 73-1 (50 mg, yield 37 %) as a white solid.

### 73-2: Synthesis of Compound 73

Compound 73-1 (50 mg, 0.11 mmol) was subjected to the same synthetic procedure as for Compound 2 to give Compound 73 (16 mg, yield 39 %) as a white solid.

### Preparation Example 74: Synthesis of Compound 74

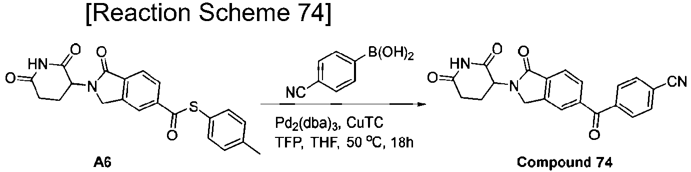

(4-Cyanophenyl)boronic acid (88 mg, 0.60 mmol) and Compound A6 (200 mg, 0.51 mmol) were subjected to the same synthetic procedure as for Compound 1 to give Compound 74 (9.3 mg, yield 4.9 %) as a white solid.

### Preparation Example 75: Synthesis of Compound 75

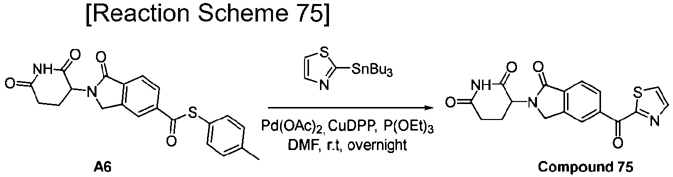

2-(Tributylstannyl)thiazole (95 mg, 0.25 mmol) was subjected to the same synthetic procedure as for Compound 65 to give Compound 75 (8.8 mg, yield 9.7 %) as a white solid.

### Preparation Example 76: Synthesis of Compound 76

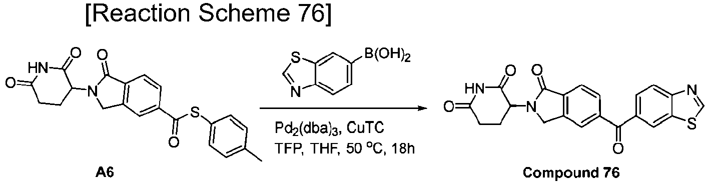

Benzo[d]thiazol-6-ylboronic acid (102 mg, 0.57 mmol) and Compound A6 (205 mg, 0.52 mmol) were subjected to the same synthetic procedure as for Compound 1 to give Compound 76 (10.3 mg, yield 5.3 %) as a white solid.

### Preparation Example 77: Synthesis of Compound 77

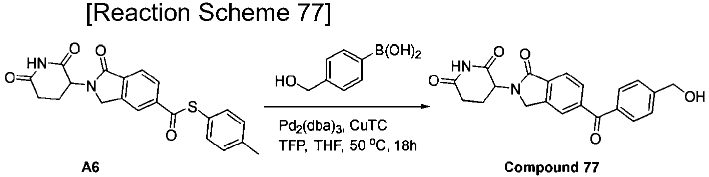

(4-(Hydroxymethyl)phenyl)boronic acid (139 mg, 0.91 mmol) and Compound A6 (300 mg, 0.76 mmol) were subjected to the same synthetic procedure as for Compound 1 to give Compound 77 (6.7 mg, yield 2.3 %) as a white solid.

### Example 1: Analysis of Synthesized Compounds

To analyze the properties of the compounds synthesized in the Preparation Examples, LC-MS and NMR analyses were performed.

As a result, the structures and IUPAC names of the compounds are shown in Tables 1 to 8, and the analysis results (LC-MS and NMR) of the compounds are shown in Tables 9 to 16 below.

**[Table 1]**

| Compounds | Structure | IUPAC Name | Formula No. |
|---|---|---|---|
| Compound 1 | 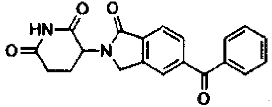 | 3-(5-benzoyl-1-oxoisoindolin-2-yl)piperidine-2,6-dione | 1 |
| Compound 2 | 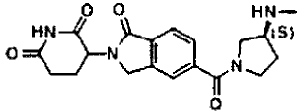 | 3-(5-((S)-3-{methylamino)pyrrolidine-1-carbonyl)-1-oxoisoindolin-2-yl)piperidine-2,6-dione | 2 |
| Compound 3 | 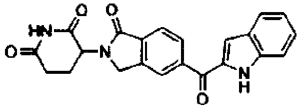 | 3-(5-(1H-indole-2-carbonyl)-1-oxoisoindolin-2-yl)piperidine-2,6-dione | 3 |
| Compound 4 | 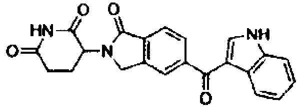 | 3-(5-(1H-indole-3-carbonyl)-1-oxoisoindolin-2-yl)piperidine-2.6-dione | 4 |
| Compound 5 | 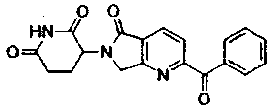 | 3-(2-benzoyl-5-oxo-5,7-dihydro-6H-pyrrolo[3.4-b]pyridin-6-yl)piperidine-2,6-dione | 5 |
| Compound 6 | 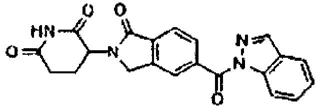 | 3-(5-(1H-indazole-1-carbonyl)-1-oxoisoindolin-2-yl)piperidine-2,6-dione | 6 |
| Compound 7 | 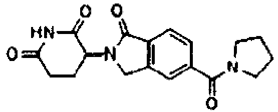 | 3-(1-oxo-5-(pyrrolidine-1-carbonyl)isoindolin-2-yl)piperidine-2,6-dione | 7 |
| Compound 8 | 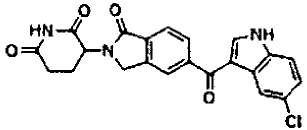 | 3-(5-(5-chloro-1H-indole-3-carbonyl)-1-oxoisoindolin-2-yl)piperidine-2,6-dione | 8 |
| Compound 9 | 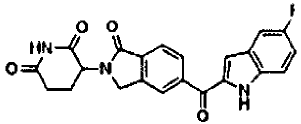 | 3-(5-(5-fluoro-1H-indole-2-carbonyl)-1-oxoisoindolin-2-yl)piperidine-2,6-dione | 9 |
| Compound 10 | 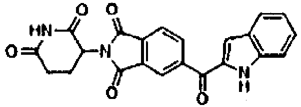 | 2-(2,6-dioxopiperidin-3-yl)-5-(1H-indole-2-carbonyl)isoindoline-1,3-dione | 10 |

**[Table 2]**

| Compounds | Structure | IUPAC Name | Formula No. |
|---|---|---|---|
| Compound 11 | 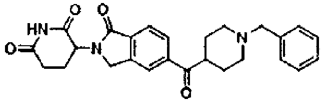 | 3-(5-(1-benzylpiperidine-4-carbonyl)-1-ox.oisoindolin-2-yl)piperidine-2,6-dione | 11 |
| Compound 12 | 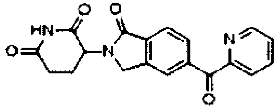 | 3-(1-oxo-5-picolinoylisoindelin-2-yi)piperidine-2,6-dione | 12 |
| Compound 13 | 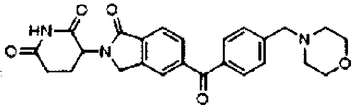 | 3-(5-(4-(morpholinomethyl)benzoyl)-1-oxoisoindolin-2-yl)piperidine-2,6-dione | 13 |
| Compound 14 | 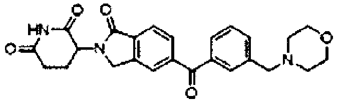 | 3-(5-(3-(morpholinomethyl)benzoyl)-1-oxoisoindolin-2-yl)piperidine-2,6-dione | 14 |
| Compound 15 | 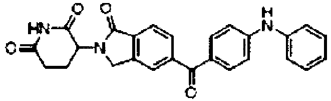 | 3-(1-oxo-5-(4-(phenylamino)benzoyl)isoindolin-2-yl)piperidine-2,6-dione | 15 |
| Compound 16 | 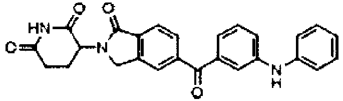 | 3-(1-oxo-5-(3-(phenylamino)benzoyl)isoindolin-2-yl)piperidine-2,6-dione | 16 |
| Compound 17 | 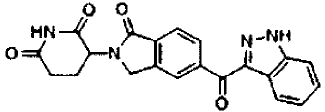 | 3-(5-(1H-indazole-3-carbonyl)-1-oxoisoindolin-2-yl)piperidine-2,6-dione | 17 |
| Compound 18 | 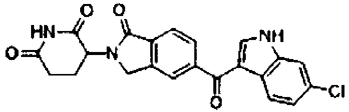 | 3-(5-(6-chloro-1H-indole-3-carbonyl)-1-oxoisoindolin-2-yl)piperidine-2,6-dione | 18 |
| Compound 19 | 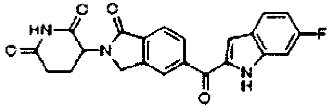 | 3-(5-(6-fluoro-1H-indole-2-carbonyl)-1-oxoisoindolin-2-yl)piperidine-2,6-dione | 19 |
| Compound 20 | 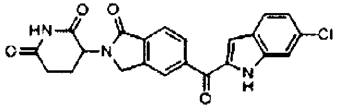 | 3-(5-(6-chloro-1H-indole-2-carbonyl)-1-oxoisoindolin-2-yl)piperidine-2,6-dione | 20 |

**[Table 3]**

| Compounds | Structure | IUPAC Name | Formula No. |
|---|---|---|---|
| Compound 21 | 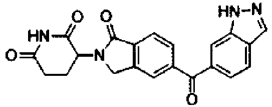 | 3-(5-(1H-indazole-6-carbonyl)-1-oxoisoindolin-2-yl)piperidine-2,6-dione | 21 |
| Compound 22 | 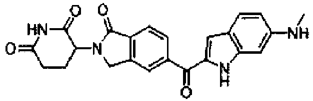 | 3-(5-(6-(methylamino)-1H-indole-2-carbonyl)-1-oxoisoindolin-2-yl)piperidine-2,6-dione | 22 |
| Compound 23 | 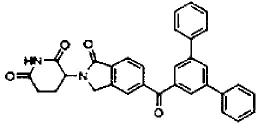 | 3-(5-([1,1':3',1"-terphenyl]-5'-carbonyl)-1-oxoisoindolin-2-yl)piperidine-2,6-dione | 23 |
| Compound 24 | 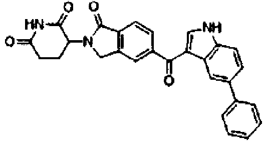 | 3-(1-oxo-5-(5-phenyl-1H-indole-3-carbonyl)isoindolin-2-yl)piperidine-2,6-dione | 24 |
| Compound 25 | 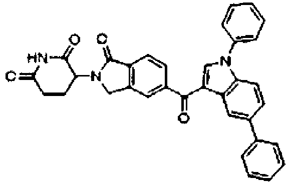 | 3-(5-(1,5-diphenyl-1H-indole-3-carbonyl)-1-oxoisoindolin-2-yl)piperidine-2,6-dione | 25 |
| Compound 26 | 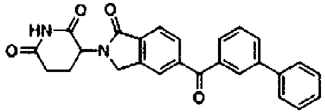 | 3-(5-([1,1'-biphenyl]-3-carbonyl)-1-oxoisoindolin-2-yl)piperidine-2,6-dione | 26 |
| Compound 27 | 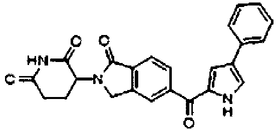 | 3-(1-oxo-5-(4-phenyl-1H-pyrrole-2-carbonyl)isoindolin-2-yl)piperidine-2,6-dione | 27 |
| Compound 28 | 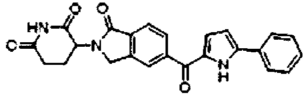 | 3-(1-oxo-5-(5-phenyl-1H-pyrrole-2-carbonyl)isoindolin-2-yl)piperidine-2,6-dione | 28 |
| Compound 29 | 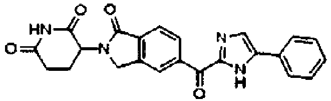 | 3-(1-oxo-5-(5-phenyl-1H-imidazole-2-carbonyl)isoindolin-2-yl)piperidine-2,6-dione | 29 |
| Compound 30 | 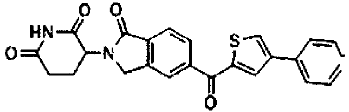 | 3-(1-oxo-5-(4-phenylthiophene-2-carbonyl)isoindolin-2-yl)piperidine-2,6-dione | 30 |

**[Table 4]**

| Compounds | Structure | IUPAC Name | Formula No. |
|---|---|---|---|
| Compound 31 | 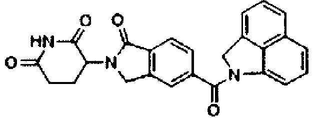 | 3-(5-(1,2-dihydrobenxo[cd]indole-1-carbonyl)-1-oxoisoindolin-2-yl)piperidine-2,6-dione | 31 |
| compound 32 | 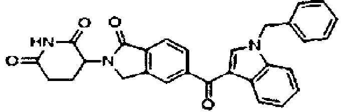 | 3-(5-(1-benzyl-1H-indole-3-carbonyl)-1-oxoisoindolin-2-yl)piperidine-2.6-dione | 32 |
| Compound 33 | 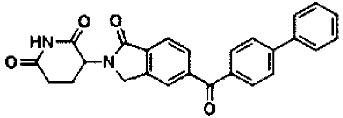 | 3-(5-([1,1'-biphenyl]-4-carbonyl)-1-oxoisoindolin-2-yl)piperidine-2,6-dione | 33 |
| Compound 34 | 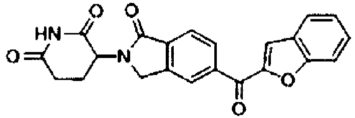 | 3-(5-(benzofuran-2-carbonyl)-1-oxoisoindolin-2-yl)piperidine-2,6-dione | 34 |
| compound 35 | 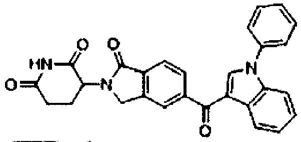 | 3-(1-oxo-5-(1-phenyl-1H-indole-3-carbonyl)isoindolin-2-yl)piperidine-2,6-dione | 35 |
| Compound 36 | 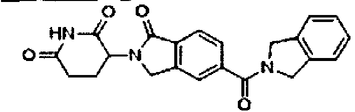 | 3-(5-(isoindoline-2-carbonyl)-1-oxoisoindolin-2-yl)piperidine-2,6-dione | 36 |
| Compound 37 | 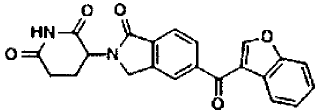 | 3-(5-(benzofuran-3-carbonyl)-1-oxoisoindolin-2-yl)piperidine-2,6-dione | 37 |
| Compound 38 | 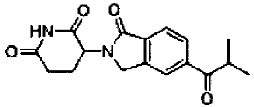 | 3-(5-isobutyryl-1-oxoisoindolin-2-yl)piperidine-2,6-dione | 38 |
| Compound 39 | 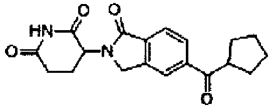 | 3-(5-(cyclopentanecarbonyl)-1-oxoisoindolin-2-yl)piperidine-2,6-dione | 39 |
| compound 40 | 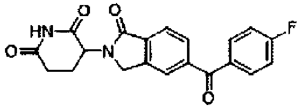 | 3-(5-(4-fluorobenzoyl)-1-oxoisoindolin-2-yl)piperidine-2,6-dione | 40 |

**[Table 5]**

| Compounds | Structure | IUPAC Name | Formula No. |
|---|---|---|---|
| Compound 41 | 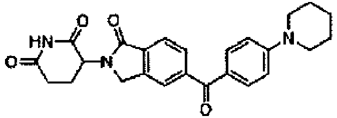 | 3-(1-oxo-5-(4-(piperidin-1-yl)benzoyl)isoindolin-2-yl)piperidine-2,6-dione | 41 |
| Compound 42 | 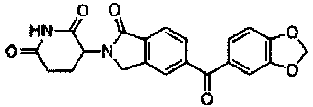 | 3-(5-(benzo[d][1,3]dioxole-5-carbonyl)-1-oxoisoindolin-2-yl)piperidine-2,6-dione | 42 |
| Compound 43 | 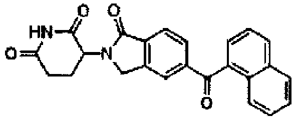 | 3-(5-(1-naphthoyl)-1-oxoisoindolin-2-yl)piperidine-2,6-dione | 43 |
| Compound 44 | 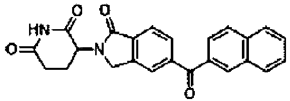 | 3-(5-(2-naphthoyl)-1-oxoisoindolin-2-yl)piperidine-2,6-dione | 44 |
| Compound 45 | 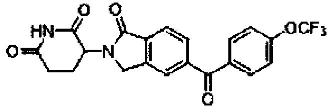 | 3-(1-oxo-5-(4-(trifluoromethoxy)benzoyl)isoindol in-2-yl)piperidine-2,6-dione | 45 |
| Compound 46 | 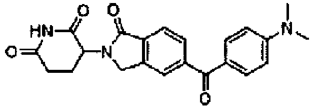 | 3-(5-(4-(dimethylamino)benzoyl)-1-oxoisoindolin-2-yl)piperidine-2,6-dione | 46 |
| Compound 47 | 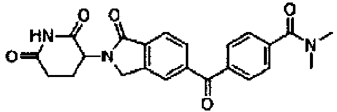 | 4-(2-(2,6-dioxopiperidin-3-yl)-1-oxoisoindoline-5-carbonyl)-N,N-dimethylbenzamide | 47 |
| Compound 48 | 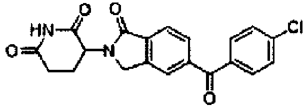 | 3-(5-(4-chlorobenzoyl)-1-oxoisoindolin-2-yl)piperidine-2,6-dione | 48 |
| Compound 49 | 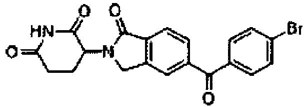 | 3-(5-(4-bromobenzoyl)-1-oxeisoindolin-2-yl)piperidine-2,6-dione | 49 |
| Compound 50 | 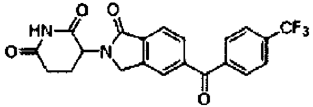 | 3-(1-oxo-5-(4-(trifluoromethyl)benzoyl)isoindoli n-2-yl)piperidine-2,6-dione | 50 |

**[Table 6]**

| Compounds | Structure | IUPAC Name | Formula No. |
|---|---|---|---|
| Compound 51 | 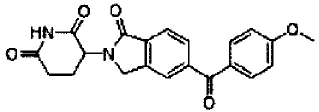 | 3-(5-(4-methoxybenzoyl)-1-oxoisoindolin-2-yl)piperidine-2,6-dione | 51 |
| Compound 52 | 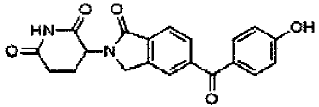 | 3-(5-(4-hydroxybenzoyl)-1-oxoisoindolin-2-yl)piperidine-2,6-dione | 52 |
| Compound 53 | 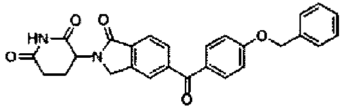 | 3-(5-(4-(benzyloxy)benzoyl)-1-oxoisoindolin-2-yl)piperidine-2,6-dione | 53 |
| Compound 54 | 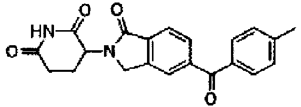 | 3-(5-(4-methylbenzoyl)-1-oxoisoindolin-2-yl)piperidine-2,6-dione | 54 |
| Compound 55 | 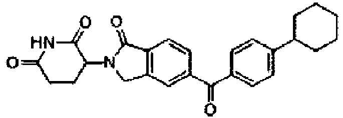 | 3-(5-(4-cyclohexylbenzoyl)-1-oxoisoindolin-2-yl)piperidine-2,6-dione | 55 |
| Compound 56 | 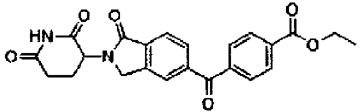 | ethyl 4-(2-(2,6-dioxopiperidin-3-yl)-1-oxoisoindoline-5-carbonyl)benzoate | 56 |
| Compound 57 | 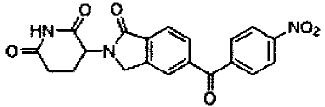 | 3-(5-(4-nitrobenzoyl)-1-oxoisoindolin-2-yl)piperidine-2,6-dione | 57 |
| Compound 58 | 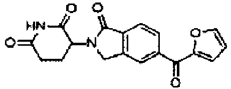 | 3-(5-(furan-2-carbonyl)-1-oxoisoindolin-2-yl)piperidine-2,6-dione | 58 |
| Compound 59 | 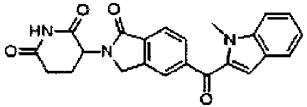 | 3-(5-(1-methyl-1H-indole-2-carbonyl)-1-oxoisoindolin-2-yl)piperidine-2,6-dione | 59 |
| Compound 60 | 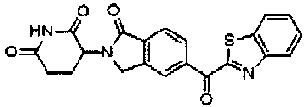 | 3-(5-(benzo[d]thiazole-2-carbonyl)-1-oxoisoindolin-2-yl)piperidine-2,6-dione | 60 |

**[Table 7]**

| Compounds | Structure | IUPAC Name | Formula No. |
|---|---|---|---|
| Compound 61 | 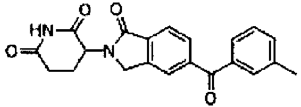 | 3-(5-(3-methylbenzoyl)-1-oxoisoindolin-2-yl)piperidine-2,6-dione | 61 |
| Compound 62 | 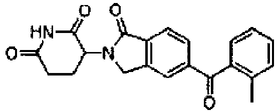 | 3-(5-(2-methylbenzoyl)-1-oxoisoindolin-2-yl)piperidine-2,6-dione | 62 |
| Compound 63 | 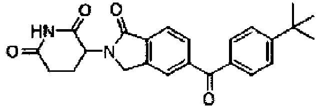 | 3-(5-(4-(tert-butyl)benzoyl)-1-oxoisoindolin-2-yl)piperidine-2,6-dione | 63 |
| Compound 64 | 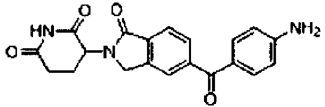 | 3-(5-(4-aminobenzoyl)-1-oxoisoindolin-2-yl)piperidine-2,6-dione | 64 |
| Compound 65 | 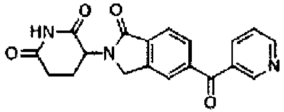 | 3-(5-nicotinoyl-1-oxoisoindolin-2-yl)piperidine-2,6-dione | 65 |
| Compound 66 | 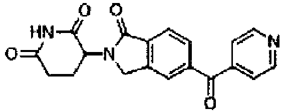 | 3-(5-isonicotinoyl-1-oxoisoindolin-2-yl)piperidine-2,6-dione | 66 |
| Compound 67 | 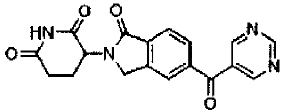 | 3-(1-oxo-5-(pyrimidine-5-carbonyl)isoindolin-2-yl)piperidine-2,6-dione | 67 |
| Compound 68 | 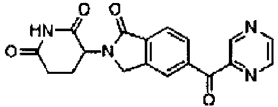 | 3-(1-oxo-5-(pyrazine-2-carbonyl)isoindolin-2-yl)piperidine-2,6-dione | 68 |
| Compound 69 | 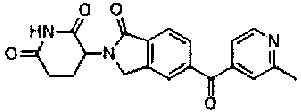 | 3-(5-(2-methylisonicotinoyl)-1-oxoisoindolin-2-yl)piperidine-2,6-dione | 69 |
| Compound 70 | 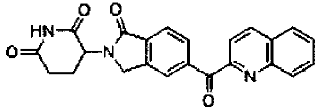 | 3-(1-oxo-5-(quinoline-2-carbonyl)isoindolin-2-yl)piperidine-2,6-dione | 70 |

**[Table 8]**

| Compounds | Structure | IUPAC Name | Formula No. |
|---|---|---|---|
| Compound 71 | 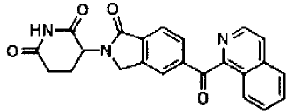 | 3-(5-(isoquinoline-1-carbonyl)-1-oxoisoindolin-2-yl)piperidine-2,6-dione | 71 |
| Compound 72 | 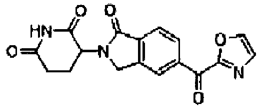 | 3-(5-(oxazole-2-carbonyl)-1-oxoisoindolin-2-yl)piperidine-2,6-dione | 72 |
| Compound 73 | 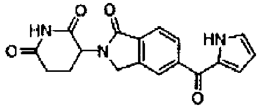 | 3-(1-oxo-5-(1H-pyrrole-2-carbonyl)isoindolin-2-yl)piperidine-2,6-dione | 73 |
| Compound 74 | 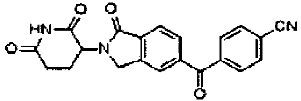 | 4-(2-(2,6-dioxopiperidin-3-yl)-1-oxoisoindoline-5-carbonyl)benzonitrile | 74 |
| Compound 75 | 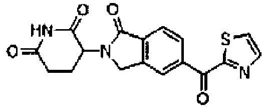 | 3-(1-oxo-5-(thiazole-2-carbonyl)isoindolin-2-yl)piperidine-2,6-dione | 75 |
| Compound 76 | 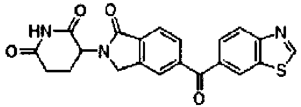 | 3-(5-(benzo[d]thiazole-6-carbonyl)-1-oxoisoindolin-2-yl)piperidine-2,6-dione | 76 |
| Compound 77 | 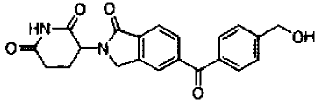 | 3-(5-(4-(hydroxymethyl)benzoyl)-1-oxoisoindolin-2-yl)piperidine-2,6-dione | 77 |

**[Table 9]**

| **Compounds** | **Characterization Data** |
|---|---|
| **Compound 1** | White solid; ¹H NMR (400 MHz, DMSO-d₆): δ 11.02 (s, 1H), 7.96 (s, 1H), 7.90 (d, J = 8.0 Hz, 1H), 7.84 (d, J = 8.0 Hz, 1H), 7.80 - 7.78 (m, 2H), 7.74 - 7.70 (m, 1H), 7.61 - 7.57 (m, 2H), 5.19 - 5.15 (m, 1H), 4.59 - 4.42 (m, 2H), 2.98 - 2.88 (m, 1H), 2.67 - 2.59 (m, 1H), 2.46 - 2.36 (m, 1H), 2.06 - 2.02 (m, 1H).; |
| | LC-MS (ESI): m/z 349.15 [M+H]⁺. |
| **Compound 2** | White solid; ¹H NMR (400 MHz, MeOD): δ 7.90 (d, J = 7.6 Hz, 1H), 7.79 (s, 1H), 7.71 (d, J = 7.6 Hz, 1H), 5.20 - 5.16 (m, 1H), 4.62 - 4.51 (m, 2H), 4.03 - 3.85 (m, 3H), 3.78 - 3.62 (m, 2H), 2.97 - 2.87 (m, 1H), 2.81 - 2.70 (m, 4H), 2.56 - 2.40 (m, 2H), 2.21 - 2.18 (m, 2H).; LC-MS (ESI): m/z 371.25 [M+H]⁺. |
| **Compound 3** | White solid ; ¹H NMR (400 MHz, DMSO-d₆): δ 12.05 (s, 1H), 11.03 (s, 1H), 8.16 (s, 1H), 8.03 (d, J = 8.0 Hz, 1H), 7.92 (d, J = 8.0 Hz, 1H), 7.72 (d, J = 8.4 Hz, 1H), 7.52 (d, J = 8.0 Hz, 1H), 7.36 - 7.32 (m, 1H), 7.22 (s, 1H), 7.13 - 7.10 (m, 1H), 5.21-5.18 (m, 1H), 4.63 - 4.46 (m, 2H), 2.98-2.90 (m, 1H), 2.66-2.56 (m, 1H), 2.46-2.33 (m, 1H), 2.08-2.04 (m, 1H).; |
| | LC-MS (ESI): m/z 388.25 [M+H]⁺. |
| **Compound 4** | White solid; ¹H NMR (400 MHz, DMSO-d₆): δ 12.16 (s, 1H), 11.02 (s, 1H), 8.29 - 8.27 (m, 1H), 8.05 - 8.01 (m, 2H), 7.89 - 7.86 (m, 2H), 7.54 - 7.53 (m, 1H), 7.30 - 7.25 (m, 2H), 5.20 - 5.16 (m, 1H), 4.60 - 4.43 (m, 2H), 2.95 - 2.90 (m, 1H), 2.64 - 2.60 (m, 1H), 2.46 - 2.39 (m, 1H), 2.06 - 2.04 (m, 1H).; |
| | LC-MS (ESI): m/z 388.10 [M+H]⁺. |
| **Compound 5** | White solid; ¹H NMR (400 MHz, DMSO-d₆): δ 11.05 (s, 1H), 8.40 (d, 1H), 8.08 (d, 1H), 7.98 (m, 2H), 7.73 - 7.69 (m, 1H), 7.58 - 7.54 (m, 2H), 5.26 - 5.21 (m, 1H), 4.65-4.44 (m, 2H), 2.99 - 2.89 (m, 1H), 2.65 - 2.58 (m, 1H), 2.46 - 2.40 (m, 1H), 2.07 - 2.04 (m, 1H).; |
| | LC-MS (ESI): m/z 350.20 [M+H]⁺. |
| **Compound 6** | White solid; ¹H NMR (400 MHz, DMSO-d₆): δ 11.03 (s, 1H), 8.57 (s, 1H), 8.48 (d, 1H), 8.19 (s, 1H), 8.08 (d, 1H), 7.99 (d, 1H), 7.90 (d, 1H), 7.73 (t, 1H), 7.52 (t, 1H), 5.20-5.16 (m, 1H), 4.62 - 4.43 (m, 2H), 2.97 - 2.89 (m, 1H), 2.65 - 2.60 (m, 1H), 2.48 - 2.41 (m, 1H), 2.09 - 2.03 (m, 1H).; |
| | LC-MS (ESI): m/z 389.00 [M+H]⁺. |
| **Compound 7** | White solid; ¹H NMR (400 MHz, DMSO-d₆): δ 11.02 (s, 1H), 7.78-7.74 (m, 2H), 7.62 (d, J = 8.0 Hz, 1H), 5.16 - 5.12 (m, 1H), 4.52-4.35 (m, 2H), 3.51 - 3.46 (m, 2H), 3.39 - 3.34 (m, 2H), 2.98 - 2.90 (m, 1H), 2.69 - 2.56 (m, 1H), 2.46 - 2.36 (m, 1H), 2.04 - 2.00 (m, 1H), 1.90 - 1.78 (m, 4H).; |
| | LC-MS (ESI): m/z 342.05 [M+H]⁺. |
| **Compound 8** | White solid; ¹H NMR (400 MHz, DMSO-d₆): δ 12.35 (s, 1H), 11.03 (s, 1H), 8.27 (s, 1H), 8.12 (d, 1H), 8.01 (s, 1H), 7.91 - 7.86 (m, 2H), 7.56 (d, J = 8.8 Hz, 1H), 7.31 (d, J = 8.8, 1H), 5.20-5.15 (m, 1H), 4.59 - 4.43 (m, 2H), 2.98 - 2.89 (m, 1H), 2.67 - 2.58 (m, 1H), 2.48 - 2.45 (m, 1H), 2.08 - 2.03 (m, 1H).; |
| | LC-MS (ESI): m/z 422.10 [M+H]⁺. |
| **Compound 9** | White solid; ¹H NMR (400 MHz, DMSO-d₆): 12.17 (s, 1H), 11.04 (s, 1H), 8.16 (s, 1H), 8.03 (d, J = 8.0 Hz, 1H), 7.92 (d, J = 8.0 Hz, 1H), 7.56 - 7.46 (m, 2H), 7.24 - 7.19 (m, 2H), 5.21-5.16 (m, 1H), 4.62 - 4.45 (m, 2H), 2.98 - 2.89 (m, 1H), 2.66 - 2.60 (m, 1H), 2.48 - 2.42 (m, 1H), 2.08 - 2.04 (m, 1H).; LC-MS (ESI): m/z 405.90 [M+H]⁺. |
| **Compound 10** | Yellow solid; ¹H NMR (400 MHz, DMSO-d₆): δ 12.13 (s, 1H), 11.17 (s, 1H), 8.36 (d, J = 7.6 Hz, 1H), 8.29 (s, 1H), 8.12 (d, J = 8.0 Hz, 1H), 7.74 (d, 1H), 7.53 (d, J = 8.4 Hz, 1H), 7.36 (t, J = 7.8 Hz, 1H), 7.20 (s, 1H), 7.13 (t, J =7.6 Hz, 1H), 5.26 - 5.21 (m, 1H), 2.97 - 2.88 (m, 1H), 2.69 - 2.54 (m, 2H), 2.14 - 2.07 (m, 1H). ; |
| | LC-MS (ESI): m/z 402.05 [M+H]⁺. |

**[Table 10]**

| **Compounds** | **Characterization Data** |
|---|---|
| **Compound 11** | White solid; ¹H NMR (400 MHz, DMSO-d₆): δ 11.01 (s, 1H), 8.20 (s, 1H), 8.08 (d, J = 8.0 Hz, 1H), 7.85 (d, J = 8.0 Hz, 1H), 7.34 - 7.23 (m, 5H), 5.17 - 5.12 (m, 1H), 4.56 - 4.39 (m, 2H), 3.52 - 3.40 (m, 3H), 2.95 - 2.84 (m, 3H), 2.65 - 2.56 (m, 1H), 2.44 - 2.36 (m, 1H), 2.20 - 1.96 (m, 3H), 1.81 - 1.75 (m, 2H), 1.63 - 1.55 (m, 2H).; |
| | LC-MS (ESI): m/z 446.10 [M+H]⁺. |
| **Compound 12** | White solid; ¹H NMR (400 MHz, DMSO-d₆): δ 11.03 (s, 1H), 8.75 (d, J = 4.8 Hz, 1H), 8.19 (s, 1H), 8.15 - 8.06 (m, 3H), 7.88 (d, 1H), 7.73 - 7.70 (m, 1H), 5.19 - 5.15 (m, 1H), 4.59 - 4.41 (m, 2H), 2.97 - 2.89 (m, 1H), 2.68 - 2.56 (m, 1H), 2.45 - 2.36 (m, 1H), 2.08 - 2.03 (m, 1H).; |
| | LC-MS (ESI): m/z 350.05 [M+H]⁺. |
| **Compound 13** | Light yellow solid ; ¹H NMR (400 MHz, DMSO-d₆): δ 11.02 (s, 1H), 7.95 (s, 1H), 7.89 (d, J = 7.6 Hz, 1H), 7.83 (d, J = 7.6 Hz, 1H), 7.76 (d, J = 8.0 Hz, 2H), 7.52 (d, J = 8.0 Hz, 2H), 5.19-5.14 (m, 1H), 4.58-4.41 (m, 2H), 3.61-3.58 (m, 6H), 2.98-2.88 (m, 1H), 2.68-2.59 (m, 1H), 2.46-2.39 (m, 5H), 2.07-2.03 (m, 1H).; |
| | LC-MS (ESI): m/z 448.20 [M+H]⁺. |
| **Compound 14** | White solid; ¹H NMR (400 MHz, DMSO-d₆): δ 11.02 (s, 1H), 7.95 (s, 1H), 7.89 (d, J = 8.0 Hz, 1H), 7.84 (d, J = 7.6 Hz, 1H), 7.72 (s, 1H), 7.68 - 7.64 (m, 2H), 7.54 (t, J = 7.6 Hz, 1H), 5.19 - 5.15 (m, 1H), 4.59 - 4.42 (m, 2H), 3.58 - 3.55 (m, 6H), 2.96 - 2.86 (m, 1H), 2.65 - 2.57 (m, 1H), 2.46 - 2.37 (m, 5H), 2.09 - 2.02 (m, 1H).;LC-MS (ESI): m/z 448.05 [M+H]⁺. |
| **Compound 15** | yellow solid; ¹H NMR (400 MHz, DMSO-d₆): δ 11.02 (s, 1 H), 8.97 (s, 1 H), 7.88-7.85 (m, 2 H), 7.76 (d, J = 8.4 Hz, 1 H), 7.71 (d, J = 8.8 Hz, 2 H), 7.37-7.33 (m, 2 H), 7.24-7.22 (m, 2 H), 7.11 (d, J = 8.8 Hz, 2 H), 7.03 (t, J = 7.6 Hz, 1 H), 5.19-5.15 (m, 1 H), 4.58-4.41 (m, 2 H), 2.96-2.89 (m, 1 H), 2.66-2.58 (m, 1 H), 2.46-2.38 (m, 1 H), 2.07-2.02 (m, 1 H).; LC-MS (ESI): m/z 440.15 [M+H]⁺. |
| **Compound 16** | yellow solid; ¹H NMR (400 MHz, DMSO-d₆): 11.03 (s, 1H), 8.46 (s, 1H), 7.98 (s, 1H), 7.91 - 7.84 (m, 2H), 7.45 - 7.35 (m, 3H), 7.29 - 7.24 (m, 2H), 7.22-7.19 (m, 1H), 7.15-7.12 (m, 2H), 6.90-6.87 (t, 1H), 5.20 - 5.15 (m, 1H), 4.58 - 4.42 (m, 2H), 2.97 - 2.88 (m, 1H), 2.65 - 2.60 (m, 1H), 2.46 - 2.40 (m, 1H), 2.08 - 2.03 (m, 1H).; LC-MS (ESI): m/z 440.15 [M+H]⁺. |
| **Compound 17** | White solid; ¹H NMR (400 MHz, DMSO-d₆): δ 14.11 (s, 1H), 11.04 (s, 1H), 8.43 (s, 1H), 8.37-8.32 (m, 2H), 7.92 (d, 1H), 7.76 (d, 1H), 7.53 (t, 1H), 7.41 (t, 1H), 5.21 - 5.16 (m, 1H), 4.63 - 4.45 (m, 2H), 2.97 - 2.89 (m, 1H), 2.68 - 2.58 (m, 1H), 2.47 - 2.42 (m, 1H), 2.08 - 2.02 (m, 1H).; LC-MS (ESI): m/z 388.95 [M]⁺. |
| **Compound 18** | White solid; ¹H NMR (400 MHz, DMSO-d₆): δ 12.28 (s, 1H), 11.04 (s, 1H), 8.26 (d, J = 8.4 Hz, 1H), 8.08 (s, 1H), 8.01 (s, 1H), 7.91 - 7.86 (m, 2H), 7.58 (s, 1H), 7.30 (d, 1H), 5.21-5.16 (m, 1H), 4.60 - 4.43 (m, 2H), 2.95 - 2.89 (m, 1H), 2.66 - 2.59 (m, 1H), 2.46 - 2.41 (m, 1H), 2.08 - 2.03 (m, 1H).; LC-MS (ESI): m/z 422.10 [M+H]⁺. |
| **Compound 19** | Yellow solid; ¹H NMR (400 MHz, DMSO-d₆): δ 12.15 (s, 1H), 11.03 (s, 1H), 8.15 (s, 1H), 8.02 (d, J = 8.0 Hz, 1H), 7.92 (d, J = 8.0 Hz, 1H), 7.78 - 7.75 (m, 1H), 7.25 - 7.20 (m, 2H), 7.03 - 6.99 (m, 1H), 5.21-5.16 (m, 1H), 4.62 - 4.45 (m, 2H), 2.98 - 2.89 (m, 1H), 2.64 - 2.59 (m, 1H), 2.46 - 2.40 (m, 1H), 2.07 - 2.04 (m, 1H).; LC-MS (ESI): m/z 406.10 [M+H]⁺. |
| **Compound 20** | Light brown solid; ¹H NMR (400 MHz, DMSO-d₆): δ 12.20 (s, 1H), 11.04 (s, 1H), 8.17 (s, 1H), 8.04 (d, J = 7.6 Hz, 1H), 7.92 (d, J = 7.6 Hz, 1H), 7.76 (d, J = 8.8 Hz, 1H), 7.53 (s, 1H), 7.26 (s, 1H), 7.15 (d, J = 8.8, 1H), 5.22 - 5.17 (m, 1H), 4.62 - 4.46 (m, 2H), 2.95 -2.90 (m, 1H), 2.68 - 2.58 (m, 1H), 2.47 - 2.42 (m, 1H), 2.08 - 2.04 (m, 1H).; LC-MS (ESI): m/z 422.05 [M+H]⁺. |

**[Table 11]**

| **Compounds** | **Characterization Data** |
|---|---|
| **Compound 21** | White solid; ¹H NMR (400 MHz, DMSO-d₆): δ 13.43 (s, 1H), 11.03 (s, 1H), 8.25 (s, 1H), 8.01 (s, 1H), 7.96 - 7.88 (m, 4H), 7.58 - 7.56 (m, 1H), 5.21 - 5.16 (m, 1H), 4.59 - 4.42 (m, 2H), 2.98 - 2.89 (m, 1H), 2.68 - 2.60 (m, 1H), 2.46 - 2.40 (m, 1H), 2.07 - 2.03 (m, 1H).; LC-MS (ESI): m/z 388.90 [M+H]⁺. |
| **Compound 22** | Orange solid; ¹H NMR (400 MHz, DMSO-d₆) δ 11.49 (s, 1 H), 11.03 (s, 1 H), 8.07 (s, 1 H), 7.95 (d, J = 7.6 Hz, 1 H), 7.87 (d, J = 7.6 Hz, 1 H), 7.39 (d, J = 8.4 Hz, 1 H), 7.01 (s, 1 H), 6.57 (d, J = 8.8 Hz, 1 H), 6.39 (s, 1 H), 6.16 (br s, 1 H), 5.20 - 5.15 (m, 1H), 4.60 - 4.43 (m, 2H), 2.98 - 2.89 (m, 1 H), 2.74 (s, 3 H), 2.66 - 2.58 (m, 1 H), 2.44 - 2.40 (m, 1 H), 2.10 - 2.00 (m, 1 H).; LC-MS (ESI): m/z 417.15 [M+H]⁺. |
| **Compound 23** | white solid; ¹H NMR (400 MHz, DMSO-d₆) δ 11.03 (s, 1H), 8.23 (s, 1H), 8.09 (s, 1H), 7.98 - 7.91 (m, 4H), 7.85 - 7.83 (m, 4H), 7.54 - 7.50 (m, 4H), 7.46 - 7.42 (m, 2H), 5.19 - 5.15 (m, 1H), 4.61 - 4.44 (m, 2H), 2.97 - 2.88 (m, 1H), 2.64 - 2.59 (m, 1H), 2.44 - 2.40 (m, 1H), 2.08 - 2.03 (m, 1H).; |
| | LC-MS (ESI): m/z 501.2 [M+H]⁺. |
| **Compound 24** | white solid; ¹H NMR (400 MHz, DMSO-d₆) δ 12.25 (s, 1H), 11.03 (s, 1H), 8.54 (s, 1H), 8.07 (d, J = 3.2 Hz, 1H), 8.02 (s, 1H), 7.92 - 7.87 (m, 2H), 7.70 - 7.68 (m, 2H), 7.64 - 7.58 (m, 2H), 7.50 (t, J = 7.6 Hz, 2H), 7.36 (t, J = 7.2 Hz, 1H), 5.21 - 5.16 (m, 1H), 4.61 - 4.44 (m, 2H), 2.99 - 2.90 (m, 1H), 2.65 - 2.61 (m, 1H), 2.47 - 2.43 (m, 1H), 2.08 - 2.04 (m, 1H).;LC-MS (ESI): m/z 463.75 [M]+, 464.80 [M+H]⁺. |
| **Compound 25** | white solid; ¹H NMR (400 MHz, DMSO-d₆) δ 11.02 (s, 1H), 8.67 (s, 1H), 8.33 (s, 1H), 8.14 (s, 1H), 8.01 (d, J = 8.0 Hz, 1H), 7.89 (d, J = 8.0 Hz, 1H), 7.76 - 7.72 (m, 4H), 7.67 - 7.63 (m, 4H), 7.56 - 7.50 (m, 3H), 7.41 - 7.37 (m, 1H), 5.20 - 5.15 (m, 1H), 4.61 - 4.45 (m, 2H), 2.98 - 2.89 (m, 1H), 2.65 - 2.60 (m, 1H), 2.45 - 2.40 (m, 1H), 2.05 - 2.03 (m, 1H).;LC-MS (ESI): m/z 540.2 [M+H]⁺. |
| **Compound 26** | white solid; ¹H NMR (400 MHz, DMSO-d₆): δ 11.03 (s, 1H), 8.03 - 8.00 (m, 3H), 7.91 (m, 2H), 7.78 - 7.75 (m, 1H), 7.74 - 7.67 (m, 3H), 7.51 - 7.48 (m, 2H), 7.43 - 7.39 (m, 1H), 5.19 - 5.15 (m, 1H), 4.60 - 4.42 (m, 2H), 2.96 - 2.88 (m, 1H), 2.63 - 2.59 (m, 1H), 2.46 - 2.36 (m, 1H), 2.07 - 2.02 (m, 1H).; |
| | MS (ESI): m/z 425.1 [M+H]⁺. |
| **Compound 27** | Yellow solid; ¹H NMR (400 MHz, DMSO-d₆): δ 12.34 (s, 1H), 11.04 (s, 1H), 8.10 (s, 1H), 7.96 (d, J = 8.0 Hz, 1H), 7.88 (d, J = 7.6 Hz, 1H), 7.76 (s, 1H), 7.67 (d, J = 7.2 Hz, 2H), 7.33 (t, 2H), 7.26 (s, 1H), 7.20 - 7.16 (m, 1H), 5.18 (dd, J = 4.8, 8.4 Hz, 1H), 4.61 - 4.57 (m, 1H), 4.49 - 4.45 (m, 1H), 2.97 - 2.89 (m, 1H), 2.64 - 2.60 (m, 1H), 2.46 - 2.39 (m, 1H), 2.08 - 2.03 (m, 1H).; LC-MS (ESI): m/z 414.1 [M+H]⁺. |
| **Compound 28** | Yellow solid; ¹H NMR (400 MHz, DMSO-d₆): δ 12.40 (s, 1H), 11.04 (s, 1H), 8.05 (s, 1H), 7.98 - 7.92 (m, 3H), 7.88 - 7.86 (m, 1H), 7.46 - 7.42 (m, 2H), 7.36 - 7.32 (m, 1H), 6.93 - 6.92 (m, 1H), 6.82 - 6.80 (m, 1H), 5.20 - 5.16 (m, 1H), 4.60 - 4.56 (m, 1H), 4.83 - 4.39 (m, 1H), 2.95 - 2.94 (m, 1H), 2.64 - 2.60 (m, 1H), 2.46 - 2.40 (m, 1H), 2.08 - 2.05 (m, 1H).;LC-MS (ESI): m/z 414.1 [M+H]⁺. |
| **Compound 29** | Yellow solid; ¹H NMR (400 MHz, DMSO-d₆): δ 13.29 (s, 1H), 10.73 (s, 1H), 8.75 (s, 1H), 8.64 (d, J = 7.6 Hz, 1H), 7.93 (t, J = 7.6 Hz, 4H), 7.50 - 7.28 (m, 3H), 5.13 (dd, J = 13.2, 5.2 Hz, 1H), 4.59 (dd, J = 40.0, 17.2 Hz, 2H), 2.97 - 2.88 (m, 1H), 2.71 - 2.63 (m, 1H), 2.46 - 2.40 (m, 1H), 2.18-2.06 (m, 1H).;LC-MS (ESI): m/z 415.2 [M+H]⁺. |
| **Compound 30** | Orange solid; ¹H NMR (400 MHz, DMSO-d₆): δ 11.05 (s, 1H), 8.50 (d, J = 1.2 Hz, 1H), 8.16 (d, J = 1.6 Hz, 2H), 8.00 (d, J = 8.8 Hz, 1H), 7.92 (d, J = 8.0 Hz, 1H), 7.80 (d, J = 7.2 Hz, 2H), 7.44 (t, J = 7.6 Hz, 2H), 7.35 (t, J = 7.2 Hz, 1H), 5.19 (dd, J = 13.2, 5.2 Hz, 1H), 4.54 (dd, J = 48.4, 18.0 Hz, 2H), 3.01 - 2.87 (m, 1H), 2.62 (d, J = 19.2 Hz, 1H), 2.47 - 2.38 (m, 1H), 2.08-2.01 (m, 1H).;LC-MS (ESI): m/z 431.0 [M+H]⁺. |

**[Table 12]**

| **Compounds** | **Characterization Data** |
|---|---|
| **Compound 31** | Yellow solid; ¹H NMR (400 MHz, DMSO-d₆): δ 11.03 (s, 1H), 8.10 - 7.76 (m, 4H), 7.72 (d, J = 8.4 Hz, 1H), 7.64-7.37 (m, 3H), 7.31 (s, 1H), 5.32 (s, 2H), 5.18 (dd, J =13.2 Hz, 4.8 Hz, 1H), 4.51 (dd, J = 51.2, 17.6 Hz, 2H), 3.01 - 2.88 (m, 1H), 2.63 (d, J = 16.4 Hz, 1H), 2.48 - 2.39 (m, 1H), 2.10 - 2.00 (m, 1H).; |
| | LC-MS (ESI): m/z 426.0 [M+H]⁺. |
| **Compound 32** | white solid; ¹H NMR (400 MHz, DMSO-d₆) δ 11.04 (s, 1H), 8.33 (s, 1H), 8.31 - 8.29 (m, 1H), 8.02 (s, 1H), 7.92 - 7.88 (m, 2H), 7.56 - 7.54 (m, 1H), 7.34 - 7.26 (m, 7H), 5.54 (s, 2H), 5.21 - 5.17 (m, 1H), 4.58 (d, J = 17.6 Hz, 1H), 4.47 (d, J = 17.6 Hz, 1H), 2.99 - 2.90 (m, 1H), 2.62 (d, J = 17.2 Hz, 1H), 2.44 - 2.36 (m, 1H), 2.06 - 2.03 (m, 1H).; |
| | LC-MS (ESI): m/z 478.2 [M+H]⁺. |
| **Compound 33** | white solid; ¹H NMR (400 MHz, DMSO-d₆) δ 11.04 (s, 1H), 8.01 (s, 1H), 7.93 - 7.88 (m, 6H), 7.79 (d, J = 7.2 Hz, 2H), 7.53 (t, J = 7.6 Hz, 2H), 7.45 (t, J = 7.2 Hz, 1H), 5.20 - 5.16 (m, 1H), 4.58 (d, J = 18.0 Hz, 1H), 4.45 (d, J = 17.6 Hz, 1H), 2.98 - 2.89 (m, 1H), 2.67 - 2.60 (m, 1H), 2.46 - 2.33 (m, 1H), 2.08 - 1.96 (m, 1H).; |
| | LC-MS (ESI): m/z 425.3 [M+H]⁺. |
| **Compound 34** | white solid; ¹H NMR (400 MHz, DMSO-d₆) δ 11.05 (s, 1H), 8.23 (s, 1H), 8.11 - 8.07 (m, 1H), 7.94 (d, J = 8.0 Hz, 1H), 7.90 - 7.87 (m, 2H), 7.82 - 7.79 (m, 1H), 7.63 - 7.59 (m, 1H), 7.44 - 7.40 (m, 1H), 5.22 - 5.17 (m, 1H), 4.60 (d, J = 17.6 Hz, 1H), 4.48 (d, J = 17.6 Hz, 1H), 2.99 - 2.89 (m, 1H), 2.68 - 2.65 (m, 1H), 2.34 - 2.32 (m, 1H), 2.08 - 2.03 (m, 1H).;LC-MS (ESI): m/z 389.1 [M+H]⁺. |
| **Compound 35** | white solid; ¹H NMR(400 MHz, DMSO-d₆) δ 11.01 (s, 1H), 8.41 (dd, J = 5.7, 3.2 Hz, 1H), 8.27 (s, 1H), 8.10 (s, 1H), 7.97 (d, J = 7.9 Hz, 1H), 7.86 (d, J = 7.8 Hz, 1H), 7.70 (d, J = 7.6 Hz, 2H), 7.64 - 7.55 (m, 3H), 7.51 (t, J = 7.4 Hz, 1H), 7.38 (p, J = 7.2 Hz, 2H), 5.16 (dd, J = 13.2, 5.0 Hz, 1H), 4.50 (dd, J = 47.0, 17.6 Hz, 2H), 2.98 - 2.85 (m, 1H), 2.60 (d, J = 17.6 Hz, 1H), 2.41 (dd, J = 13.1, 4.2 Hz, 1H), 2.09 - 1.96 (m, 1H). ;LC-MS (ESI): m/z 463.9 [M+H]⁺. |
| **Compound 36** | white solid; ¹H NMR (400 MHz, DMSO-d₆) δ 11.02 (s, 1H), 7.85-7.82 (m, 2H), 7.72 (d, J = 7.6 Hz, 1H), 7.41 (d, J = 7.2 Hz, 1H), 7.34-7.24 (m, 3H), 5.16 (dd, J1 = 13.2 Hz, J2 = 4.8 Hz, 1H), 4.90 (S, 2H), 4.79-4.72 (m, 2H), 4.53 (d, J = 17.6 Hz, 1H), 4.40 (d, J = 17.6 Hz, 1H), 2.98-2.89 (m, 1H), 2.64-2.59 (m, 1H), 2.45-2.38 (m, 1H), 2.06-2.01 (m, 1H). ; |
| | LC-MS (ESI): m/z 390.0 [M+H]⁺. |
| **Compound 37** | white solid; ¹H NMR (400 MHz, DMSO-d₆) δ 11.02 (s, 1H), 8.79 (s, 1H), 8.20 - 8.16 (m, 1H), 8.14 (s, 1H), 8.03 - 7.97 (m, 1H), 7.90 (d, J = 7.9 Hz, 1H), 7.77 (dd, J = 6.8, 1.8 Hz, 1H), 7.51 - 7.43 (m, 2H), 5.17 (dd, J = 13.3, 5.1 Hz, 1H), 4.52 (dd, J = 42.9, 17.7 Hz, 2H), 2.99 - 2.87 (m, 1H), 2.61 (dd, J = 15.2, 2.1 Hz, 1H), 2.44 (dd, J = 13.2, 4.5 Hz, 1H), 2.08 - 2.01 (m, 1H).;MS (ESI): m/z 389.0 [M+H]⁺. |
| **Compound 38** | white solid; ¹H NMR (400 MHz, DMSO-d₆) δ 11.02 (s, 1H), 8.19 (s, 1H), 8.09 (d, J = 8.0 Hz, 1H), 7.86 (d, J = 8.0 Hz, 1H), 5.15 (dd, J = 13.3, 5.1 Hz, 1H), 4.55 (d, J = 17.7 Hz, 1H), 4.42 (d, J = 17.7 Hz, 1H), 3.77 - 3.65 (m, 1H), 2.99 - 2.85 (m, 1H), 2.63-2.59 (m, 1H), 2.45-2.34 (m, 1H), 2.09 - 1.96 (m, 1H), 1.13 (d, J = 6.8 Hz, 6H).;MS (ESI): m/z 315.1 [M+H]⁺. |
| **Compound 39** | white solid; ¹H NMR (400 MHz, DMSO-d₆) δ 11.02 (s, 1H), 8.21 (s, 1H), 8.10 (d, J = 8.4 Hz, 1H), 7.86 (d, J = 7.6 Hz, 1H), 5.16-5.14 (m, 1H), 4.56-4.39 (m, 2H), 3.92-3.88 (m, 1H), 2.95-2.87 (m, 1H), 2.65-2.59 (m, 1H), 2.44-2.39 (m, 1H), 2.03-1.90 (m, 3H), 1.75-1.77 (m, 2H), 1.60-1.64 (m, 4H);MS (ESI): m/z 341.1 [M+H]⁺. |
| **Compound 40** | white solid; ¹H NMR (400 MHz, DMSO-d₆) δ 11.03 (s, 1H), 7.96 (s, 1H), 7.92-7.83 (m, 4H), 7.45-7.40 (m, 2H), 5.18 (dd, J = 13.2, 5.2 Hz, 1H), 4.57 (d, J = 5.2 Hz, 1H), 4.44 (d, J = 18.0 Hz, 1H), 2.98-2.89 (m, 1H), 2.65-2.59 (m, 1H), 2.45-2.38 (m, 1H), 2.08-2.01 (m, 1H).;MS (ESI): m/z 367.1 [M+H]⁺. |

**[Table 13]**

| **Compounds** | **Characterization Data** |
|---|---|
| **Compound 41** | yellow solid; ¹H NMR (400 MHz, DMSO-d₆) δ 11.02 (s, 1H), 7.86-7.84 (m, 2H), 7.73 (d, J = 8.8 Hz,1H), 7.66 (d, J = 9.2 Hz, 2H), 7.00 (d, J = 9.2 Hz, 2H), 5.17-5.15 (m, 1H), 4.57-4.39 (m, 2H), 3.43 - 3.42 (m, 4H), 2.95-2.91 (m, 1H), 2.66-2.60 (m, 1H), 2.44 - 2.39 (m, 1H), 2.05 - 2.01 (m, 1H), 1.62-1.58 (m, 6H).; MS (ESI): m/z 432.2 [M+H]⁺. |
| **Compound 42** | white solid; ¹H NMR (400 MHz, DMSO-d₆) δ 11.02 (s, 1H), 7.91-7.87 (m, 2H), 7.80-7.77 (m, 1H), 7.35-7.33 (m, 2H), 7.08 (d, J = 8.4 Hz, 1H), 6.18 (s, 2H), 5.17-5.15 (m, 1H), 4.58-4.40 (m, 2H), 2.95 - 2.86 (m, 1H), 2.63-2.59 (m, 1H), 2.42-2.39 (m, 1H), 2.04 - 2.01 (m, 1H). ; MS (ESI): m/z 393.1 [M+H]⁺. |
| **Compound 43** | white solid; ¹H NMR (400 MHz, DMSO-d₆) δ 11.02 (s, 1H), 8.19 (d, J = 7.6 Hz, 1H), 8.09 (d, J = 7.6 Hz, 1H), 8.01 (s, 1H), 7.99 (d, J = 8.0 Hz, 1H) 7.88 (s, 2H), 7.67-7.58 (m, 4H), 5.17-5.15 (m, 1H), 4.55-4.43 (m, 2H), 2.92 - 286 (m, 1H), 2.63-2.59 (m, 1H), 2.40-2.37 (m, 1H), 2.03 - 2.01 (m, 1H).; MS (ESI): m/z 399.0 [M+H]⁺. |
| **Compound 44** | white solid; ¹H NMR (400 MHz, DMSO-d₆) δ 10.9 (s, 1H), 8.37 (s, 1H), 8.14 - 8.10 (m, 2H), 8.04-8.03 (m, 2H), 7.94-7.91 (m, 3H), 7.73-7.69 (m, 1H), 7.65-7.61 (m, 1H), 5.20-5.16 (m, 1H), 4.60-4.43 (m, 2H), 2.93- 2.90 (m, 1H), 2.63-2.59 (m, 1H), 2.45-2.41 (m, 1H), 2.07 - 2.03 (m, 1H). ;MS (ESI): m/z 399.1 [M+H]⁺. |
| **Compound 45** | white solid; ¹H NMR (400 MHz, DMSO-d₆) δ 11.04 (s, 1H), 8.00 (s, 1H), 7.95-7.86 (m, 4H), 7.58 (d, J = 8.0 Hz, 2H), 5.18 (dd, J = 12.8, 5.2 Hz, 1H), 4.50 (dd, J = 49.0, 17.6 Hz, 2H), 2.98-2.89 (m, 1H), 2.64-2.59 (m, 1H), 2.51-2.41 (m, 1H), 2.07-2.01 (m, 1H). ;MS (ESI): m/z 433.0 [M+H]⁺. |
| **Compound 46** | yellow solid; ¹H NMR (400 MHz, DMSO-d₆) δ 11.02 (s, 1H), 7.86-7.83 (m, 2H), 7.73-7.67 (m, 3H), 6.78 (d, J = 8.8 Hz, 2H), 5.20-5.15 (m, 1H), 4.57-4.39 (m, 2H), 3.05 (s, 6H), 2.97-2.89 (m, 1H), 2.63-2.59 (m, 1H), 2.43-2.38 (m, 1H), 2.06-2.02 (m, 1H). ; MS (ESI): m/z 392.2 [M+H]⁺. |
| **Compound 47** | white solid; ¹H NMR (400 MHz, DMSO-d₆) δ 11.03 (s, 1H), 7.99 (s, 1H), 7.90-7.87 (m, 2H), 7.83 (d, J = 8.4 Hz, 2H), 7.58 (d, J = 8.4 Hz, 2H), 5.18-5.15 (m, 1H), 4.58-4.40 (m, 2H), 3.02 (s, 3H), 2.96-2.87 (m, 4H), 2.64-2.59 (m, 1H), 2.42-2.38 (m, 1H), 2.04-2.01 (m, 1H). ;MS (ESI): m/z 420.1 [M+H]⁺. |
| **Compound 48** | white solid; ¹H NMR (400 MHz, DMSO-d₆) δ 11.03 (s, 1H), 7.97 (s, 1H), 7.91 (d, J = 8.0 Hz, 1H), 7.86 (d, J = 7.6 Hz, 1H), 7.83-7.80 (m, 2H), 7.68-7.65 (m, 2H), 5.18 (dd, J = 13.6, 5.2 Hz, 1H), 4.50 (dd, J = 48.6, 17.6 Hz, 2H), 2.98-2.89 (m, 1H), 2.64-2.59 (m, 1H), 2.45-2.38 (m, 1H), 2.08-2.01 (m, 1H). ;MS (ESI): m/z 383.0, 385.0 [M+H]⁺. |
| **Compound 49** | white solid; ¹H NMR (400 MHz, DMSO-d₆) δ 11.02 (s, 1H), 7.96 (s, 1H), 7.91-7.89 (m, 1H), 7.86-7.84 (m, 1H), 7.81-7.79 (m, 2H), 7.74-7.71 (m, 2H), 5.17 (dd, J = 13.2, 5.2 Hz, 1H), 4.49 (dd, J = 48.0, 17.6 Hz, 2H), 2.97-2.88 (m, 1H), 2.64-2.63 (m, 1H), 2.60-2.49 (m, 1H), 2.45-2.37 (m, 1H).;MS (ESI): m/z 427.0 429.0 [M+H]⁺. |
| **Compound 50** | white solid; ¹H NMR (400 MHz, DMSO-d₆) δ 11.01 (s, 1H), 8.01 (s, 1H), 7.99-7.88 (m, 6H), 5.17 (dd, J = 13.2, 5.2 Hz, 1H), 4.58-4.54 (m, 1H), 4.46-4.42 (m, 1H), 2.97-2.88 (m, 1H), 2.63-2.59 (m, 1H), 2.45-2.37 (m, 1H), 2.07-2.02 (m, 1H).;MS (ESI): m/z 417.0 [M+H]⁺. |

**[Table 14]**

| **Compounds** | **Characterization Data** |
|---|---|
| **Compound 51** | white solid; ¹H NMR (400 MHz, DMSO-d₆) δ 11.03 (s, 1H), 7.92-7.88 (m, 2H), 7.82-7.79 (m, 3H), 7.14-7.10 (m, 2H), 5.18 (dd, J = 13.2, 5.2 Hz, 1H), 4.50 (dd, J = 50.0, 17.6 Hz, 2H), 3.88 (s, 3H), 2.98-2.89 (m, 1H), 2.65-2.60 (m, 1H), 2.45-2.39 (m, 1H), 2.08-2.02 (m, 1H).; MS (ESI): m/z 379.0 [M+H]⁺. |
| **Compound 52** | white solid; ¹H NMR (400 MHz, DMSO-d₆) δ 11.03 (s, 1H), 10.54 (s, 1H), 7.89-7.87 (m, 2H), 7.77 (dd, J = 7.6, 1.6 Hz, 1H), 7.73-7.69 (m, 2H), 6.93-6.90 (m, 2H), 5.17 (dd, J = 13.2, 5.2 Hz, 1H), 4.49 (dd, J = 50.2, 17.6 Hz, 2H), 2.98-2.89 (m, 1H), 2.64-2.60 (m, 1H), 2.45-2.37 (m, 1H), 2.08-2.01 (m, 1H).; MS (ESI): m/z 365.1 [M+H]⁺. |
| **Compound 53** | white solid; ¹H NMR (400 MHz, DMSO-d₆) δ 11.03 (s, 1H), 7.92-7.88 (m, 2H), 7.81-7.79 (m, 3H), 7.50-7.48 (m, 2H), 7.44-7.41 (m, 2H), 7.38-7.34 (m, 1H), 7.21-7.18 (m, 2H), 5.25 (s, 2H), 5.17 (dd, J = 13.2, 5.2 Hz, 1H), 4.50 (dd, J = 50.6, 17.6 Hz, 2H), 2.98-2.89 (m, 1H), 2.64-2.58 (m, 1H), 2.45-2.37 (m, 1H), 2.08-2.01 (m, 1H).; MS (ESI): m/z 455.2 [M+H]⁺. |
| **Compound 54** | white solid; ¹H NMR (400 MHz, DMSO-d₆) δ 11.02 (s, 1H), 7.98-7.76 (m, 3H), 7.70 (d, J = 8.0 Hz, 2H), 7.40 (d, J = 8.0 Hz, 2H), 5.17 (dd, J = 13.3, 5.1 Hz, 1H), 4.50 (dd, J = 49.9, 17.7 Hz, 2H), 3.00-2.86 (m, 1H), 2.68-2.60 (m, 1H), 2.45-2.31 (m, 4H), 2.11-1.99 (m, 1H). ;MS (ESI): m/z 363.2 [M+H]⁺. |
| **Compound 55** | white solid; ¹H NMR (400 MHz, DMSO-d₆) δ 11.03 (s, 1H), 7.94 (s, 1H), 7.89 (d, J = 7.8 Hz, 1H), 7.82 (d, J = 7.8 Hz, 1H), 7.73 (d, J = 8.2 Hz, 2H), 7.44 (d, J = 8.2 Hz, 2H), 5.17 (dd, J = 13.3, 5.1 Hz, 1H), 4.56 (d, J = 17.7 Hz, 1H), 4.43 (d, J = 17.7 Hz, 1H), 3.00-2.84 (m, 1H), 2.66-2.60 (m, 1H), 2.44-2.36 (m, 1H), 2.08-1.99 (m, 1H), 1.82 (d, J = 7.9 Hz, 4H), 1.72 (d, J = 13.1 Hz, 1H), 1.50-1.35 (m, 4H), 1.29-1.22 (m, 2H).;MS (ESI): m/z 431.3 [M+H]⁺. |
| **Compound 56** | white solid; ¹H NMR (400 MHz, DMSO-d₆) δ 11.03 (s, 1H), 8.13 (d, J = 8.2 Hz, 2H), 7.98 (s, 1H), 7.95-7.80 (m, 4H), 5.17 (dd, J = 13.3, 5.1 Hz, 1H), 4.56 (d, J = 17.8 Hz, 1H), 4.44 (d, J = 17.7 Hz, 1H), 4.38 (q, J = 7.1 Hz, 2H), 2.99-2.83 (m, 1H), 2.64-2.60 (m, 1H), 2.45-2.34 (m, 1H), 2.06-2.03 (m, 1H), 1.35 (t, J = 7.1 Hz, 3H); MS (ESI): m/z 421.2 [M+H]⁺. |
| **Compound 57** | white solid; ¹H NMR (400 MHz, DMSO-d₆) δ 11.03 (s, 1H), 8.40-8.38 (m, 2H), 8.03-8.00 (m, 3H), 7.94-7.90 (m, 2H), 5.19-5.15 (m, 1H), 4.58-4.20 (m, 2H), 2.95-2.88 (m, 1H), 2.64-2.59 (m, 1H), 2.49-2.39 (m, 1H), 2.07-2.02 (m, 1H).;MS (ESI): m/z 394.0 [M+H]⁺. |
| **Compound 58** | white solid; ¹H NMR (400 MHz, DMSO-d₆) δ 11.03 (s, 1H), 8.17 (dd, J = 1.7, 0.7 Hz, 1H), 8.12 (s, 1H), 8.00 (dd, J = 7.9, 1.3 Hz, 1H), 7.89 (d, J = 8.3 Hz, 1H), 7.48 (dd, J = 3.6, 0.6 Hz, 1H), 6.83 (dd, J = 3.6, 1.7 Hz, 1H), 5.17 (dd, J = 13.3, 5.1 Hz, 1H), 4.51 (dd, J = 48.3, 17.7 Hz, 2H), 2.99-2.87 (m, 1H), 2.65-2.59 (m, 1H), 2.48-2.41 (m, 1H), 2.07-1.91 (m, 1H). ;MS (ESI): m/z 339.0 [M+H]⁺. |
| **Compound 59** | white solid; ¹H NMR (400 MHz, DMSO-d₆) δ 11.04 (s, 1H), 8.09 (s, 1H), 7.98 (dd, J = 7.9, 1.3 Hz, 1H), 7.91 (d, J = 7.9 Hz, 1H), 7.72 (d, J = 8.0 Hz, 1H), 7.66 (dd, J = 8.6, 0.7 Hz, 1H), 7.43 (ddd, J = 8.4, 7.0, 1.1 Hz, 1H), 7.20 - 7.13 (m, 1H), 7.09 (d, J = 0.7 Hz, 1H), 5.19 (dd, J = 13.3, 5.1 Hz, 1H), 4.59 (d, J = 17.7 Hz, 1H), 4.46 (d, J = 17.6 Hz, 1H), 4.09 (s, 3H), 3.00 - 2.86 (m, 1H), 2.64-2.61 (m, 1H), 2.46-2.40 (m, 1H), 2.06 (dd, J = 9.5, 4.2 Hz, 1H).;MS (ESI): m/z 402.1 [M+H]⁺. |
| **Compound 60** | white solid; ¹H NMR (400 MHz, DMSO-d₆) δ 11.05 (s, 1H), 8.63 (s, 1H), 8.57 (d, J = 7.9 Hz, 1H), 8.34-8.31 (m, 2H), 7.99 (d, J = 8.0 Hz, 1H), 7.72-7.67 (m, 2H), 5.20 (dd, J = 13.3, 5.1 Hz, 1H), 4.64 (d, J = 17.7 Hz, 1H), 4.50 (d, J = 17.7 Hz, 1H), 3.04 - 2.85 (m, 1H), 2.67 - 2.60 (m, 1H), 2.46-2.41 (m, 1H), 2.08-2.05 (m, 1H).; MS (ESI): m/z 406.0 [M+H]⁺. |

**[Table 15]**

| **Compounds** | **Characterization Data** |
|---|---|
| **Compound 61** | white solid; ¹H NMR (400 MHz, DMSO-d₆) δ 11.03 (s, 1H), 7.95 (s, 1H), 7.90 (d, J = 8.0 Hz 1H), 7.84-7.82 (m, 1H), 7.60-7.45 (m, 4H), 5.17 (dd, J = 13.3, 5.1 Hz, 1H), 4.56 (d, J = 17.7 Hz, 1H), 4.44 (d, J = 17.7 Hz, 1H), 3.02-2.85 (m, 1H), 2.67-2.64 (m, 1H), 2.45-2.34 (m, 4H), 2.12 - 1.97 (m, 1H). ; MS (ESI): m/z 363.1 [M+H]⁺. |
| **Compound 62** | white solid; ¹H NMR (400 MHz, DMSO-d₆) δ 11.02 (s, 1H), 7.93 (s, 1H), 7.89 (d, J = 7.9 Hz, 1H), 7.82 (d, J = 8.0 Hz, 1H), 7.53-7.46 (m, 1H), 7.40 (d, J = 7.6 Hz, 1H), 7.37-7.29 (m, 2H), 5.15 (dd, J = 13.3, 5.1 Hz, 1H), 4.54 (d, J = 17.8 Hz, 1H), 4.42 (d, J = 17.8 Hz, 1H), 3.00-2.81 (m, 1H), 2.63-2.59 (m, 1H), 2.41-2.35 (m, 1H), 2.26 (s, 3H), 2.08-1.97 (m, 1H).; MS (ESI): m/z 363.3 [M+H]⁺. |
| **Compound 63** | white solid; ¹H NMR (400 MHz, DMSO-d₆) δ 11.03 (s, 1H), 7.96 (s, 1H), 7.92-7.80 (m, 2H), 7.79-7.70 (m, 2H), 7.66-7.55 (m, 2H), 5.17 (dd, J = 13.3, 5.0 Hz, 1H), 4.56 (d, J = 17.7 Hz, 1H), 4.43 (d, J = 17.8 Hz, 1H), 2.99-2.84 (m, 1H), 2.67-2.59 (m, 1H), 2.44-2.35 (m, 1H), 2.11-1.98 (m, 1H), 1.34 (s, 9H).; MS (ESI): m/z 405.1 [M+H]⁺. |
| **Compound 64** | white solid; ¹H NMR (400 MHz, DMSO-d₆) δ 11.01 (s, 1H), 7.84-7.80 (m, 2H), 7.70-7.67 (m, 1H), 7.56-7.53 (m, 2H), 6.62-6.59 (m, 2H), 6.25 (s, 2H), 5.18-5.14 (m, 1H), 4.56-4.39 (m, 2H), 2.92 - 2.89 (m, 1H), 2.67-2.54 (m, 1H), 2.49-2.32 (m, 1H), 2.05 - 2.02 (m, 1H). ;MS (ESI): m/z 364.1 [M+H]⁺. |
| **Compound 65** | white solid; ¹H NMR (400 MHz, DMSO-d₆) δ 11.03 (s, 1H), 8.94 (d, J = 2.0 Hz, 1H), 8.87 (dd, J = 5.0, 1.5 Hz, 1H), 8.17 (dd, J = 8.0, 2.0 Hz, 1H), 8.02 (s, 1H), 7.94-7.88 (m, 2H), 7.63 (dd, J = 8.0, 5.0 Hz, 1H), 5.17 (dd, J = 13.5, 5.0 Hz, 1H), 4.57 (d, J = 18.0 Hz, 1H), 4.45 (d, J = 18.0 Hz, 1H), 2.97-2.88 (m, 1H), 2.65-2.58 (m, 1H), 2.45-2.36 (m, 1H), 2.08-2.01 (m, 1H);MS (ESI): m/z 350.0 [M+H]⁺. |
| **Compound 66** | white solid; ¹H NMR (400 MHz, DMSO-d₆) δ 11.04 (s, 1H), 8.84 (dd, J = 4.4, 1.6 Hz, 2H), 8.03 (s, 1H), 7.92-7.91 (m, 2H), 7.68 (dd, J = 4.4, 1.6 Hz, 2H), 5.17 (dd, J = 13.2, 5.2 Hz, 1H), 4.50 (dd, J = 50, 18 Hz, 2H), 2.99 - 2.85 (m, 1H), 2.67-2.59 (m, 1H), 2.43-2.33 (m, 1H), 2.09 - 1.99 (m, 1H). ; MS (ESI): m/z 350.1 [M+H]⁺. |
| **Compound 67** | white solid; ¹H NMR (400 MHz, DMSO-d₆) δ 11.04 (s, 1H), 9.46 (s, 1H), 9.15 (s, 2H), 8.09 (s, 1H), 7.99 - 7.92 (m, 2H), 5.18 (dd, J = 13.2, 5.2 Hz, 1H), 4.58 (d, J = 18.0 Hz, 1H), 4.46 (d, J = 18.0 Hz, 1H), 2.97 - 2.87 (m, 1H), 2.65 - 2.58 (m, 1H), 2.46 - 2.37 (m, 1H), 2.07 - 2.00 (m, 1H).;MS (ESI): m/z 351.0 [M+H]⁺. |
| **Compound 68** | white solid; ¹H NMR (400 MHz, DMSO-d₆) δ 11.04 (s, 1H), 9.24 (d, J = 1.5 Hz, 1H), 8.96 (d, J = 2.5 Hz, 1H), 8.83 (dd, J = 2.5, 1.5 Hz, 1H), 8.20 (s, 1H), 8.10 (d, J = 8.0 Hz, 1H), 7.91 (d, J = 7.9 Hz, 1H), 5.17 (dd, J = 13.3, 5.1 Hz, 1H), 4.51 (dd, J = 54.5, 17.7 Hz, 2H), 3.00 - 2.83 (m, 1H), 2.72 - 2.58 (m, 1H), 2.46 - 2.37 (m, 1H), 2.10 - 1.96 (m, 1H).;MS (ESI): m/z 351.1 [M+H]⁺. |
| **Compound 69** | white solid; ¹H NMR (400 MHz, DMSO-d₆) δ 11.03 (s, 1H), 8.70 (d, J = 4.5 Hz, 1H), 8.00 (s, 1H), 7.93-7.89 (m, 2H), 7.53 (s, 1H), 7.46 (d, J = 5.0 Hz, 1H), 5.19 (dd, J = 13.5, 5.5 Hz, 1H), 4.58 (d, J = 18.0 Hz, 1H), 4.46 (d, J = 18.0 Hz, 1H), 2.96-2.89 (m, 1H), 2.64-2.58 (m, 4H), 2.47-2.36 (m, 1H), 2.06-2.02 (m, 1H). ; MS (ESI): m/z 364.0 [M+H]⁺. |
| **Compound 70** | white solid; ¹H NMR (400 MHz, DMSO-d₆) δ 11.03 (s, 1H), 8.67 (d, J = 8.5 Hz, 1H), 8.29 (s, 1H), 8.20-8.11 (m, 4H), 7.92-7.87 (m, 2H), 7.79 (t, J = 7.5 Hz, 1H), 5.18 (dd, J = 13.3, 5.1 Hz, 1H), 4.52 (dd, J = 64.5, 17.7 Hz, 2H), 3.00-2.88 (m, 1H), 2.64-2.61 (m, 1H), 2.45-2.34 (m, 1H), 2.09 - 2.01 (m, 1H). ;MS (ESI): m/z 400.0 [M+H]⁺. |

**[Table 16]**

| **Compounds** | **Characterization Data** |
|---|---|
| **Compound 71** | white solid; ¹H NMR (400 MHz, DMSO-d₆) δ 11.02 (s, 1H), 8.64 (d, J = 5.2 Hz, 1H), 8.17-8.13 (m, 3H), 8.09 (s, 1H), 7.98 (dd, J = 8.0, 1.2 Hz, 1H), 7.92 - 7.88 (m, 2H), 7.77 - 7.73 (m, 1H), 5.16 (dd, J = 13.2, 4.8 Hz, 1H), 4.47 (dd, J = 53.2, 17.6 Hz, 2H), 2.96 - 2.87 (m, 1H), 2.68 - 2.58 (m, 1H), 2.44 - 2.32 (m, 1H), 2.08 - 1.99 (m, 1H).; MS (ESI): m/z 400.0 [M+H]⁺. |
| **Compound 72** | ¹H NMR (400 MHz, DMSO-d₆) δ 11.04 (s, 1H), 8.56 (s, 2H), 8.48 (dd, J = 8.0, 0.8 Hz, 1H), 7.94 (d, J = 8.0 Hz, 1H), 7.70 (s, 1H), 5.18 (dd, J = 13.2, 5.2 Hz, 1H), 4.61 (d, J = 17.6 Hz, 1H), 4.47 (d, J = 17.6 Hz, 1H), 2.98 - 2.89 (m, 1H), 2.68 - 2.60 (m, 1H), 2.45 - 2.33 (m, 1H), 2.08 - 2.03 (m, 1H). |
| **Compound 73** | ¹H NMR (400 MHz, DMSO-d₆) δ 12.16 (s, 1H), 11.02 (s, 1H), 8.02 (s, 1H), 7.96-7.79 (m, 2H), 7.28-7.26 (m, 1H), 6.87-6.85 (m, 1H), 6.48-6.19 (m, 1H), 5.17 (dd, J = 13.3, 5.1 Hz, 1H), 4.56 (d, J = 17.6 Hz, 1H), 4.44 (d, J = 17.7 Hz, 1H), 3.01-2.84 (m, 1H), 2.69-2.62 (m, 1H), 2.43-2.32 (m, 1H), 2.10-1.98 (m, 1H). |
| **Compound 74** | ¹H NMR (400 MHz, D MSO-d₆) δ 11.02 (s, 1H), 8.06 (d, J = 8.3 Hz, 2H), 7.99 (s, 1H), 7.94-7.87 (m, 4H), 5.17 (dd, J = 13.3, 5.1 Hz, 1H), 4.56 (d, J = 17.8 Hz, 1H), 4.44 (d, J = 17.9 Hz, 1H), 2.99-2.85 (m, 1H), 2.67-2.59 (m, 1H), 2.45-2.33 (m, 1H), 2.10-1.97 (m, 1H). |
| **Compound 75** | ¹H NMR (400 MHz, DMSO-d₆) δ 11.03 (s, 1H), 8.58 (s, 1H), 8.50 (d, J = 9.2 Hz, 1H), 8.35 (d, J = 3.2 Hz, 1H), 8.28 (d, J = 2.8 Hz, 1H), 7.94 (d, J = 8.4 Hz, 1H), 5.18 (dd, J = 13.2, 4.8 Hz, 1H), 4.54 (dd, J = 55.2, 18 Hz, 2H), 2.98-2.89 (m, 1H), 2.64-2.60 (m, 1H), 2.49-2.38 (m, 1H), 2.08-2.04 (m, 1H). |
| **Compound 76** | ¹H NMR (400 MHz, DMSO-d₆) δ 11.03 (s, 1H), 9.64 (s, 1H), 8.67 (s, 1H), 8.25 (d, J = 8.4 Hz, 1H), 8.01 (s, 1H), 7.99-7.89 (m, 3H), 5.17 (dd, J = 13.2, 5.2 Hz, 1H), 4.51 (dd, J = 48.0, 17.6 Hz, 2H), 2.97-2.88 (m, 1H), 2.64-2.59 (m, 1H), 2.45-2.34 (m, 1H), 2.07-2.03 (m, 1H). |
| **Compound 77** | ¹H NMR (400 MHz, DMSO-d₆) δ 11.03 (s, 1H), 7.94 (s, 1H), 7.90 (d, J = 7.8 Hz, 1H), 7.83 (d, J = 7.9 Hz, 1H), 7.76 (d, J = 8.2 Hz, 2H), 7.52 (d, J = 8.1 Hz, 2H), 5.41 (t, J = 5.7 Hz, 1H), 5.17 (dd, J = 13.4, 5.1 Hz, 1H), 4.62 (d, J = 5.7 Hz, 2H), 4.56 (d, J = 17.7 Hz, 1H), 4.44 (d, J = 17.7 Hz, 1H), 3.00-2.87 (m, 1H), 2.64-2.60 (m, 1H), 2.44-2.35 (m, 1H), 2.12-1.96 (m, 1H). |

### Example 2: Evaluation of Binding Affinity of Synthesized Compounds for Cereblon

To confirm the binding affinity of the compounds synthesized in the Preparation Examples for a Cereblon (CRBN) protein, the following experiment was performed using E3scan^{™} from Eurofins.

Specifically, CRBN was produced using a HEK-293 cell line and labeled with DNA for qPCR detection. Streptavidin-coated magnetic beads were treated with a biotinylated small-molecule ligand at room temperature for 30 minutes to generate an affinity resin for E3scan analysis. The liganded beads were blocked with excess biotin and washed with a blocking buffer (SeaBlock (Pierce), 1 % BSA, 0.05 % Tween 20, 1 mM DTT) to remove unbound ligands and prevent non-specific binding. A binding reaction was performed by mixing CRBN, the liganded affinity beads, and a test compound in a 1× binding buffer (20 % SeaBlock, 0.17× PBS, 0.05 % Tween 20, 6 mM DTT). The test compound was prepared as a 100× stock in 100 % DMSO and diluted directly into the assay solution. All reactions were performed in a polypropylene 384-well plate with a final reaction volume of 0.02 mL. The assay plate was incubated with shaking at room temperature for 1 hour, and then the affinity beads were washed with a wash buffer (1× PBS, 0.05 % Tween 20). Then, the beads were resuspended in an elution buffer (1× PBS, 0.05 % Tween 20, 0.5 µM non-biotinylated affinity ligand) and incubated with shaking at room temperature for 30 minutes. The CRBN concentration in the eluate was measured by qPCR. The test compound and a control (lenalidomide) were analyzed at a concentration of 5 µM, and the binding affinity was calculated as '% Ctrl', which is a relative binding affinity, using the following equation. A lower '% Ctrl' indicates a stronger binding affinity. Meanwhile, DMSO was used as a negative control (100 % Ctrl), and a control compound (lenalidomide) was used as a positive control (0 % Ctrl). $\left(\frac{test compound signal - positive control signal}{negative control signal - positive control signal}\right) \times 100$

The results of the binding affinity analysis of the compounds synthesized in the Preparation Examples for CRBN are shown in the table below.

**[Table 17]**

| **Compounds** | **CRBN binding affinity (% Ctrl @ 5µM)** |
|---|---|
| **Lenalidomide** | A |
| **Compound 1** | C |
| **Compound 3** | A |
| **Compound 4** | B |
| **Compound 5** | C |
| **Compound 6** | A |
| **Compound 7** | A |
| **Compound 10** | C |
| **Compound 11** | A |
| **Compound 12** | A |
| **Compound 15** | A |
| **Compound 16** | A |
| **Compound 17** | A |
| **Compound 21** | A |
| **Compound 22** | A |
| **Compound 24** | D |
| **Compound 25** | D |
| **Compound 26** | A |
| **Compound 27** | A |
| **Compound 28** | C |
| **Compound 30** | A |
| **Compound 31** | A |
| **Compound 34** | A |
| **Compound 35** | A |
| **Compound 36** | A |
| **Compound 37** | C |
| Binding affinity range: A: 0 ≤ '% Ctrl' < 0.1, B: 0.1 ≤ '% Ctrl' < 1, C: 1 ≤ % Ctrl' < 10, D: 10 ≤ '% Ctrl' < 35, E: '% Ctrl' ≥ 35 | |

As confirmed in Table 17 above, it can be seen that the compounds according to the present disclosure exhibit a strong binding affinity for CRBN.

### Example 3: Evaluation of Degradation Activity of Synthesized Compounds against GSPT1

To confirm the degradation activity of the compounds synthesized in the Preparation Examples against a GSPT1 protein, a Jurkat cell line was treated with the compounds, and the amount of the GSPT1 protein present in the cells was measured. The amount of the GSPT1 protein was measured using a Western blotting detection method.

### 3-1: Cell Line Culture

As a culture medium for the Jurkat cell line, an RPMI medium containing 10 % heat-inactivated FBS and 1 % penicillin/streptomycin (P/S) antibiotics was used. The culture medium was stored refrigerated and warmed in a 37 °C water bath before use. The Jurkat cell line (manufacturer: KCLB, Cat. No.: 40152) was used for the test after a stabilization period of at least 2 weeks after thawing, and cell culture was performed based on information provided by the cell line manufacturer regarding the thawing and subculture conditions.

### 3-2: Western Blotting Analysis

The Jurkat cell line was seeded in a 6-well plate at a density of 3 × 10⁶ cells/well, treated with a test compound at different concentrations, and incubated in a 37 °C CO₂ incubator for 24 hours. The cell line treated with the drug for 24 hours was washed with DPBS and then lysed by adding a RIPA buffer. Then, the mixture was centrifuged at 12,000 rpm at 4 °C for 10 minutes, and the supernatant was collected to obtain proteins. The obtained proteins were quantified using a BCA Protein Assay Kit (Thermo, 23225), and a 4× loading buffer (GenDEPOT, L1100-001) was added to prepare samples containing the proteins at the same concentration. The prepared samples were electrophoresed using 4-12 % Bis-Tris Midi Protein Gels (Invitrogen, WG1403BOX) and a SureLock^{™} Tandem Midi Gel Tank (Invitrogen, STM1001) and then blotted onto a PVDF membrane (Thermo, IB24001) using a transfer device (Thermo, IB21001). Then, the membrane was blocked using a TBS-T (T&I, BTT-9110) buffer containing 5 % BSA (GenDEPOT, A0100-010), and the membrane was washed with 1× TBS-T. After washing, a primary antibody was prepared using a TBS-T buffer containing 5 % BSA (GSTP1 1:1000, β-actin 1:3000) and applied, and the membrane was incubated at 4 °C overnight. Thereafter, the membrane was washed with 1× TBS-T, and a secondary antibody was prepared using a TBS-T buffer containing 5 % skim milk (GSTP1 1:3000, β-actin 1:5000) and applied, and the membrane was incubated at room temperature for 1 hour. After washing the membrane with 1× TBS-T, an Amersham ECL Western blotting detection reagent (Cytiva, RPN2235) was applied, and protein bands were identified using an Amersham Imager 680 (Cytiva, #29270769) instrument. For the identified protein bands, a change in protein expression was analyzed using an ImageQuant TL program. The results for a target protein obtained through the Western blot test were normalized using the results for β-actin, which is a loading control. Then, the degradation activity of the test compound against the target protein was analyzed by setting the normalized results for the target protein in a vehicle-only treated group (DMSO) to 100 %.

The results of analyzing the degradation activities of the compounds synthesized in the Preparation Examples against the GSPT1 protein are shown in the table below. In addition, the Western blotting results for some of the compounds are shown in FIG. 1.

**[Table 18]**

| **Compounds** | **GSPT1 Degradation (%)** | | |
|---|---|---|---|
| | **0.1 µM** | **1 µM** | **10 µM** |
| **Compound 1** | D | C | B |
| **Compound 3** | A | A | A |
| **Compound 4** | B | A | A |
| **Compound 6** | D | B | B |
| **Compound 7** | C | B | B |
| **Compound 8** | A | A | A |
| **Compound 9** | A | A | A |
| **Compound 10** | D | C | A |
| **Compound 14** | D | C | C |
| **Compound 15** | B | A | A |
| **Compound 16** | C | B | A |
| **Compound 17** | C | B | B |
| **Compound 18** | D | B | A |
| **Compound 19** | A | A | A |
| **Compound 20** | A | A | A |
| **Compound 21** | B | A | A |
| **Compound 22** | A | A | A |
| **Compound 26** | C | C | B |
| **Compound 27** | A | A | A |
| **Compound 28** | B | A | A |
| **Compound 30** | D | B | B |
| **Compound 31** | B | A | A |
| **Compound 32** | B | A | A |
| **Compound 33** | A | A | A |
| **Compound 34** | B | A | A |
| **Compound 35** | A | A | A |
| **Compound 36** | A | A | A |
| **Compound 37** | B | A | A |
| **Compound 39** | D | D | C |
| **Compound 40** | D | B | B |
| **Compound 41** | B | B | A |
| **Compound 42** | A | A | A |
| **Compound 43** | D | D | C |
| **Compound 44** | A | A | A |
| **Compound 45** | C | A | A |
| **Compound 46** | A | A | A |
| **Compound 48** | B | B | B |
| **Compound 49** | B | B | B |
| **Compound 50** | B | B | A |
| **Compound 51** | B | B | B |
| **Compound 52** | B | A | A |
| **Compound 53** | B | A | A |
| **Compound 54** | B | A | A |
| **Compound 55** | B | A | A |
| **Compound 57** | E | D | B |
| **Compound 59** | B | A | A |
| **Compound 60** | D | D | C |
| **Compound 61** | A | A | A |
| **Compound 63** | A | A | A |
| **Compound 64** | C | A | A |
| **Compound 69** | E | D | B |
| **Compound 70** | E | D | C |
| **Compound 73** | A | A | A |
| **Compound 74** | E | E | C |
| **Compound 76** | A | A | A |
| Degradation activity range: A: 90 < % ≤ 100, B: 70 < % ≤ 90, C: 50 < % ≤ 70, D: 30 < % ≤ 50, E: % ≤ 30 | | | |

As confirmed in Table 18 above, it can be seen that the compounds according to the present disclosure exhibit excellent activity in degrading GSPT1.

### Example 4: Evaluation of Cytotoxicity of Synthesized Compounds against Cancer Cells

To evaluate the cytotoxicity of the compounds synthesized in the Preparation Examples against cancer cells, a cell viability assay following treatment with the synthesized compounds was performed using a T-cell leukemia cell line Jurkat (KCLB, Cat# 40152), a human acute monocytic leukemia cell line MV-4-11 (ATCC, Cat# CRL-9591), and a human multiple myeloma cell line MM.1S (ATCC, Cat# CRL-2974).

### 4-1: Cell Culture

As a culture medium for the cell lines, an RPMI medium containing 10 % heat-inactivated FBS and 1 % penicillin/streptomycin (P/S) antibiotics was used. The culture medium was stored refrigerated and warmed in a 37 °C water bath before use. Each cell line was used for the test after a stabilization period of at least 2 weeks after thawing, and cell culture was performed based on information provided by the cell line manufacturer regarding the thawing and subculture conditions.

### 4-2: Evaluation of Cell Viability

The Jurkat, MV-4-11, or MM.1S cell line was seeded in a 96-well plate at a density of 1 × 10⁴ cells/well and incubated in a 37 °C CO₂ incubator overnight. After incubation, a prepared test compound was applied to each cell line, and the cells were incubated in a 37 °C CO₂ incubator for 72 hours. After 72 hours of incubation, a Cell Counting Kit-8 (Dojindo, Cat# CK04-20) reagent was added, and the cells were incubated in a 37 °C CO₂ incubator. Then, the absorbance was measured at 450 nm using a SpectraMax M3 Microplate Reader (N=2, duplicate measurement). The evaluation concentrations for evaluating the cytotoxicity of the compounds were 10 points: 100 µM, 20 µM, 4 µM, 0.8 µM, 0.16 µM, 0.032 µM, 0.0064 µM, 0.00128 µM, 0.000256 µM, and 0 µM, and the test was performed at 2 wells/concentration point (n=2). A GI₅₀ was derived using a GraphPad Prism program.

The cytotoxicity of the compounds synthesized in the Preparation Examples against each cell line is shown in the table below (activity range: A: GI₅₀ ≤ 0.1 µM, B: 0.1 < GI₅₀ ≤ 1 µM, C: 1 < GI₅₀ ≤ 10 µM, D: GI₅₀ > 10 µM).

**[Table 19]**

| **Compounds** | **Jurkat Cell Line** - **Cell viability (GI50)** |
|---|---|
| **Compound 1** | B |
| **Compound 3** | A |
| **Compound 4** | B |
| **Compound 8** | A |
| **Compound 9** | A |
| **Compound 10** | C |
| **Compound 15** | B |
| **Compound 16** | C |
| **Compound 17** | B |
| **Compound 18** | B |
| **Compound 19** | A |
| **Compound 20** | A |
| **Compound 21** | B |
| **Compound 22** | A |
| **Compound 26** | C |
| **Compound 27** | A |
| **Compound 28** | B |
| **Compound 30** | B |
| **Compound 31** | B |
| **Compound 32** | B |
| **Compound 33** | A |
| **Compound 34** | A |
| **Compound 35** | A |
| **Compound 36** | A |
| **Compound 37** | B |
| **Compound 39** | C |
| **Compound 40** | B |
| **Compound 41** | B |
| **Compound 42** | A |
| **Compound 43** | C |
| **Compound 44** | A |
| **Compound 45** | B |
| **Compound 46** | A |
| **Compound 48** | B |
| **Compound 49** | A |
| **Compound 50** | B |
| **Compound 51** | B |
| **Compound 52** | B |
| **Compound 53** | B |
| **Compound 54** | B |
| **Compound 55** | A |
| **Compound 59** | B |
| **Compound 61** | A |
| **Compound 63** | A |
| **Compound 64** | B |
| **Compound 70** | C |
| **Compound 73** | A |
| **Compound 76** | A |

**[Table 20]**

| **Compounds** | **MV-4-11 Cell Line** - **Cell viability (GI50)** |
|---|---|
| **Compound 3** | A |
| **Compound 15** | A |
| **Compound 17** | A |
| **Compound 18** | A |
| **Compound 21** | D |
| **Compound 22** | A |

**[Table 21]**

| **Compounds** | **MM.1S Cell Line** - **Cell viability (GI50)** |
|---|---|
| **Compound 3** | A |
| **Compound 4** | A |
| **Compound 9** | A |
| **Compound 15** | A |
| **Compound 17** | A |
| **Compound 18** | A |
| **Compound 19** | A |
| **Compound 20** | A |
| **Compound 21** | C |
| **Compound 22** | A |
| **Compound 27** | A |
| **Compound 31** | A |
| **Compound 33** | A |
| **Compound 34** | A |
| **Compound 35** | A |
| **Compound 37** | A |
| **Compound 42** | A |
| **Compound 44** | D |
| **Compound 46** | A |
| **Compound 49** | C |
| **Compound 52** | A |
| **Compound 55** | A |
| **Compound 59** | A |
| **Compound 63** | A |
| **Compound 70** | D |

As confirmed in Tables 19 to 21 above, it can be seen that the compounds according to the present disclosure exhibit cytotoxicity against various types of cancer cells.

The above description of the present disclosure is provided for the purpose of illustration, and those of ordinary skill in the art to which the present disclosure pertains will understand that the present disclosure may be easily modified into other specific forms without changing the technical spirit or essential features thereof. Therefore, it should be understood that the embodiments described above are illustrative in all respects and not restrictive.

## Claims

1. A compound represented by Formula 78 below, an isomer thereof, a pharmaceutically acceptable salt thereof, or a solvate thereof: 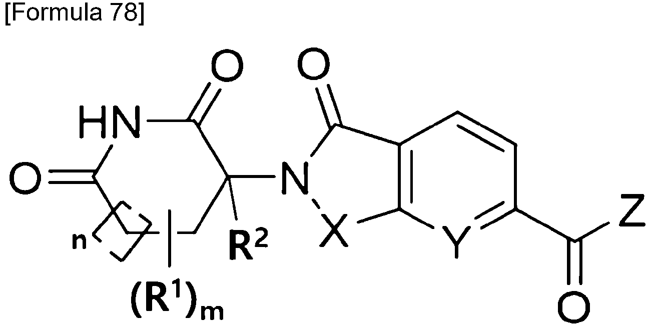
wherein, in Formula 78,
one or more R¹ are each independently selected from the group consisting of hydrogen, halogen, a substituted or unsubstituted C₁-C₆ alkyl group, a substituted or unsubstituted C₁-C₆ haloalkyl group, a substituted or unsubstituted C₁-C₆ alkoxy group, a substituted or unsubstituted C₁-C₆ alkylamino group, a substituted or unsubstituted C₁-C₆ alkenyl group, a substituted or unsubstituted C₁-C₆ alkynyl group, -CH₂R³, -OR³, -OCH₂R³, -NR³R⁴, -NH(C=O)R³, -(C=O)R³, -(C=O)OR³, -(C=O)NR³R⁴, -SO₂NHR³, - CN, -NO₂, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3-to 12-membered heteroaryl group,
R² is selected from the group consisting of hydrogen, deuterium, a substituted or unsubstituted C₁-C₆ alkyl group, and a substituted or unsubstituted C₁-C₆ haloalkyl group,
each R³ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
each R⁴ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
m is an integer in a range of 0 to 2,
n is an integer in a range of 0 to 2,
X is C=O or CH₂,
Y is CH or N, and
Z is selected from the group consisting of a substituted or unsubstituted C₁-C₁₀ alkyl group, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3-to 12-membered heteroaryl group.

2. The compound, isomer thereof, pharmaceutically acceptable salt thereof, or solvate thereof according to claim 1, wherein, in Formula 78,
X is CH₂, and
Y is CH.

3. The compound, isomer thereof, pharmaceutically acceptable salt thereof, or solvate thereof according to claim 1, wherein, in Formula 78,
m is 0,
n is 1, and
R² is hydrogen.

4. The compound, isomer thereof, pharmaceutically acceptable salt thereof, or solvate thereof according to claim 1, wherein, in Formula 78,
Z is selected from the group consisting of a substituted or unsubstituted C₁-C₁₀ alkyl group, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3-to 12-membered heteroaryl group, wherein
when Z is a substituted functional group, Z is substituted with one or more R^{a},
the one or more R^{a} are each independently selected from the group consisting of hydrogen, halogen, a substituted or unsubstituted C₁-C₆ alkyl group, a substituted or unsubstituted C₁-C₆ haloalkyl group, a substituted or unsubstituted C₁-C₆ alkoxy group, a substituted or unsubstituted C₁-C₆ alkenyl group, a substituted or unsubstituted C₁-C₆ alkynyl group, -CH₂R³, -OR³, -OCH₂R³, -NR³R⁴, -NH(C=O)R³, -NHSO₂R³, -(C=O)R³, -(C=O)OR³, -(C=O)NR³R⁴, -SO₂NHR³, -CN, -NO₂, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group, each R³ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group, and
each R⁴ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group.

5. The compound, isomer thereof, pharmaceutically acceptable salt thereof, or solvate thereof according to claim 1, wherein, in Formula 78,
Z comprises one or more selected from the group consisting of a substituted or unsubstituted C₁-C₁₀ alkyl group, a substituted or unsubstituted C₃-C₁₂ cycloalkyl group, a substituted or unsubstituted 3- to 12-membered heteroaryl group, and groups represented by Formulas 82-1 to 82-52: 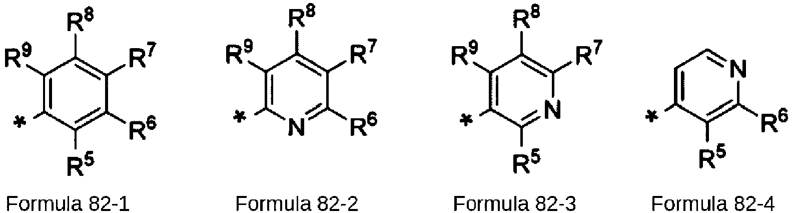 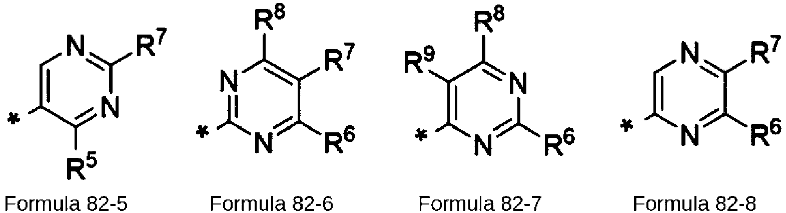 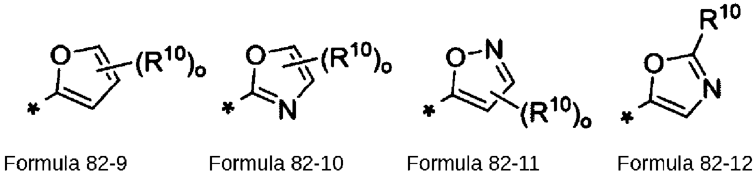 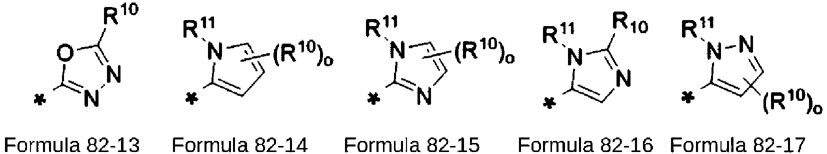 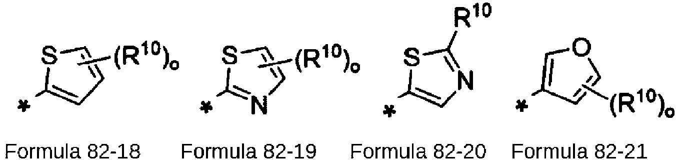 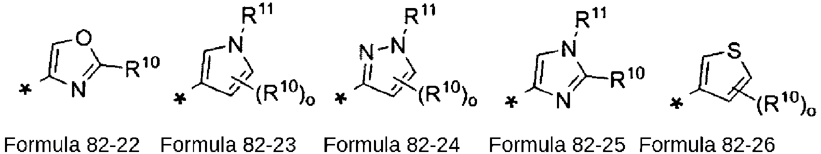 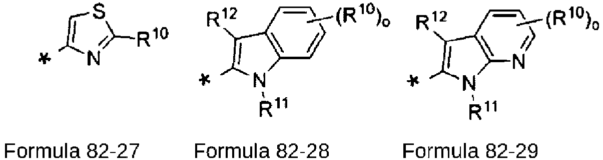 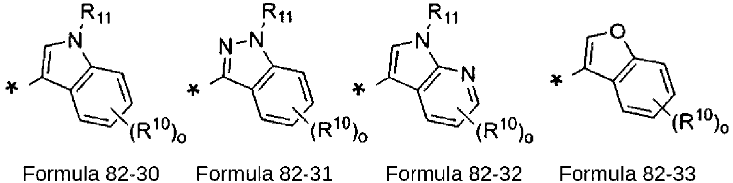 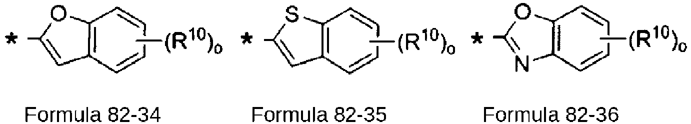 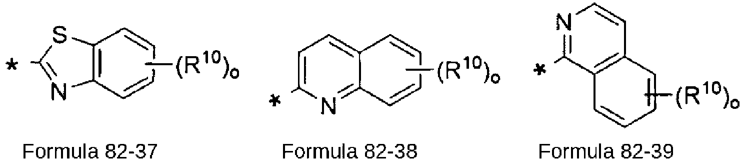 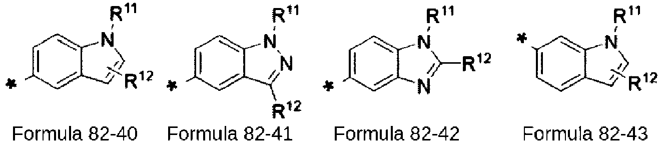 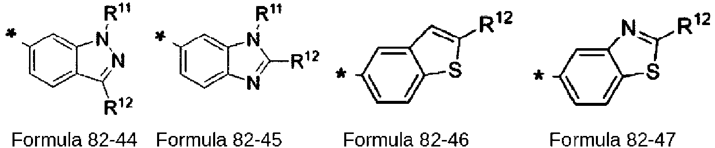 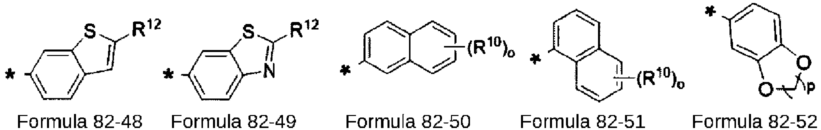
wherein, in the above Formulas,
R⁵, R⁶, R⁷, R⁸, and R⁹ are each independently selected from the group consisting of hydrogen, halogen, a substituted or unsubstituted C₁-C₆ alkyl group, a substituted or unsubstituted C₁-C₆ haloalkyl group, a substituted or unsubstituted C₁-C₆ alkoxy group, a substituted or unsubstituted C₁-C₆ alkylamino group, a substituted or unsubstituted C₁-C₆ alkenyl group, a substituted or unsubstituted C₁-C₆ alkynyl group, -CH₂R³, -OR³, -OCH₂R³, -NR³R⁴, -NH(C=O)R³, -NHSO₂R³, -(C=O)R³, -(C=O)OR³, -(C=O)NR³R⁴, - SO₂NHR³, -CN, -NO₂, a substituted or unsubstituted C₃-C₁₂ cycloalkyl group, a substituted or unsubstituted C₃-C₁₂ cycloalkenyl group, a substituted or unsubstituted 3- to 12-membered heterocycloalkyl group, a substituted or unsubstituted 3- to 12-membered heterocycloalkenyl group, a substituted or unsubstituted C₃-C₁₂ aryl group, and a substituted or unsubstituted 3- to 12-membered heteroaryl group,
R¹⁰, R¹¹, and R¹² are each independently selected from the group consisting of hydrogen, halogen, -OR³, -NR³R⁴, a substituted or unsubstituted C₁-C₆ alkyl group, a substituted or unsubstituted C₁-C₆ haloalkyl group, a substituted or unsubstituted phenyl group, and a substituted or unsubstituted benzyl group,
each R³ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
each R⁴ is independently selected from the group consisting of hydrogen, halogen, a hydroxy group, an amine group, a substituted or unsubstituted C₁-C₆ alkyl group, a substituted or unsubstituted C₁-C₃ haloalkyl group, a substituted or unsubstituted C₃-C₁₂ cycloalkyl group, a substituted or unsubstituted 3- to 12-membered heterocycloalkyl group, a substituted or unsubstituted C₃-C₁₂ aryl group, and a substituted or unsubstituted 3- to 12-membered heteroaryl group,
o is an integer in a range of 0 to 2,
p is an integer in a range of 1 to 3, and
* represents a point of attachment to an adjacent atom.

6. The compound, isomer thereof, pharmaceutically acceptable salt thereof, or solvate thereof according to claim 5, wherein in Formula 78,
Z comprises one or more selected from the group consisting of groups represented by the following formulas:

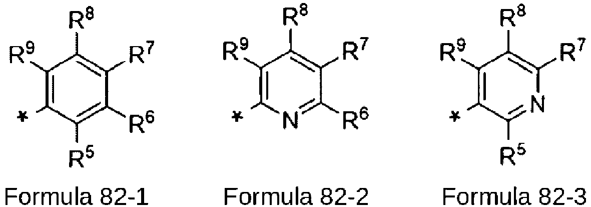

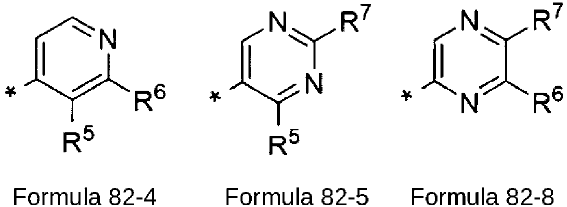

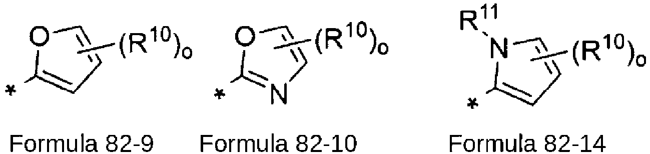

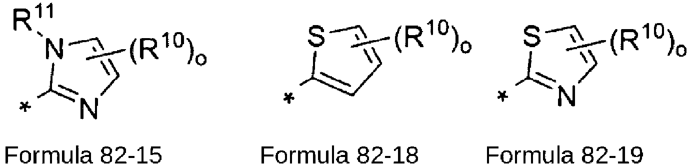

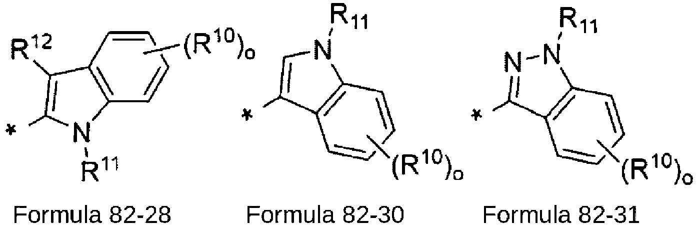

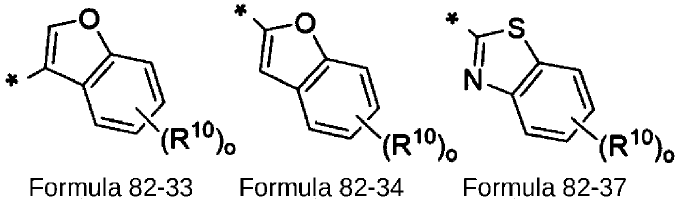

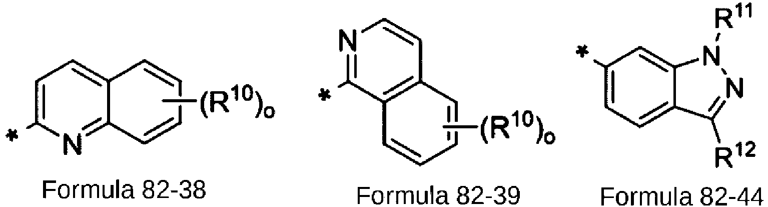

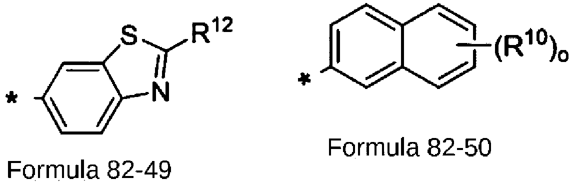

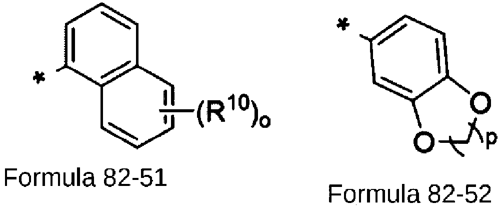

7. The compound, isomer thereof, pharmaceutically acceptable salt thereof, or solvate thereof according to claim 5, wherein in Formula 78,
Z comprises one or more selected from the group consisting of groups represented by the following formulas: 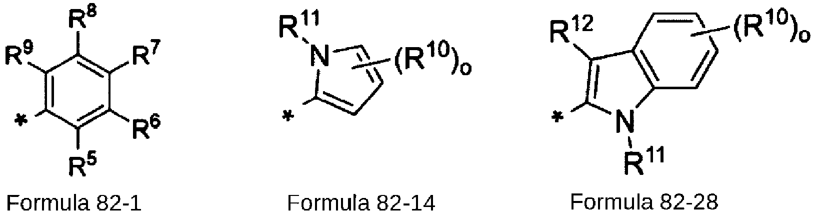 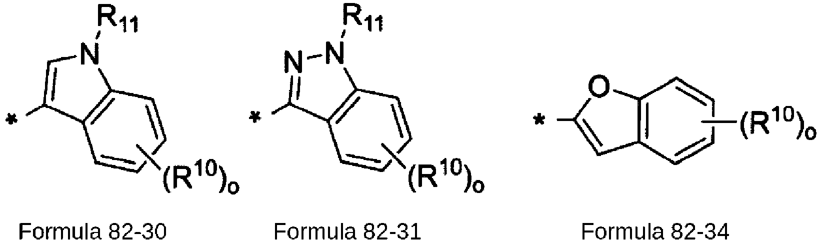 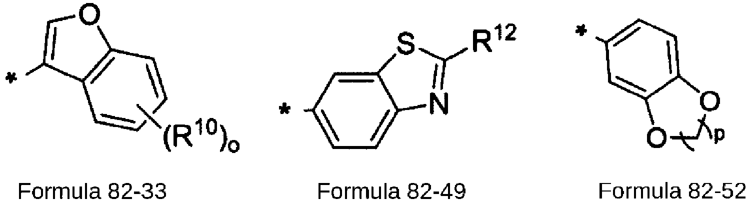
wherein, in Formula 82-1, R⁵, R⁸, and R⁹ are hydrogen.

8. The compound, isomer thereof, pharmaceutically acceptable salt thereof, or solvate thereof, according to any one of claims 5 to 7,
R⁶ and R⁷ in Formulas 82-1 to 82-52 are each independently selected from the group consisting of hydrogen, halogen, a hydroxy group, a C₁-C₆ alkyl group, a C₁-C₃ haloalkyl group, -CH₂R³, -OR³, -NR³R⁴, a C₃-C₈ cycloalkyl group, a 3- to 8-membered heterocycloalkyl group, a C₆-C₁₀ aryl group, and a 5- to 10-membered heteroaryl group, and
o is 0 or 1.

9. The compound, isomer thereof, pharmaceutically acceptable salt thereof, or solvate thereof according to claim 1, wherein, in Formula 78,
Z comprises one or more selected from the group consisting of groups represented by Formulas 83-1 to 83-11: 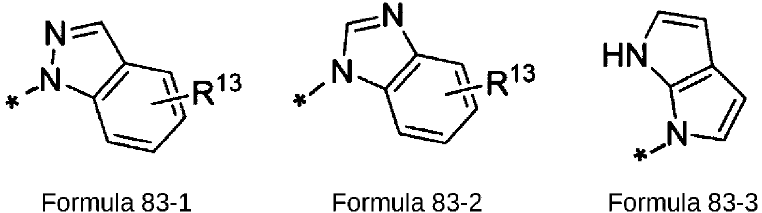 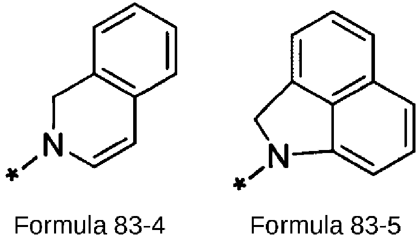 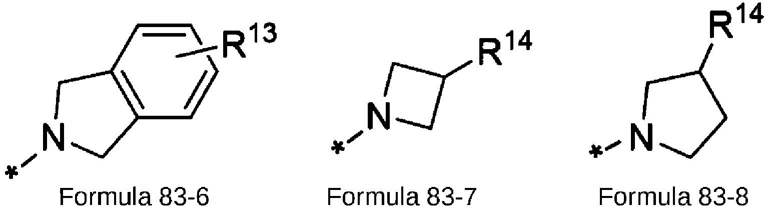 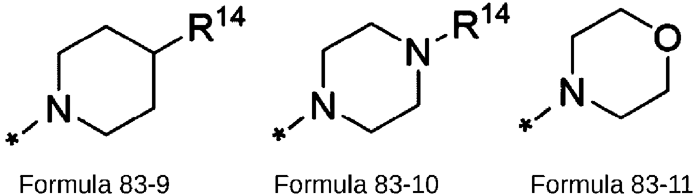
wherein, in the above Formulas,
R¹³ and R¹⁴ are each independently selected from the group consisting of hydrogen, halogen, a substituted or unsubstituted C₁-C₄ alkyl group, CF₃, NHR¹⁵, OR¹⁵, and a substituted or unsubstituted C₃-C₁₂ aryl group,
each R¹⁵ is independently selected from the group consisting of hydrogen, a substituted or unsubstituted C₁-C₄ alkyl group, a substituted or unsubstituted C₁-C₂ haloalkyl group, and a substituted or unsubstituted C₃-C₁₂ aryl group, and
* represents a point of attachment to an adjacent atom.

10. The compound, isomer thereof, pharmaceutically acceptable salt thereof, or solvate thereof according to claim 9, wherein, in Formula 78,
Z comprises one or more selected from the group consisting of groups represented by the following formulas:

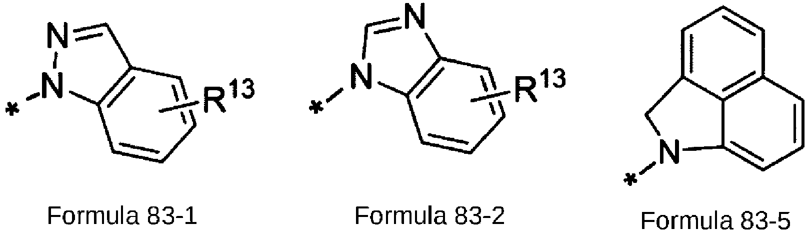

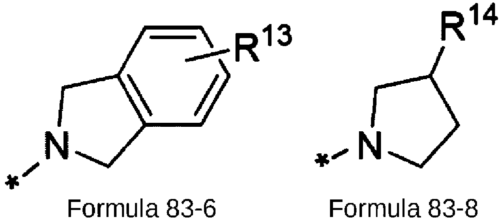

11. The compound, isomer thereof, pharmaceutically acceptable salt thereof, or solvate thereof according to claim 1, wherein the compound comprises one or more compounds selected from the group consisting of Compounds 1 to 77 described in Tables 1 to 8 of the specification.

12. The compound, isomer thereof, pharmaceutically acceptable salt thereof, or solvate thereof according to claim 1, wherein the compound is capable of binding to a Cereblon (CRBN) protein.

13. The compound, isomer thereof, pharmaceutically acceptable salt thereof, or solvate thereof according to claim 1, wherein the compound is for modulating activity of a Cereblon protein or a complex comprising the Cereblon protein.

14. The compound, isomer thereof, pharmaceutically acceptable salt thereof, or solvate thereof according to claim 1, wherein the compound modulates activity or degradation of a GSPT1 protein.

15. A pharmaceutical composition for preventing or treating a proliferative disease, comprising the compound, isomer thereof, pharmaceutically acceptable salt thereof, or solvate thereof according to any one of claims 1 to 14.

16. The pharmaceutical composition according to claim 15, wherein the proliferative disease is a GSPT1-related disease.

17. The pharmaceutical composition of claim 15, wherein the proliferative disease is selected from adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen-dependent and independent breast cancer, Burkitt's lymphoma, corpus cancer, carcinoma of unknown primary tumor (CUP-syndrome), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose, and throat tumors, hematologic tumor, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose, and throat regions), colon carcinoma, craniopharyngiomas, oral cancer, cancer of the central nervous system, liver cancer, liver metastases, leukemia, eyelid tumor, lung cancer, lymphomas, stomach cancer, malignant melanoma, malignant neoplasia, malignant tumors of the gastrointestinal tract, breast carcinoma, rectal cancer, medulloblastomas, melanoma, meningiomas, Hodgkin's/non-Hodgkin's lymphomas, mycosis fungoides, nasal cancer, neurinoma, neuroblastoma, kidney cancer, renal cell carcinomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, esophageal cancer, T-cell lymphoma, thymoma, tube carcinoma, eye tumors, urethral cancer, urologic tumors, urothelial carcinoma, vulva cancer, wart appearance, soft tissue tumors, soft tissue sarcoma, nephroblastoma, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypothalamic glioma, cerebellar astrocytoma, cerebral astrocytoma, neuroectodermal tumor, pineal tumors, sarcoma of the uterus, salivary gland cancers, anal gland adenocarcinomas, mast cell tumors, pelvis tumor, ureter tumor, hereditary papillary renal cancers, sporadic papillary renal cancers, intraocular melanoma, hepatocellular carcinoma, cholangiocarcinoma, mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymph sarcoma, rhabdomyosarcoma, malignant histiocytosis, fibroblastic sarcoma, hemangiosarcoma, hemangiopericytoma, leiomyosarcoma (LMS), canine mammary carcinoma, feline mammary carcinoma, and combinations thereof.

18. A GSPT1 degrader comprising the compound, isomer thereof, pharmaceutically acceptable salt thereof, or solvate thereof according to any one of claims 1 to 14.
